(19) Europäisches Patentamt
European Patent Office
Office européen des brevets

(11) Numéro de publication : **0 542 622 A1**

(12) # DEMANDE DE BREVET EUROPEEN

(21) Numéro de dépôt : **92403046.3**

(22) Date de dépôt : **12.11.92**

(51) Int. Cl.$^5$ : **C07J 63/00,** A61K 31/56

(30) Priorité : **13.11.91 FR 9113907**

(43) Date de publication de la demande :
**19.05.93 Bulletin 93/20**

(84) Etats contractants désignés :
**PT**

(71) Demandeur : **RHONE-POULENC RORER SA**
**20, avenue Raymond Aron**
**F-92160 Antony (FR)**

(72) Inventeur : **Bouboutou, Romaine**
**67, av Aristide Briand**
**F-94110 Arcueil (FR)**
Inventeur : **Dereu, Norbert**
**4 allée du Potager**
**f-91170 Viry Chatillon (FR)**
Inventeur : **Evers, Michel**
**9, rue de Campine**
**B-4000 Liège (BE)**
Inventeur : **Gueguen, Jean-Christophe**
**35, rue du Mont Valérien**
**F-92210 St Cloud (FR)**
Inventeur : **James, Claude, Domaine de Lésigny**
**3, Allée Louis de Broglie**
**F-77150 Lésigny (FR)**
Inventeur : **Poujade, Christèle**
**81, rue de Paris**
**F-94340 Joinville (FR)**
Inventeur : **Reisdorf, Daniel**
**39, av de la Républic**
**F-94320 Thiais (FR)**
Inventeur : **Ribeill, Yves**
**29, The Durdans**
**Langdon Hills SS16 6DA Basildon Essex (GB)**
Inventeur : **Soler, Françoise**
**39-41, rue Saint-Fargeau**
**F-75020 Paris (FR)**

(74) Mandataire : **Savina, Jacques et al**
**Rhône-Poulenc Rorer S.A. Direction Brevets (t144) 20 avenue Raymond Aron**
**F-92165 Antony Cédex (FR)**

(54) **Nouveaux dérivés du lupane, leur préparation et les compositions pharmaceutiques qui les contiennent.**

(57) Nouveaux dérivés du lupane de formule générale (I) dans laquelle R est :

```
——(CH)n——X——(CH2)m——Y——(C)p——COOR'
   |                     / \
   R"                  R°   R°°
```

dans laquelle R' et R" identiques ou différents sont H ou alcoyle, X est une liaison, carbamoyle ou aminocarbonyle éventuellement substitués, Y est une liaison ou phénylène, R° et R°° identiques ou différents sont H ou alcoyle ou R° est OH, hydroxyalcoyle, phényle, benzyle, carbamoylméthyle, ou forme un hétérocycle avec l'azote de X lorsque X est carbamoyle et Y est une liaison, et, n, m et p sont des entiers de 0 à 16, $4 \leq m+n+p \leq 16$,
$R_1$ est méthyle, $R_2$ est hydroxy, méthyle, hydroxyméthyle, $-CH_2OR'_2$, $-CH_2SR'_2$ ou $-CH_2NHR'_2$ ou , $R_2$ est amino substitué ou dialcoylamino dont les parties alcoyle peuvent former un hétérocycle,
$R_3$ est H ou forme avec $R_1$ ou $R_2$ méthylène ou oxo,
$R_4$ et $R_5$ sont différents et représentent H ou hydroxy, ou forment ensemble oxo, hydroxyimino ou alcoyloxyimino qui peut être substitué, et $R_6$ et $R_7$ sont H, ou bien 02
$R_4$ et $R_5$, et $R_6$ et $R_7$ forment ensemble des radicaux oxo, les radicaux alcoyle (1 à 4C) étant droits ou ramifiés, ainsi que leurs sels lorsqu'ils existent et leur préparation.
Les nouveaux produits selon l'invention sont particulièrement intéressants dans le domaine du SIDA.

EP 0 542 622 A1

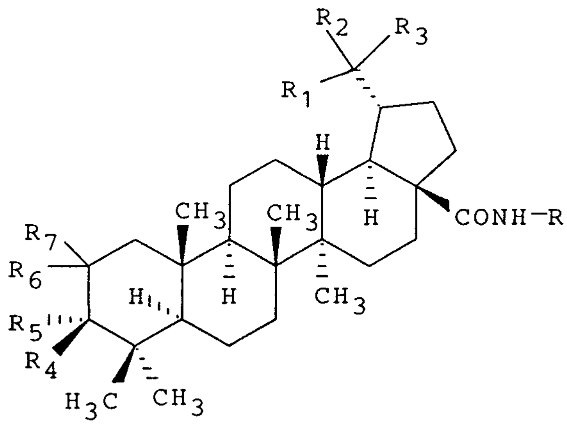

(I)

La présente invention concerne de nouveaux dérivés du lupane, de formule générale :

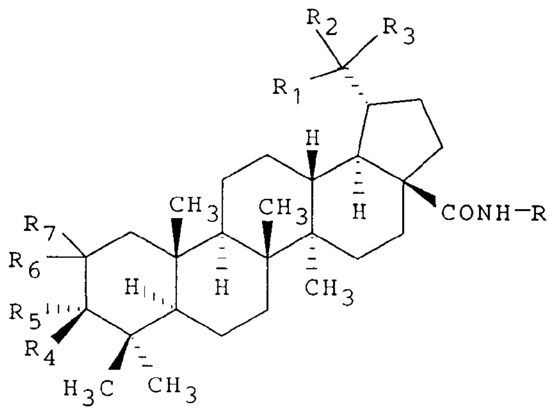

(I)

leurs sels, leur préparation et les compositions pharmaceutiques qui les contiennent.

Dans la demande de brevet japonais J 01 143 832 ont été décrits des dérivés de la bétuline de formule générale :

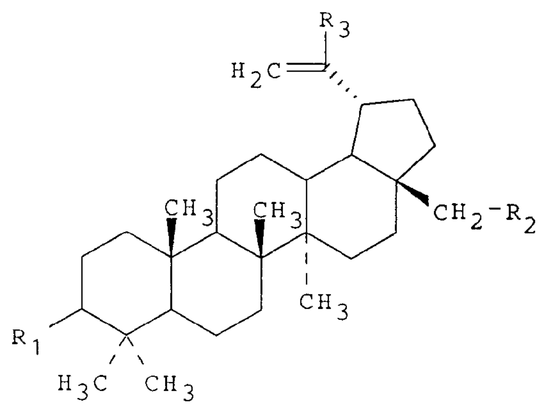

dans laquelle $R_1$ et $R_2$ sont hydroxy ou acyloxy et $R_3$ est notamment méthyle. Ces dérivés sont utiles dans le domaine des anticancéreux.

Il a été maintenant trouvé que les dérivés du lupane de formule générale (I) dans laquelle :

R représente un radical de formule générale :

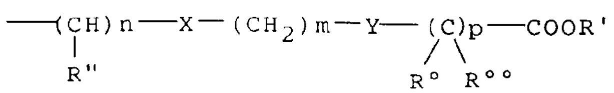

(II)

dans laquelle R' et R" identiques ou différents sont des atomes d'hydrogène ou des radicaux alcoyle,

X est une liaison ou représente un radical carbamoyle, N-méthyl carbamoyle, aminocarbonyle ou N-méthylaminocarbonyle,

Y est une liaison ou représente un radical phénylène,

R° et R°° identiques ou différents sont des atomes d'hydrogène ou des radicaux alcoyle (étant entendu que R° et R°° ne sont pas nécessairement identiques sur chaque motif -CR°R°°-), ou R° est hydroxy, hydroxyalcoyle, phényle, benzyle, carbamoylméthyle ou bien, lorsque Y est une liaison et X est carbamoyle, R° peut former un cycle à 5 ou 6 chaînons avec l'atome d'azote contenu dans X, ce cycle pouvant en outre comprendre un autre hétéroatome choisi parmi l'oxygène ou le soufre et,

n, m et p sont des nombres entiers de 0 à 16 tels que m + n + p soit compris entre 4 et 16,

$R_1$ est un radical méthyle, ou forme avec $R_3$ un radical méthylène ou un radical oxo,

$R_2$ est un radical hydroxy, méthyle, hydroxyméthyle ou un radical $-CH_2OR'_2$, $-CH_2SR'_2$ ou $-CH_2NHR'_2$ pour lequel $R'_2$ est alcoyle, hydroxyalcoyle, dihydroxyalcoyle, acétamidoalcoyle, ou acétyle, ou $R_2$ est un radical amino

substitué par un radical hydroxyalcoyle ou carboxyhydroxyalcoyle, ou un radical dialcoylamino dont les parties alcoyle peuvent former avec l'atome d'azote auquel elle sont rattachées, un hétérocycle à 5 ou 6 chaînons contenant éventuellement un autre hétéroatome choisi parmi l'oxygène, le soufre ou l'azote et éventuellement N-alcoylé,

$R_3$ est un atome d'hydrogène ou forme avec $R_1$ ou $R_2$ un radical méthylène ou un radical oxo,

$R_4$ et $R_5$ sont différents et représentent un atome d'hydrogène ou un radical hydroxy, ou forment ensemble un radical oxo, hydroxyimino ou alcoyloxyimino éventuellement substitué (par un radical carboxy ou dialcoylamino dont les parties alcoyle peuvent former avec l'atome d'azote auquel elles sont rattachées, un hétérocycle à 5 ou 6 chaînons contenant éventuellement un autre hétéroatome choisi parmi l'oxygène, l'azote ou le soufre et éventuellement substitué par un radical alcoyle) et

$R_5$ et $R_7$ sont des atomes d'hydrogène, ou bien

$R_4$ et $R_5$, et $R_5$ et $R_7$ forment ensemble des radicaux oxo,

ainsi que leurs sels pharmaceutiquement acceptables lorsqu'ils existent, manifestent un effet cytoprotecteur d e cellules infectées par un virus HIV (Human Immunodeficiency Virus) ainsi qu'une activité inhibitrice de la production de transcriptase inverse d'un virus HIV.

Dans la formule générale ci-dessus, il est entendu que les radicaux alcoyle sont droits ou ramifiés et contiennent 1 à 4 atomes de carbone.

Selon l'invention les produits de formule générale (I) peuvent être obtenus par action d'un amino acide de formule générale :

$$H_2N - R \qquad (III)$$

dans laquelle R est défini comme ci-dessus et dont le cas échéant la fonction acide a été préalablement protégée, sur le chlorure de l'acide de formule générale :

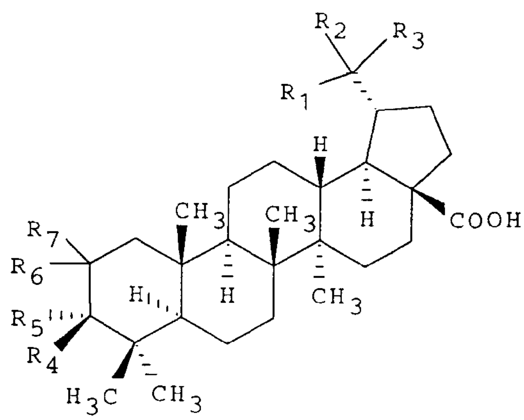

(IV)

dans laquelle $R_1$, $R_2$, $R_3$, $R_4$, $R_5$, $R_5$ et $R_7$ sont définis comme précédemment, suivie le cas échéant de la transformation en un ester pour lequel le symbole R' est alcoyle, puis de l'élimination des radicaux protecteurs.

Il est entendu que lorsque $R_2$, $R_4$ ou $R_5$ représentent ou contiennent un radical hydroxy, celui-ci est préalablement protégé; il en est de même si $R_2$ contient un radical carboxy ou si le substituant $-CR_1R_2R_3$ en position 19 est un radical carboxy.

La réaction de l'amino acide sur le chlorure de l'acide lupèn-20(29)oïque-28 de formule générale (IV) s'effectue selon les méthodes habituelles qui n'altèrent pas le reste de la molécule. On opère notamment en présence d'une base organique azotée telle qu'une trialcoylamine (triéthylamine par exemple) dans un solvant organique tel qu'un solvant chloré (chloroforme, dichloro-1,2 éthane, dichlorométhane) ou dans le tétrahydrofurane ou dans un mélange de ces solvants, à une température comprise entre 15 et 30°C.

La fonction acide de l'amino acide de formule générale (III) est protégée par tout radical compatible et dont la mise en place et l'élimination n'altère pas le reste de la molécule. De même les radicaux $R_2$, $R_4$ ou $R_5$ du dérivé du lupane de formule générale (IV) sont protégés par des radicaux compatibles et pouvant être mis en place et éliminés sans toucher au reste de la molécule. A titre d'exemple les groupements protecteurs peuvent être choisis parmi les radicaux décrits par T.W. GREENE, Protective Groups in Organic Synthesis, J. Wiley-Interscience Publication (1991) ou par Mc Omie, Protective Groups in Organic Chemistry, Plenum Press (1973). Il est notamment avantageux d'utiliser des radicaux protecteurs pouvant être éliminés simultanément. On choisira des radicaux pouvant être éliminés en milieu neutre, basique ou acide. Les protections peuvent être effectuées avantageusement à l'état d'esters. Dans ce cas, l'élimination des radicaux protecteurs s'effectue par hydrolyse en milieu basique, notamment en présence de soude à une température comprise entre 10

et 50°C.

A titre d'exemple, les radicaux hydroxy peuvent être protégés à l'état d'ester (formyloxy, acétoxy, i.butoxy, trichloracétyloxy, phénoxyacétyloxy, benzyloxy), à l'état de cétone, sous forme de carbonate par un radical -COOR$_a$ dans lequel R$_a$ est un radical alcoyle ou benzyle éventuellement substitués ou encore par un radical trialcoylsilyle; les radicaux protecteurs d'acide peuvent être choisis parmi les radicaux alcoyle (méthyle, éthyle, t.butyle), alcoyle substitué (trichloréthyle, haloéthyle, p.toluènesulfonyléthyle, benzyle, benzyle substitué. par un radical nitro, benzhydryle, triphénylméthyle, benzyloxyméthyle...), alcoyloxyalcoyle (méthoxyméthyle), tétrahydropyranyle ou triméthylsilyle.

Les dérivés du lupane de formule générale (I) dans lesquels le radical R possède un centre chiral, présentent des formes isomères. Il est entendu que ces formes isomères et leurs mélanges entrent dans le cadre de la présente invention. La préparation de l'une ou l'autre de ces formes dépend du choix de l'amino acide de départ.

Le cas échéant, la transformation en un ester pour lequel R' est un radical alcoyle, s'effectue selon les méthodes habituelles qui n'altèrent le reste de la molécule. Notamment on opère par action d'un iodure d'alcoyle.

Selon l'invention le dérivé du lupane de formule générale (I) pour lequel $R_4$ et $R_5$ forment ensemble un radical hydroxyimino ou alcoyloxyimino éventuellement substitué comme défini précédemment, et les autres radicaux sont définis comme précédemment peut également être obtenu par action de l'hydroxylamine ou un de ses dérivés de formule générale :

$$R_8\text{-O-NH}_2 \qquad (V)$$

dans laquelle $R_8$ est un atome d'hydrogène ou un radical alcoyle éventuellement substitué (par un radical carboxy ou dialcoylamino dont les parties alcoyle peuvent former avec l'atome d'azote auquel elles sont rattachées, un hétérocycle à 5 ou 6 chaînons contenant éventuellement un autre hétéroatome choisi parmi l'oxygène, l'azote ou le soufre et éventuellement substitué par un radical alcoyle), sur un dérivé du lupane de formule générale (I) dans laquelle $R_4$ et $R_5$ forment ensemble un radical oxo, puis le cas échéant libération des radicaux protecteurs.

Il est entendu que si $R_8$ contient un radical carboxy ou si le dérivé du lupane de formule générale (I) contient des radicaux carboxy ou hydroxy, ces derniers sont préalablement protégés. La protection et la libération des radicaux protecteurs s'effectuent selon les méthodes citées précédemment.

La réaction s'effectue avantageusement à partir du chlorhydrate du dérivé de formule générale (V) dans la pyridine à la température de reflux du mélange réactionnel, ou dans un alcool en présence d'un carbonate alcalin à la température de reflux du mélange réactionnnel.

Selon l'invention le dérivé du lupane de formule générale (I) pour lequel $R_1$ et $R_2$ sont des radicaux méthyle, $R_3$ est un atome d'hydrogène et les autres radicaux sont définis comme précédemment peut également être obtenu par réduction d'un dérivé du lupane de formule générale (I) pour lequel $R_1$ et $R_3$ forment ensemble un radical méthylène et $R_2$ est défini comme ci-dessus.

La réduction s'effectue par toute méthode connue qui n'affecte pas le reste de la molécule. Notamment on opère par hydrogénation catalytique en présence de palladium sur charbon, sous pression atmosphérique, à une température comprise entre 15 et 25°C dans un solvant tel qu'un alcool (méthanol, éthanol par exemple).

Le cas échéant les radicaux susceptibles d'interférer avec la réaction sont préalablement protégés. La protection et l'élimination des radicaux protecteurs s'effectue selon les méthodes citées précédemment.

Selon l'invention le dérivé du lupane de formule générale (I) pour lequel $R_2$ est un radical hydroxy, $R_1$ est un radical méthyle, $R_3$ est un atome d'hydrogène et les autres radicaux sont définis comme précédemment peut également être obtenu par réduction d'un dérivé du lupane de formule générale (I) pour lequel $R_2$ et $R_3$ forment ensemble un radical oxo et $R_1$ est un radical méthyle.

La réduction s'effectue par toute méthode connue qui n'affecte pas le reste de la molécule. Notamment la réduction s'effectue par un borohydrure alcalin (borohydrure de sodium par exemple) en présence de chlorure de tétrabutylammonium ou de tétraéthylammonium, dans un solvant tel qu'un hydrocarbure aromatique (toluène par exemple), à une température comprise entre 50°C et la température de reflux du mélange réactionnel.

Le cas échéant les radicaux susceptibles d'interférer avec la réaction sont préalablement protégés. La protection et l'élimination des radicaux protecteurs s'effectue selon les méthodes citées précédemment.

Selon l'invention le dérivé du lupane de formule générale (I) pour lequel $R_1$ et $R_3$ forment ensemble un radical oxo, $R_2$ est un radical hydroxy et les autres radicaux sont définis comme précédemment peut également être obtenu par oxydation d'un dérivé du lupane de formule générale (I) pour lequel $R_1$ et $R_3$ forment ensemble un radical oxo et $R_2$ est un radical méthyle.

La réaction s'effectue généralement par action d'un hypochlorite ou d'un hypobromite. Par exemple par action de l'hypobromite de sodium qui peut être preparé in situ, en milieu hydroorganique, notamment dans le

dioxane, à une température comprise entre -10 et 20°C.

Le cas échéant les radicaux susceptibles d'interférer avec la réaction sont préalablement protégés. La protection et l'élimination des radicaux protecteurs s'effectuent selon les méthodes citées précédemment.

Selon l'invention le dérivé du lupane de formule générale (I) pour lequel X (dans le radical R) représente un radical carbamoyle et les autres radicaux sont définis comme précédemment, peut être également obtenu à partir d'un dérivé du lupane de formule générale :

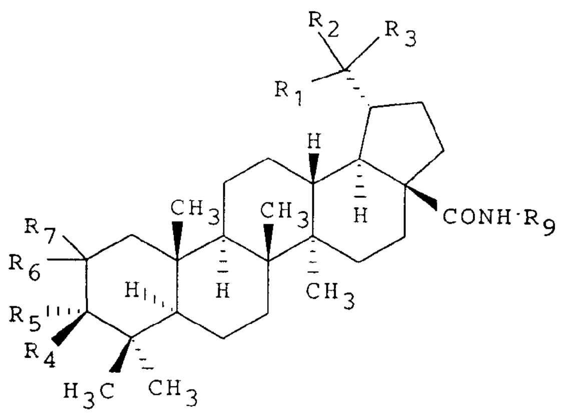

(VI)

dans laquelle $R_1$, $R_2$, $R_3$, $R_4$, $R_5$, $R_6$ et $R_7$ sont définis comme précédemment et $R_9$ représente un radical de formule générale :

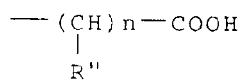

(VII)

dans laquelle n et R" sont définis comme précédemment, par action d'une amine de formule générale :

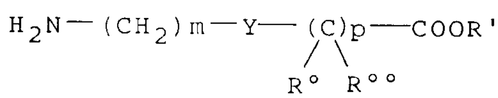

(VIII)

dans laquelle R', R°, R°°, m et p sont définis comme précédemment puis le cas échéant transformation de l'acide obtenu en un ester pour lequel R' est un radical alcoyle et élimination le cas échéant des radicaux protecteurs.

Il est entendu que les radicaux représentant ou contenant des radicaux pouvant interférer avec la réaction sont préalablement protégés. La protection et la libération des radicaux protecteurs s'effectuent comme décrit précédemment.

La condensation de l'amine de formule générale (VIII) s'effectue de préférence à partir du chlorhydrate de l'amine. La réaction s'effectue selon les méthodes habituelles de condensation d'une amine sur un acide. Notamment, on opère en présence d'un accepteur d'acide tel qu'une base organique azotée (trialcoylamine, pyridine, N-méthylmorpholine, diaza-1,8 bicyclo[5.4.0]undécène-7, diaza-1,5 bicyclo (4.3.0]nonène-5 par exemple) dans un solvant organique comme un solvant chloré (dichlorométhane, dichloréthane, chloroforme par exemple) ou un amide (diméthylformamide par exemple). Il est également possible d'opérer en présence d'un agent de condensation tel qu'un carbodiimide (dicyclohexylcarbodiimide, chlorhydrate de 1-(3-diméthylaminopropyl)-3-éthylcarbodiimide par exemple) et éventuellement en présence d'un catalyseur tel que le N-hydroxybenzotriazole ou le N-hydroxysuccinimide, à une température comprise entre -20 et 40°C.

L'amino acide de formule générale (III) et le dérivé du lupane de formule générale (IV) peuvent être préparés comme décrit ci-après dans les exemples.

Notamment, les dérivés du lupane de formule générale (IV) pour lesquels $R_1$ est un radical méthyle, $R_2$ est un radical hydroxy ou méthyle et $R_3$ est un atome d'hydrogène peuvent être préparés selon le procédé de réduction décrit précédemment pour la préparation des produits de formule générale (I) pour lesquels $R_2$ est un radical hydroxy.

Les dérivés du lupane de formule générale (IV) pour lesquels $R_1$ est un radical méthyle et $R_2$ et $R_3$ forment

ensemble un radical oxo peuvent être préparés selon la méthode décrite par A. VYSTRCIL et coll., Collect. Czech. Chem. Comm., 35, 295 (1970).

Les dérivés du lupane de formule générale (IV) pour lesquels $R_2$ est un radical hydroxy et $R_1$ et $R_3$ forment ensemble un radical oxo peuvent être préparés par analogie avec la méthode décrite pour la préparation des produits correspondants de formule générale (I), par oxydation de la méthylcétone correspondante. Il est entendu que la protection de l'acide en position -19 ainsi obtenu est indispensable pour la mise en oeuvre dans la réaction suivante.

Les dérivés du lupane de formule générale (IV) pour lesquels $R_2$ est un radical hydroxyméthyle peuvent être préparés par action de l'acétate d'argent sur le dérivé bromé correspondant pour lequel $R_2$ est bromométhyle et pour lequel, si nécessaire, le radical $R_4$ ou $R_5$ est préalablement protégé. La réaction s'effectue avantageusement dans un solvant organique comme par exemple le toluène, à une température comprise entre 20°C et la température de reflux du mélange réactionnel. Le dérivé bromé de départ peut être obtenu par action d'un agent de bromation comme par exemple le tribromure de tétrabutylammonium ou le N-bromosuccinimide sur le dérivé correspondant de l'acide lup-20(29)-èn-28-oïque. La réaction s'effectue dans un solvant chloré comme le chlorure de méthylène, le chloroforme, ou le tétrachlorure de carbone à une température voisine de 25°C.

Les dérivés du lupane de formule générale (IV) pour lesquels $R_2$ est un radical amino substitué et $R_1$ et $R_3$ forment ensemble un radical oxo peuvent être préparés par action de l'amine correspondante sur le dérivé du lupane de formule générale (IV) pour lequel $R_2$ est un radical hydroxy et $R_1$ et $R_3$ forment ensemble un radical oxo. La réaction s'effectue par toute méthode connue pour obtenir un amide à partir d'un acide sans toucher au reste de la molécule. On opère notamment par action de l'amine sur le chlorure de l'acide de formule générale (IV), dans un solvant chloré, à une température comprise entre -20 et 40°C. Il est entendu que les fonctions pouvant interférer avec la réaction sont préalablement protégées, notamment lorsque le radical amino substitué est un radical carboxy hydroxy alcoyle, il est nécessaire de protéger le radical carboxy.

Les dérivés du lupane de formule générale (IV) pour lesquels $R_2$ est un radical amino substitué et $R_1$ et $R_3$ forment ensemble un radical oxo peuvent être préparés par action de l'amine correspondante sur le dérivé du lupane pour lequel $R_2$ est un radical bromométhyle en présence de tétra[triphénylphosphine] palladium et d'une base azotée.

Les dérivés du lupane de formule générale (IV) pour lesquels $R_2$ est un radical $-CH_2OR'_2$ ou $-CH_2SR'_2$ et $R_1$ et $R_3$ forment ensemble un radical méthylène peuvent être préparés à partir du dérivé du lupane pour lequel $R_2$ est bromométhyle, par action de l'alcoolate ou du thiolate correspondant.

L'acide 3α-hydroxylup-20(29)-èn-28-oïque (acide 3α-bétulinique) et ses dérivés substitués en position 20 ou 30 peuvent être obtenus selon ou par analogie avec la méthode décrite par W. HERZ, Phytochemistry, 11, 3061 (1972).

Les dérivés du lupane de formule générale (IV) pour lesquels $R_4$ et $R_5$ forment ensemble un radical oxo, peuvent être préparés par oxydation du dérivé du lupane de formule générale (IV) pour lequel $R_4$ et $R_5$ représentent respectivement un atome d'hydrogène et un radical hydroxy. L'oxydation s'effectue avantageusement par un agent oxydant comme l'anhydride chromique.

Les dérivés du lupane de formule générale (IV) pour lesquels $R_4$ et $R_5$, et $R_6$ et $R_7$ forment ensemble des radicaux oxo, peuvent être préparés par oxydation du dérivé du lupane de formule générale (IV) pour lequel $R_4$ et $R_5$ forment ensemble un radical oxo.

Les dérivés du lupane de formule générale (IV) pour lesquels $R_4$ et $R_5$ forment ensemble un radical hydroxyimino ou alcoyloxyimino éventuellement substitué (par un radical carboxy ou dialcoylamino dont les parties alcoyle peuvent former avec l'atome d'azote auquel elles sont rattachées, un hétérocycle à 5 ou 6 chaînons contenant éventuellement un autre hétéroatome choisi parmi l'oxygène, l'azote ou le soufre et éventuellement substitué par un radical alcoyle) peuvent être préparés par analogie avec le procédé décrit précédemment pour la préparation des dérivés du lupane de formule générale (I) pour lesquels $R_4$ et $R_5$ forment ensemble un radical $=N-O-R_8$.

Le chlorure d'acide du dérivé du lupane de formule générale (IV) peut être préparé selon les méthodes connues. A titre d'exemple, on opère par action du chlorure d'oxalyle ou du chlorure de thionyle, dans un solvant chloré comme le chloroforme, le dichloroéthane ou le dichlorométhane.

Les dérivés du lupane de formule générale (VI) peuvent être préparés par analogie avec la méthode décrite pour la préparation des produits de formule générale (I), à partir d'un produit de formule générale (IV).

Les produits de formule générale (VIII) peuvent être préparés selon ou par analogie avec les méthodes décrites ci-après dans les exemples.

Les nouveaux dérivés du lupane de formule générale (I) peuvent être purifiés le cas échéant par des méthodes physiques telles que la cristallisation ou la chromatographie.

Les produits selon l'invention peuvent être transformés en sels métalliques ou en sels d'addition avec une

base azotée selon les méthodes connues en soi. Ces sels peuvent être obtenus par action d'une base métallique (par exemple alcaline ou alcalino terreuse), de l'ammoniaque ou d'une amine sur un produit selon l'invention, dans un solvant approprié tel que l'eau ou un alcool. Le sel formé précipite après concentration éventuelle de la solution ; il est séparé par filtration.

Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN'-diméthyléthanolamine, benzylamine, dicyclohexylamine, N-benzylphénéthylamine, NN'-dibenzyléthylènediamine, diphénylènediamine, benzhydrylamine, quinine, choline, arginine, lysine, leucine, dibenzylamine).

Les nouveaux dérivés du lupane selon la présente invention sont particulièrement utiles pour la prophylaxie et le traitement du SIDA (syndrome d'immunodéficience acquise) et de syndromes associés [ARC (AIDS related complex)]. Par prophylaxie nous sous-entendons le traitement des sujets qui ont été exposés aux virus HIVs, en particulier les séropositifs asymptomatiques qui présentent le risque de développer la maladie dans les mois ou les années à venir après la primoinfection.

Les produits selon l'invention, qui sont inhibiteurs de l'effet cytopathogène du HIV et inhibiteurs de la production de transcriptase inverse en culture cellulaire à des concentrations dépourvues d'effet cytotoxique ou cytostatique, sont particulièrement intéressants.

Les activités ont été mises en évidence dans les tests suivants :

Activité vis-à-vis de l'effet cytopathogène du virus HIV

Les produits en poudre ont été mis en solution à raison de 2 mg de produit par 2 ml (environ 4 x $10^{-3}$ M) dans un mélange de diméthylformamide (DMF) et de L-lysine (base) à (1:19, vol:vol). On ajoute d'abord 1 volume de DMF et on solubilise le produit au mieux puis on ajoute 9 volumes d'une solution de L-lysine base à 4 x $10^{-3}$ M dans de l'eau distillée. De cette manière on obtient une solution stock de produit à 10 % de DMF et contenant un rapport molaire (produit/lysine) proche de 1. Le test est réalisé sur la lignée lymphoblastoïde CEM clone 13. Dans une microplaque de 96 puits on dépose 25 µl/puits d'une solution de produit à tester dans du tampon phosphate isotonique (TPI) ou de TPI seul dans le cas des contrôles. Les produits sont étudiés à différentes concentrations (souvent 8), à raison de 6 puits par concentration. On ajoute alors 125 µl d'une suspension de cellules CEM (8 x $10^4$ cellules par ml) dans le milieu RPMI contenant 10 % de sérum de veau foetal, 100 UI/ml de pénicilline, 100 µg/ml de streptomycine et 2 µmoles/ml de glutamine et les microplaques sont incubées une heure à 37°C, sous une atmosphère contenant 5 % de gaz carbonique. Pour chaque concentration, l'essai est partagé en deux parties : une partie (3 puits) sur cellules infectées, pour la détermination de l'activité antivirale et l'autre partie (3 puits) sur cellules non infectées, pour déterminer la cytotoxicité des produits. On infecte alors la première série avec HIV-1 (100 µl par puits d'une suspension de virus LAV-1-BRU contenant 200-300 TCID50) tandis que l'autre série reçoit 100 µl de milieu RPMI tel que défini précédemment. Au bout de 7 jours d'incubation, 100 µl de cellules sont prélevés pour mesurer la viabilité cellulaire [déterminée selon une modification de la technique décrite par R. Pauwels et coll., J. Virol. Meth., 20, 309-321 (1988)]. On ajoute à ce prélèvement 10 µl d'une solution contenant 7 mg de MTT [bromure de 3-(4,5-diméthyl 2-thiazolyl)-2,5-diphényltétrazolium] par ml de tampon phosphate isotonique. Après 3 heures d'incubation à 37°C le surnageant est enlevé. Le MTT est converti en un sel de formazan (bleu) uniquement à l'intérieur des cellules vivantes. On ajoute alors 100 µl d'isopropanol (contenant 0,04 mole/l d'acide chlorhydrique) et les microplaques sont agitées jusqu'à la solubilisation du bleu de formazan. L'absorbance à 540 nm est lue avec un lecteur automatique de réactions ELISA en microplaques. Cette absorbance est proportionnelle à la quantité de cellules vivantes.

Le taux de protection (en %) d'un produit donné est déterminé à partir des densités optiques (DO) par la formule :

$$\frac{\text{DO(cell. traitées et inf.)} - \text{DO(cell. non traitées et inf.)}}{\text{DO(cell. traitées et non inf.)} - \text{DO(cell. non traitées et inf.)}}$$

Le cas échéant la concentration inhibitrice 50 % est déterminée.

Les résultats démontrent que pour des concentrations de produit testé comprises entre 0,5 et 30 µg/ml, on obtient une diminution significative de l'effet cytopathogène.

Le taux de protection procuré par les produits selon l'invention est compris entre 20 et 100 %.

La concentration inhibitrice 50 % des produits selon l'invention est comprise entre 0,30 et 100 µg/ml lorsqu'elle peut être déterminée.

Détermination de la multiplication virale par dosage de la transcriptase inverse du virus HIV

L'activité de la transcriptase inverse est mesurée directement sur 50 μl de surnageant de culture. Selon la méthode décrite par O. Schwartz et coll., AIDS Research and Human Retrovirus 4(6), 441-448 (1988). 10 μl de tampon contenant 0,5 M de KCl, 5 mM de dithiothreitol (DTT) et 0,5 % de triton X-100 sont ajoutés à tous les micropuits contenant les 50 μl de surnageant à tester. 40 μl de tampon contenant les 1,25 mM d'éthylène glycol-bis (2-aminoéthyl éther) tétraacétique acide (EGTA), 0,125 mM de Tris. HCl pH 7,8, 12,5 mM de $MgCl_2$, 3 μCi de ($^3H$) thymidine triphosphate (TTP), 0,05 $DO_{260}$ d'acide polyadénylique - acide thymidylique (poly rA-oligo dT) sont ajoutés ensuite. La microplaque est couverte au moyen d'un film plastique et incubée 60 minutes à 37°C.

On arrête la réaction en ajoutant 20μl d'une solution glacée de 120 mM de $Na_4P_2O_7$ dans 60 % d'acide trichloracétique et les échantillons sont placés 15 minutes sur le lit de glace.

Les précipités sont filtrés sur fibres de verre en utilisant un laveur de cellules (Skatron) et les filtres sont lavés avec une solution de 12 mM de $Na_4P_2O_7$ dans 5 % d'acide trichloracétique.

Les filtres sont séchés et la radioactivité est comptée après ajout d'un liquide scintillant.

On observe des inhibitions de la production de transcriptase inverse associée au virus, comprises entre 20 et 100 % aux concentrations testées (0,30 à 100 μg/ml).

D'un intérêt particulier sont les produits de formule générale (I) dans laquelle :
R représente un radical de formule générale :

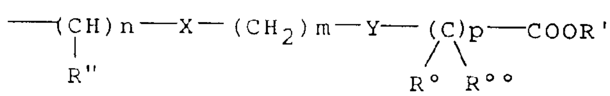

dans laquelle R' et R" identiques ou différents sont des atomes d'hydrogène ou des radicaux alcoyle,
X est une liaison ou représente un radical carbamoyle, N-méthyl carbamoyle, aminocarbonyle,
Y est une liaison ou représente un radical phénylène,
R° et R°° identiques ou différents sont des atomes d'hydrogène ou des radicaux alcoyle (étant entendu que R° et R°° ne sont pas nécessairement identiques sur chaque motif -CR°R°°-), ou R° est hydroxy, hydroxyalcoyle, phényle, benzyle, carbamoylméthyle ou bien, lorsque Y est une liaison et X est carbamoyle, R° peut former un cycle à 5 ou 6 chaînons avec l'atome d'azote contenu dans X et,
n, m et p sont des nombres entiers de 0 à 16 tels que m + n + p soit compris entre 4 et 16,
$R_1$ est un radical méthyle, ou forme avec $R_3$ un radical méthylène ou un radical oxo,
$R_2$ est un radical hydroxy, méthyle, hydroxyméthyle ou un radical $-CH_2SR'_2$, ou $-CH_2NHR'_2$ pour lequel $R'_2$ est alcoyle, hydroxyalcoyle, acétamidoalcoyle, ou acétyle, ou $R_2$ est un radical amino substitué par un radical hydroxyalcoyle ou carboxyhydroxyalcoyle, ou un radical dialcoylamino dont les parties alcoyle peuvent former avec l'atome d'azote auquel elle sont rattachées, un hétérocycle à 5 ou 6 chaînons contenant éventuellement un autre atome d'azote et éventuellement N-méthylé,
$R_3$ est un atome d'hydrogène ou forme avec $R_1$ ou $R_2$ un radical méthylène ou un radical oxo,
$R_4$ et $R_5$ sont différents et représentent un atome d'hydrogène ou un radical hydroxy, ou forment ensemble un radical oxo, ou alcoyloxyimino éventuellement substitué (par un radical carboxy ou dialcoylamino dont les parties alcoyle peuvent former avec l'atome d'azote auquel elles sont rattachées, un hétérocycle à 5 ou 6 chaînons contenant éventuellement un autre atome d'azote éventuellement substitué par un radical méthyle) et
$R_5$ et $R_7$ sont des atomes d'hydrogène, ou bien
$R_4$ et $R_5$, et $R_5$ et $R_7$ forment ensemble des radicaux oxo,
étant entendu que les radicaux alcoyle sont droits ou ramifiés et contiennent 1 à 4 atomes de carbone, ainsi que leurs sels lorsqu'ils existent.

Et parmi ces produits, plus spécialement actifs sont :

- la N'-(N-[3β-hydroxylup-20(29)-èn-28-oyl]-8-aminooctanoyl]-β-alanine ;
- la N'-[N-[3β-hydroxylup-20(29)-èn-28-oyl]-8-aminooctanoyl]-L-alanine ;
- la N'-[N-[3β-hydroxylup-20(29)-èn-28-oyl]-8-aminooctanoyl]-L-thréonine ;
- la N'-[N-[3β-hydroxylup-20(29)-èn-28-oyl]-8-aminooctanoyl]-L-statine ;
- l'acide N-[3β-hydroxylup-20(29)-èn-28-oyl]-7-aminoheptylcarbamoylacétique.

Les exemples suivants donnés à titre non limitatif illustrent la présente invention.

## Exemple 1

On ajoute 650 mg de chlorhydrate de 5-aminopentanoate de méthyle puis 1,07 $cm^3$ de triéthylamine à 50 $cm^3$ d'une solution chloroformique de chlorure de 3β-acétoxylup-20(29)èn-28-oyle (préparée à partir de 1,7 g d'acide 3β-acétoxylup-20(29)-èn-28-oïque). La solution est alors agitée 20 heures à une température voisine de 20°C. Après addition de 60 $cm^3$ d'eau distillée la phase organique est décantée et la phase aqueuse est lavée par 40 $cm^3$ au total de chloroforme. Les phases organiques rassemblées sort lavées par 40 $cm^3$ au total d'eau distillée, séchées sur du sulfate de sodium anhydre et concentrées à sec sous pression réduite (2,7 kPa) à une température voisine de 40°C. On obtient 1,9 g d'une meringue jaune qui est chromatographiée sur une colonne de 2,2 cm de diamètre contenant 38 g de silice (0,02-0,045 mm) éluée par de l'oxyde de diisopropyle en recueillant des fractions de 10 $cm^3$. Après élimination des 23 premières fractions, les 17 suivantes sont réunies et concentrées sous pression réduite (2,7 kPa) à une température voisine de 40°C. On obtient ainsi 1,1 g de N-[3β-acétoxylup-20(29)-èn-28-oyl]-5-aminopentanoate de méthyle sous forme d'une meringue blanche (Rf=0,35 ; chromatographie sur couche mince de silice ; éluant : oxyde de diisopropyle).

18 $cm^3$ de soude 4N sont ajoutés à une solution de 1,1 g de N-(3β-acétoxylup-20(29)-èn-28-oyl]-5-aminopentanoate de méthyle dans 18 $cm^3$ de méthanol et 9 $cm^3$ de tétrahydrofurane et le mélange réactionnel est agité pendant 15 heures à une température voisine de 20°C. On dilue par 40 $cm^3$ d'eau distillée et agite de nouveau pendant 15 minutes environ. La suspension est alors acidifiée à un pH voisin de 2 à l'aide de 21 $cm^3$ d'acide chlorhydrique 4N. Après 1 heure d'agitation, le solide est séparé par filtration, lavé 5 fois par 100 $cm^3$ au total d'eau distillée jusqu'à élimination des ions chlorures (test au nitrate d'argent) et séché sous pression réduite (13,5 Pa) à une température voisine de 20°C. On obtient ainsi 820 mg d'acide N-[3β-hydroxylup-20(29)-èn-28-oyl]-5-aminopentanoïque sous la forme d'une meringue blanche fondant vers 160°C.

Le chlorure de 3β-acétoxylup-20(29)-èn-28-oyle est préparé d'après J. PROVITA et A.VYSTRCIL, Collect. Czech. Chem. Commun., 41, 1200 (1976) .

## Exemple 2

On ajoute 0,53 $cm^3$ de triéthylamine et 380 mg de chlorhydrate de 6-aminohexanoate de méthyle à une solution de 1 g de chlorure de 3β-acétoxylup-20(29)-èn-28-oyle en solution dans 50 $cm^3$ de chlorure de méthylène, on maintient l'agitation pendant 20 heures à une température voisine de 20°C, puis on ajoute 100 $cm^3$ d'eau distillée. La phase organique est décantée, la phase aqueuse est extraite par 75 $cm^3$ au total de chlorure de méthylène, les extraits organiques sont rassemblés, séchés sur du sulfate de magnésium anhydre et concentrés à sec sous pression réduite (2,7 kPa) à une température voisine de 40°C. Le résidu obtenu est purifié par HPLC par fractions de 250 mg, sur un colonne de 5 cm de diamètre contenant 200 g de silice greffée $C_{18}$(0,01 mm) éluée avec un mélange d'acétonitrile, d'eau et de tétrahydrofurane 75-10-15 en volumes en recueillant des fractions de 75 $cm^3$. Les 3 premières fractions sont éliminées; les 2 suivantes sont réunies et concentrées sous pression réduite (13,5 Pa) à une température voisine de 35°C. On obtient ainsi 838 mg de N-(3β-acétoxylup-20(29)-èn-28-oyl]-6-aminohexanoate de méthyle sous forme d'une meringue blanche [Rf=0,3 ; chromatographie sur couche mince de silice ; éluant : cyclohexane-acétate d'éthyle (80-20 en volumes)].

Une solution de 800 mg de N-[3β-acétoxylup-20(29)-èn-28-oyl]-6-amino hexanoate de méthyle dans 13 $cm^3$ de méthanol, 6,5 $cm^3$ de tétrahydrofurane et 1,6 $cm^3$ de soude 4N est agitée pendant 15 heures à une température voisine de 20°C. Après addition de 2 $cm^3$ d'acide chlorhydrique 5N et de 20 $cm^3$ d'eau distillée, l'agitation est maintenue pendant 30 minutes. Le solide est séparé par filtration, lavé par 50 $cm^3$ au total d'eau distillée et séché à l'air. On obtient ainsi 750 mg d'acide N-[3β-hydroxylup-20(29)-èn-28-oyl]-6-amino hexanoïque, sous la forme d'un solide blanc, fondant vers 135°C.

## Exemple 3

On ajoute 320 mg de chlorhydrate de 7-aminoheptanoate de méthyle puis 0,62 $cm^3$ de triéthylamine à une solution de 1 g de chlorure de 3β-acétoxylup-20(29)-èn-28-oyle dans 30 $cm^3$ de dichlorométhane. La solution est agitée 20 heures à une température voisine de 20°C et le solvant est évaporé à sec sous pression réduite (2,7 kPa) à une température voisine de 40°C. Les 1,77 g de meringue beige obtenus sont chromatographiés sur une colonne de 3 cm de diamètre contenant 80 g de silice (0,02-0,045 mm) éluée avec un mélange de cyclohexane et d'acétate d'éthyle 80-20 (en volumes) en recueillant des fractions de 60 $cm^3$. Les 23 premières fractions sont éliminées; les 8 suivantes sont réunies et concentrées sous pression réduite (2,7 kPa) à une température voisine de 30°C. On obtient ainsi 930 mg de N-[3β-acétoxylup-20(29)-èn-28-oyl]-7-aminoheptanoate de méthyle sous forme d'une meringue blanche [Rf=0,2 ; chromatographie sur couche mince de silice ;

éluant : cyclohexane-acétate d'éthyle 80-20 (en volumes)].

Un volume de 1,9 $cm^3$ de soude 7N est ajouté en 5 minutes environ à une solution de 930 mg de N-[3β-acétoxylup-20(29)-èn-28-oyl]-7-aminoheptanoate de méthyle dans 7 $cm^3$ de méthanol et 10 $cm^3$ de tétrahydrofurane et le mélange réactionnel est agité pendant 15 heures à une température voisine de 20°C et le solvant est évaporé à sec sous pression réduite (2,7 kPa) à une température voisine de 30°C. Le résidu est dilué dans 40 $cm^3$ d'eau distillée et acidifié à un pH voisin de 2 à l'aide d'acide chlorhydrique 5N. Après 20 minutes d'agitation, le solide est séparé par filtration, lavé par 100 $cm^3$ au total d'eau distillée et séché sous pression réduite (13,5 Pa) à une température voisine de 20°C. On obtient ainsi 740 mg d'acide N-[3β-hydroxylup-20(29)-èn-28-oyl]-7-aminoheptanoïque fondant vers 134°C.

Le 7-aminoheptanoate de méthyle est préparé d'après C.F. HORN, B.T. FREURE, H. VINEYARD, H.J. DECKER, Angew. Chem., 74, 531 (1962).

## Exemple 4

On ajoute 0,53 $cm^3$ de triéthylamine et 490 mg de chlorhydrate de 8-aminooctanoate de méthyle à une solution de 1 g de chlorure de 3β-acétoxylup-20(29)-èn-28-oyle en solution dans 50 $cm^3$ de chlorure de méthylène et maintient l'agitation pendant 20 heures à une température voisine de 20°C. Après addition de 100 $cm^3$ d'eau distillée, la phase organique est décantée, la phase aqueuse est extraite par 75 $cm^3$ au total de chlorure de méthylène, les extraits organiques sont rassemblés, séchés sur du sulfate de magnésium anhydre et concentrés à sec sous pression réduite (2,7 kPa) à une température voisine de 35°C. Le résidu obtenu est purifié par HPLC, par fractions de 250 mg sur une colonne de 5 cm de diamètre contenant 300 g de silice greffée $C_{18}$(0,015-0,020 mm) éluée avec un mélange d'acétonitrile, d'eau et de tétrahydrofurane 70-15-15 (en volumes), sous une pression de 370 Psi en recueillant des fractions de 75 $cm^3$. Les 3 premières fractions sont éliminées ; les 2 suivantes sont réunies et concentrées sous pression réduite (2,7 kPa) à une température voisine de 35°C. On obtient ainsi 750 mg de N-[3β-acétoxylup-20(29)-èn-28-oyl]-8-aminooctanoate de méthyle sous forme d'une meringue blanche [Rf=0,3 ; chromatographie sur couche mince de silice ; éluant : cyclohexane acétate d'éthyle 80-20 en (volumes)].

Une solution de 750 mg de N-[3β-acétoxylup-20(29)-èn-28-oyl]-8-amino octanoate de méthyle dans 12 $cm^3$ de méthanol, 6 $cm^3$ de tétrahydrofurane et 1,5 $cm^3$ de soude 4N est agitée pendant 15 heures à une température voisine de 20°C. Après addition de 10 $cm^3$ de méthanol, de 2$cm^3$ d'acide chlorhydrique 5N puis de 25 $cm^3$ d'eau distillée, l'agitation est maintenue pendant 30 minutes à une température voisine de 20°C et le solide est séparé par filtration, lavé par 50 $cm^3$ au total d'eau distillée, et séché sous pression réduite (13,5 Pa) à une température voisine de 40°C. On obtient ainsi 710 mg d'acide N-[3β-hydroxylup-20(29)-èn-28-oyl]-8-aminooctanoïque, sous la forme d'un solide blanc fondant vers 140°C.

Le chlorhydrate du 8-aminooctanoate de méthyle peut être obtenu de la manière suivante :

A une solution de 1 g d'acide 8-aminooctanoïque dans 40 $cm^3$ de méthanol, on ajoute 0,7 $cm^3$ de chlorure de thionyle, à une température voisine de -20°C et maintient l'agitation pendant 12 heures à une température voisine de 20°C. Le solvant est éliminé sous pression réduite (2,7 kPa) à une température voisine de 35°C. On obtient ainsi 1,48 g de chlorhydrate de 8-aminooctanoate de méthyle, sous la forme d'un solide blanc.

## Exemple 5

On ajoute 310 mg de chlorhydrate de 10-aminodécanoate de méthyle puis 0,35 $cm^3$ de triéthylamine à 11 $cm^3$ d'une solution chloroformique de chlorure de 3β-acétoxylup-20(29)-èn-28-oyle (préparée à partir de 550 mg d'acide 3β-acétoxylup-20(29)-èn-28-oïque). La solution est alors agitée 15 heures à une température voisine de 20°C. Après addition de 15 $cm^3$ d'eau distillée, le mélange réactionnel est agité pendant 1 heure, la phase organique est décantée et la phase aqueuse est extraite par 20 $cm^3$ au total de chloroforme. Les phases organiques rassemblées sont lavées par 20 $cm^3$ au total d'eau distillée, séchées sur du sulfate de sodium anhydre et concentrées à sec sous pression réduite (2,7 kPa) à une température voisine de 30°C. On obtient 730 mg d'une meringue jaune qui est chromatographiée sur une colonne de 2,1 cm de diamètre contenant 35 g de silice (0,02-0,045 mm) éluée avec un mélange de cyclohexane et d'acétate d'éthyle 60-40 (en volumes) en recueillant des fractions de 5 $cm^3$. Après élimination des 21 premières fractions, les 17 suivantes sont réunies et concentrées sous pression réduite (2,7 kPa) à une température voisine de 30°C. On obtient ainsi 600 mg de N-[3β-acétoxylup-20(29)-èn-28-oyl]-10-aminodécanoate de méthyle sous forme d'une meringue blanche [Rf=0,72 ; chromatographie sur couche mince de silice ; éluant : cyclohexane-acétate d'éthyle 60-40 (en volumes)].

8,7 $cm^3$ de soude 4N sont ajoutés à une solution de 600 mg de N-[3β-acétoxylup-20(29)-èn-28-oyl]-10-aminodécanoate de méthyle dans 9 $cm^3$ de méthanol et 4,5 $cm^3$ de tétrahydrofurane et le mélange réactionnel

est agité pendant 15 heures à une température voisine de 20°C. On dilue par 40 $cm^3$ d'eau distillée et acidifie à l'aide de 10 $cm^3$ d'acide chlorhydrique 4N. Après 1 heure d'agitation, on ajoute 15 $cm^3$ d'éther éthylique et isole la phase organique. La phase aqueuse est extraite par 30 $cm^3$ au total d'éther. Les phases organiques rassemblées sont lavées par 30 $cm^3$ au total d'eau distillée, séchées sur du sulfate de sodium anhydre et évaporées à sec sous pression réduite (2,7 kPa) à une température voisine de 30°C. On obtient ainsi 550 mg d'acide N-[3β-hydroxylup-20(29)-èn-28-oyl-10-aminodécanoïque fondant vers 100°C.

Le chlorhydrate de 10-aminodécanoate de méthyle peut être préparé comme décrit dans la demande de brevet allemand 971 392.

## Exemple 6

On ajoute 580 mg de chlorhydrate de 11-aminoundécanoate de méthyle puis 0,62 $cmn^3$ de triéthylamine à une solution de 1,03 g de chlorure de 3β-acétoxylup-20(29)-èn-28-oyle dans 30 $cm^3$ de dichlorométhane. La solution est alors agitée 48 heures à une température voisine de 20°C et le solvant est évaporé à sec sous pression réduite (2,7 kPa) à une température voisine de 20°C. On obtient 2 g d'une meringue jaune qui est chromatographiée sur une colonne de 2,7 cm de diamètre contenant 80 g de silice (0,02-0,045 mm) éluée avec de l'oxyde de diisopropyle en recueillant des fractions de 20 $cm^3$. Après élimination des 10 premières fractions, les 6 suivantes sont réunies et concentrées sous pression réduite (2,7 kPa) à une température voisine de 30°C. On obtient ainsi 1,29 g de N-[3β-acétoxylup-20(29)-èn-28-oyl]-11-undécanoate de méthyle sous forme d'une meringue blanche.

4,5 $cm^3$ de soude 4N sont ajoutés à une solution de 1,28 g de N-[3β-acétoxylup-20(29)-èn-28-oyl]-11-aminoundécanoate de méthyle dans 8 $cm^3$ de méthanol et 13 $cm^3$ de tétrahydrofurane, le mélange réactionnel est agité pendant 24 heures à une température voisine de 20°C et le solvant est évaporé à sec sous pression réduite (2,7 kPa) à une température voisine de 30°C. Le résidu est mis en suspension dans 60 $cm^3$ d'eau distillée. On acidifie à un pH voisin de 2 à l'aide de 3,6 $cm^3$ d'acide chlorhydrique 5N. Après 20 minutes d'agitation, le solide est séparé par filtration, lavé à l'eau distillée jusqu'à un pH voisin de 7 et séché à l'air à une température voisine de 20°C. On obtient ainsi 1,2 g d'acide N-[3β-hydroxylup-20(29)-èn-28-oyl]-11-aminoundécanoïque, sous la forme d'un solide blanc fondant vers 115°C.

Le 11-aminoundécanoate de méthyle est préparé d'après H. ZAHN, J. KUNDE, Chem. Ber., 94, 2470 (1961).

## Exemple 7

On ajoute 243 mg de chlorhydrate de 11-aminoundécanoate de méthyle puis 0,27 $cm^3$ de triéthylamine dans 20 $cm^3$ d'un mélange tétrahydrofurane/ chloroforme 50-50 (en volumes), contenant 453 mg de chlorure de 3α-acétoxylup-20(29)-èn-28-oyle. La solution est agitée 20 heures à une température voisine de 20°C et le solvant est évaporé à sec sous pression réduite (2,7 kPa) à une température voisine de 30°C. Le résidu est repris par 60 $cm^3$ d'éther éthylique et 25 $cm^3$ d'eau distillée, la phase organique est décantée et la phase aqueuse est extraite par 25 $cm^3$ au total d'éther éthylique. Les phases organiques rassemblées sont lavées par 45 $cm^3$ au total d'eau distillée, séchées sur du sulfate de magnésium anhydre et concentrées à sec sous pression réduite (2,7 kPa) à une température voisine de 30°C. Les 600 mg de meringue jaune clair obtenus sont chromatographiés sur une colonne de 2,5 cm de diamètre contenant 56 g de silice (0,063-0,200 mm) éluée avec un mélange de cyclohexane et d'acétate d'éthyle 85-15 (en volumes) en recueillant des fractions de 7 $cm^3$. Les 10 premières fractions sont éliminées; les 17 suivantes sont réunies et concentrées sous pression réduite (2,7 kPa) à une température voisine de 30°C. On obtient ainsi 810 mg de N-[3α-acétoxylup-20(29)-èn-28-oyl]-11-aminoundécanoate de méthyle sous forme d'une meringue blanche.

5,8 $cm^3$ de soude 4N sont ajoutés en 5 minutes environ à une solution de 400 mg de N-[3α-acétoxylup-20(29)-èn-28-oyl]-11-aminoundécanoate de méthyle dans 7 $cm^3$ de méthanol et 3,5 $cm^3$ de tétrahydrofurane et le mélange réactionnel est agité pendant 24 heures à une température voisine de 20°C. On dilue par 35 $cm^3$ d'eau distillée et évapore les solvants organiques sous pression réduite (4,05 kPa) à une température voisine de 30°C. La suspension aqueuse est alors acidifiée à un pH voisin de 1 à l'aide de 6,8 $cm^3$ d'acide chlorhydrique 4N. Après 1 heure d'agitation, on extrait la phase aqueuse avec 50 $cm^3$ d'éther éthylique. La phase organique est décantée et lavée par 45 $cm^3$ au total d'eau distillée, séchée sur du sulfate de magnésium anhydre, et concentrée à sec sous pression réduite (4,05 kPa) à une température voisine de 30°C. On obtient ainsi 384 mg d'une meringue blanchâtre qui est mise en suspension dans 12 $cm^3$ d'oxyde de diisopropyle, séparée par filtration et lavée par 8 $cm^3$ au total d'oxyde de diisopropyle. Le solide (320 mg) est remis en suspension dans 10 $cm^3$ d'oxyde de diisopropyle, on ajoute 3 $cm^3$ d'éther éthylique et on laisse sous agitation pendant 1 heure. Le solide est séparé par filtration, lavé par 10 $cm^3$ au total d'oxyde de diisopropyle et séché à pression réduite

(13,5 Pa) à une température voisine de 35°C. On obtient ainsi 290 mg d'acide N-[3α-hydroxylup-20(29)-èn-28-oyl]-11-aminoundécanoïque, sous la forme d'un solide blanc, fondant vers 220°C.

Le chlorure de 3α-acétoxylup-20(29)-èn-28-oyle peut être préparé de la manière suivante :

Une solution de 267 mg de chlorure d'oxalyle et de 437 mg d'acide 3α-acétylbétulinique dans 14 $cm^3$ de chloroforme est maintenue sous agitation à une température voisine de 20°C pendant 18 heures et le solvant est évaporé sous pression réduite (2,7 kPa) à une température voisine de 30°C. Le résidu est repris dans 20 $cm^3$ de cyclohexane et le solvant est évaporé à sec sous pression réduite dans les mêmes conditions. Cette opération est répétée 2 fois. On obtient ainsi 453 mg de chlorure de 3α-acétoxylup-20(29)-èn-28-oyle.

L'acide 3α-acétoxylup-20(29)-èn-28-oïque peut être obtenu de la manière suivante :

Une solution de 490 mg d'anhydride (3α-acétoxylup-20(29)-èn-28 oïque) acétique dans 49 $cm^3$ d'éthanol à 70 % est chauffé 1 heures 30 minutes au reflux en présence de 4,9 $cm^3$ d'ammoniaque 4N. La suspension est refroidie vers 20°C, l'insoluble est séparé par filtration, lavé par 20 $cm^3$ au total d'éthanol absolu, et les filtrats réunis sont concentrés à sec sous pression réduite (2,7 kPa) à une température voisine de 30°C. On obtient 452 mg d'acide 3α-acétoxylup-20(29)-èn-28-oïque sous la forme d'un solide crème.

L'anhydride (3α-acétoxylup-20(29)-èn-28-oïque) acétique peut être préparé de la manière suivante :

Un mélange de 445 mg d'acide 3α-hydroxylup-20(29)-èn-28-oïque (acide 3α-bétulinique), de 1,14 g d'acétate de sodium et de 4,5 $cm^3$ d'anhydride acétique est chauffé 1 heure 15 minutes au reflux. La solution est ensuite refroidie vers 20°C, puis 15 $cm^3$ d'eau distillée sont ajoutés goutte à goutte, et l'agitation est maintenue pendant 1 heure. Le solide formé est séparé par filtration et lavé à l'aide de 25 $cm^3$ au total d'eau distillée. Le résidu est alors dissous dans 50 $cm^3$ d'éther éthylique, 10 $cm^3$ d'eau distillée sont ajoutés, la phase organique est décantée, lavée par 10 $cm^3$ d'eau distillée, séchée sur du sulfate de magnésium anhydre et concentrée à sec sous pression réduite (2,7 kPa) à une température voisine de 30°C. On obtient ainsi 0500 mg d' anhydride (3α-acétoxylup-20(29)-èn-28-oïque) acétique, sous la forme d'une meringue blanche.

L'acide 3α-bétulinique est obtenu selon W. HERZ, Phytochemistry, 11, 3061 (1972).

## Exemple 8

On ajoute 610 mg de chlorhydrate de 11-aminoundécanoate de méthyle puis 0,68 $cm^3$ de triéthylamine à 20 $cm^3$ d'une solution chloroformique de chlorure de 3-oxolup-20(29)-èn-28-oyle (préparé à partir d'1 g d'acide 3-oxolup-20(29)-èn-28-oïque). La solution est alors agitée 15 heures à une température voisine de 20°C. Après addition de 40 $cm^3$ d'eau distillée la phase organique est décantée et la phase aqueuse est extraite par 30 $cm^3$ au total de chloroforme. Les phases organiques rassemblées sont lavées par 30 $cm^3$ au total d'eau distillée, séchées sur du sulfate de sodium anhydre et concentrées à sec sous pression réduite (2,7 kPa) à une température voisine de 40°C. On obtient 1,5 g d'une meringue jaune qui est chromatographiée sur une colonne de 3,5 cm de diamètre contenant 150 g de silice (0,02-0,045 mm) éluée avec un mélange de chlorure de méthylène et de méthanol 98-2 (en volumes) en recueillant des fractions de 20 $cm^3$. Après élimination des 45 premières fractions, les 30 suivantes sont réunies et concentrées sous pression réduite (2,7 kPa) à une température voisine de 50°C. On obtient ainsi 1,1 g de N-[3-oxolup-20 (29)-èn-28-oyl]-11-aminoundécanoate de méthyle sous forme d'une meringue blanche [Rf=0,5 ; chromatographie sur couche mince de silice ; éluant : dichlorométhane-méthanol 98-2 (en volumes)].

9 $cm^3$ de soude 4N sont ajoutés à une solution de 600 mg de N-[3-oxolup-20(29)-èn-28-oyl]-11-aminoundécanoate de méthyle dans 9 $cm^3$ de méthanol et 4,5 $cm^3$ de tétrahydrofurane et le mélange réactionnel est agité pendant 17 heures à une température voisine de 20°C. On dilue par 20 $cm^3$ d'eau distillée et acidifie à l'aide de 12 $cm^3$ d'acide chlorhydrique 4N. Après 15 minutes d'agitation, la suspension est additionnée de 25 $cm^3$ d'acétate d'éthyle et la phase organique est séparée. La phase aqueuse est extraite par 30 $cm^3$ au total d'acétate d'éthyle, les phases organiques rassemblées sont lavées par 30 $cm^3$ au total d'eau distillée, séchées sur sulfate de sodium anhydre et concentrées à sec sous pression réduite (13,5 Pa) à une température voisine de 30°C. On obtient ainsi 340 mg d'acide N-(3-oxolup-20(29)-èn-28-oyl]-11-aminoundécanoïque, sous la forme d'une poudre blanche fondant vers 90°C.

Le chlorure de 3-oxolup-20(29)-èn-28-oyle peut être préparé de la manière suivante :

A une solution de 2 g d'acide 3-oxolup-20(29)-èn-28-oïque dans 44 $cm^3$ de chloroforme, on ajoute sous agitation 0,76 $cm^3$ de chlorure d'oxalyle et on maintient l'agitation à une température voisine de 20°C pendant 20 heures. Le solvant est alors évaporé à sec sous pression réduite (2,7 kPa) à une température voisine de 35°C. Les traces de chlorure d'oxalyle sont éliminées en mettant le résidu en suspension dans 10 $cm^3$ de cyclohexane et en évaporant à sec le solvant sous pression réduite (2,7 kPa) à une température voisine de 35°C. On obtient 2,2 g de chlorure de 3-oxolup-20(29)-èn-28-oyle sous la forme d'une meringue jaune.

L'acide 3-oxolup-20(29)-èn-28-oïque est préparé d'après R. KAWAGUCHI, K. W. KIM, J. Pharm. Soc. Jpn, 60, 595, 1940.

**Exemple 9**

On ajoute 380 mg de chlorhydrate de 11-aminoundécanoate de méthyle puis 0,56 $cm^3$ de triéthylamine à une solution de 800 mg de chlorure de 30-acétoxy-3-oxolup-20(29)èn-28-oyle dans 40 $cm^3$ de dichlorométhane. La solution est alors agitée 1 heure et 30 minutes à une température voisine de 20°C et le solvant est évaporé à sec sous pression réduite (2,7 kPa) à une température voisine de 35°C. Le résidu obtenu est chromatographié sur une colonne de 4 cm de diamètre et contenant 125 g de silice (0,02-0,045 mm) éluée avec un mélange de cyclohexane et d'acétate d'éthyle 6-4 (en volumes) en recueillant des fractions de 25 $cm^3$. Les 9 premières fractions obtenues sont éliminées, les 6 suivantes sont concentrées à sec sous pression réduite (2,7 kPa) à une température voisine de 45°C. On obtient ainsi 960 mg de N-[30-acétoxy-3-oxolup-20(29)-èn-28-oyl]-11-aminoundéca-noate de méthyle sous forme d'une laque incolore.

On ajoute 1, 6 $cm^3$ de soude 4N à une solution de 930 mg de N-[30-acétoxy-3-oxolup-20(29)-èn-28-oyl]-11-aminoundécanoate de méthyle dans 13 $cm^3$ de méthanol et 6, 5 $cm^3$ de tétrahydrofurane et le mélange réactionnel est agité pendant 15 heures à une température voisine de 20°C. On acidifie à l'aide de 1,3 $cm^3$ d'acide chlorhydrique 5N et on dilue par 100 $cm^3$ d'eau distillée. Après 1 heure d'agitation, le solide est séparé par filtration, lavé par 30 $cm^3$ au total d'eau distillée. On obtient ainsi 730 mg d'acide N-(30-hydroxy-3-oxo lup-20 (29)-èn-28-oyl]-11-aminoundécanoïque fondant à une température voisine de 100°C.

Le chlorure de 30-acétoxy 3-oxolup-20(29)-èn-28-oyle est préparé de la manière suivante :

A une solution de 770 mg d'acide 30-acétoxy-3-oxolup-20(29)-èn-28-oïque dans 38,5 $cm^3$ de dichlorométhane, on ajoute 0,22 $cm^3$ de chlorure d'oxalyle. Après 15 heures d'agitation à une température voisine de 20°C, le solvant est évaporé sous pression réduite (2,7 kPa) à une température voisine de 35°C. On obtient 800 mg de chlorure de 30-acétoxy-3-oxolup-20(29)-èn-28-oyle sous la forme d'une meringue blanche qui est utilisée sans autre purification.

L'acide 30-acétoxy-3-oxolup-20(29)-èn-28-oïque est obtenu de la manière suivante :

On ajoute, goutte à goutte, en 30 minutes environ, une solution de 1,6 g d'anhydride chromique dans 1,6 $cm^3$ d'eau distillée et 16 $cm^3$ de pyridine refroidie à 0°C, à une solution de 2,34 g d'acide 30-acétoxy-3β-hydroxylup-20(29)-èn-28-oïque dans 28 $cm^3$ de pyridine à 0°C. Le mélange réactionnel est agité 1 heure à 0°C, puis 12 heures à une température voisine de 20°C et diluée par 250 $cm^3$ d'eau distillée. On ajoute 100 $cm^3$ d'acétate d'éthyle et la phase organique est décantée. La phase aqueuse est extraite par 2 fois 100 $cm^3$ d'acétate d'éthyle, les phases orga-iques sont rassemblées, lavées par 200 $cm^3$ au total d'eau distillée, séchées sur du sulfate de magnésium anhydre, et évaporées à sec sous pression réduite (2,7 kPa) à une température voisine de 40°C. Le résidu obtenu (2,4 g) est chromatographié sur une colonne de 5 cm de diamètre contenant 200 g de silice (0,020-0,045 mm), éluée par un mélange de cyclohexane et d'acétate d'éthyle 6-4 (en volumes) en recueillant des fractions de 80 $cm^3$. Les 9 premières fractions sont éliminées, les 8 suivantes sont rassemblées et concentrées à sec sous pression réduite (2,7 kPa) à une température voisine de 40°C. On obtient ainsi 1,55 g d'acide 30-acétoxy-3-oxolup-20(29)-èn-28-oïque sous la forme d'une meringue blanche.

L'acide 30-acétoxy-3β-hydroxylup-20(29)-èn-28-oïque peut être préparé de la manière suivante :

Un mélange de 4,44 g d'acide 30-bromo-3β-hydroxylup-20(29)-èn-28-oïque, 2,08 g d'acétate d'argent dans 250 $cm^3$ de toluène est chauffé une demi-heure au reflux. L'insoluble est filtré à chaud et rincé par 75 $cm^3$ au total de toluène bouillant. Les filtrats réunis sont ensuite évaporés sous pression réduite (2,7 kPa). Le résidu (4,32 g) est chromatographié sur une colonne de 6 cm de diamètre et contenant 350 g de silice (0,02-0,045 mm) éluée avec un mélange de cyclohexane et d'acétate d'éthyle 7-3 (en volumes) en recueillant des fractions de 100 $cm^3$. Les 14 premières fractions obtenues sont éliminées, les 11 suivantes sont concentrées sous pression réduite (13,5 Pa) à une température voisine de 45°C. On obtient ainsi 2,34 g d'acide 30-acétoxy-3β-hydroxylup-20(29)-èn-28-oïque sous forme d'une meringue blanche. [Rf=0,26 ; chromatographie sur couche mince de silice ; éluant : cyclohexane-acétate d'éthyle 70-30 (en volumes)].

L'acide 30-bromo 3β-hydroxylup-20(29)-èn-28-oïque est obtenu de la manière suivante :

Une solution de 4 g d'acide 3β-hydroxylup-20(29)-èn-28-oïque et 4,32 g de tribromure de tétrabutylammonium dans 80 $cm^3$ de chloroforme et 80 $cm^3$ de tétrahydrofurane est agitée 2 jours à une température voisine de 25°C. Le mélange réactionnel est lavé successivement 2 fois par 50 $cm^3$ d'eau distillée, 2 fois par 25 $cm^3$ d'une solution de thiosulfate de sodium 0,1N et par 2 fois 25 $cm^3$ d'eau distillée puis séché sur du sulfate de magnésium anhydre. La phase organique est évaporée sous pression réduite (2,7 kPa) et à une température voisine de 40°C. On obtient un solide pâteux jaune (5,01 g) qui est chromatographié sur une colonne de 6 cm de diamètre, contenant 350 g de silice (0,02-0,045 mm) et éluée avec un mélange de chlorure de méthylène et d'acétate d'éthyle 90-10 (en volumes) en recueillant des fractions de 50 $cm^3$. Les 5 premières fractions obtenues sont éliminées, les 70 suivantes sont évaporées sous pression réduite (2,7 kPa) à une température voisine de 40°C. On obtient ainsi 4,44 g d'acide 30-bromo 3β-hydroxy-lup-20(29)-èn-28-oïque sous forme d'un solide blanc. [Rf=0,3 ; chromatographie sur couche mince de silice ; éluant : chlorure de méthylène-acétate

d'éthyle 90-10 (en volumes)].

## Exemple 10

On ajoute 580 mg de chlorhydrate de 11-aminoundécanoate de méthyle puis 0,65 $cm^3$ de triéthylamine à 20 $cm^3$ d'une solution chloroformique de chlorure de 2,3-dioxolup-20(29)-èn-28-oyle (préparée à partir de 1 g d'acide 2,3-dioxolup-20(29)-èn-28-oïque). La solution est agitée 24 heures à une température voisine de 20°C. On ajoute 40 $cm^3$ d'eau distillée, l'agitation est maintenue pendant 15 minutes puis la phase organique est décantée et la phase aqueuse est extraite par 30 $cm^3$ au total de chloroforme. Les phases organiques rassemblées sont lavées par 30 $cm^3$ au total d'eau distillée, séchées sur du sulfate de sodium anhydre et concentrées à sec sous pression réduite (2,7 kPa) à une température voisine de 40°C. On obtient 1,4 g d'une meringue jaune qui est chromatographiée une première fois sur une colonne de 2,7 cm de diamètre contenant 70 g de silice (0,02-0,045 mm) en éluant avec un mélange de cyclohexane et d'acétate d'éthyle 80-20 (en volumes) et en recueillant des fractions de 10 $cm^3$. Après élimination des 33 premières fractions, les 12 suivantes sont réunies et concentrées sous pression réduite (2,7 kPa) à une température voisine de 40°C. Les 700 mg d'huile jaune pâle obtenus sont chromatographiés une nouvelle fois sur une colonne de 2,8 cm de diamètre contenant 70 g de silice (0,020-0,045 mm). On élue avec un mélange de chlorure de méthylène et d'acétate d'éthyle 90-10 (en volumes), en recueillant des fractions de 15 $cm^3$. Les 14 premières fractions sont éliminées; les 7 suivantes sont réunies et concentrées sous pression réduite (2,7 kPa) à une température voisine de 35°C. On obtient 470 mg de N-(2,3-dioxolup-20(29)-èn-28-oyl)-11-aminodécanoate de méthyle sous la forme d'une meringue jaune [[Rf=0,37 ; chromatographie sur couche mince de silice ; éluant : chloroforme-méthanol 95-5 (en volumes)]. 7 $cm^3$ de soude 4N sont ajoutés à une solution de 470 mg de N-[2,3-dioxolup-20(29)-èn-28-oyl]-11-aminoundécanoate de méthyle dans 7 $cm^3$ de méthanol et 3,5 $cm^3$ de tétrahydrofurane et le mélange réactionnel est agité pendant 15 heures à une température voisine de 20°C. On dilue par 15 $cm^3$ d'eau distillée, acidifie à un pH voisin de 3-4 à l'aide de 9 $cm^3$ d'acide chlorhydrique 4N, maintient l'agitation pendant 15 minutes et ajoute 30 $cm^3$ de chloroforme. Après décantation de la phase organique, la phase aqueuse est extraite par 20 $cm^3$ au total de chloroforme. Les phases organiques rassemblées sont lavées par 20 $cm^3$ au total d'eau distillée, séchées sur sulfate de sodium anhydre et concentrées sous pression réduite (2,7 kPa) à une température voisine de 40°C. Les sels minéraux sont éliminés en reprenant le résidu par 20 $cm^3$ d'acétate d'éthyle et 10 $cm^3$ d'acide chlorhydrique 1N. La phase aqueuse est extraite par 20 $cm^3$ au total d'acétate d'éthyle. Les phases organiques rassemblées sont lavées par 30 $cm^3$ au total d'eau distillée, séchées sur sulfate de sodium anhydre et concentrées à sec sous pression réduite (2,7 kPa) à une température voisine de 35°C. On obtient ainsi 420 mg d'une meringue blanche qui est chromatographiée sur une colonne de 2,2 cm de diamètre contenant 40 g de silice (0,060 mm). On élue avec un mélange de chloroforme et de méthanol 95-5 (en volumes) en recueillant des fractions de 5 $cm^3$. Les 16 premières fractions sont éliminées; les 6 suivantes sont réunies et concentrées sous pression réduite (12,5 Pa) à une température voisine de 35°C. On obtient 230 mg d'acide N-[2,3-dioxolup-20(29)-èn-28-oyl]-11-aminoundécanoïque, sous la forme d'une meringue blanche, fondant vers 90°C (collant).

Le chlorure de 2,3-dioxolup-20(29)-èn-28-oyle peut être préparé de la manière suivante :

A une solution de 1 g d'acide 2,3-dioxolup-20(29)-èn-28-oïque dans 21 $cm^3$ de chloroforme, on ajoute sous agitation 0,72 $cm^3$ de chlorure d'oxalyle et on maintient sous agitation à une température voisine de 20°C pendant 18 heures. Le solvant est alors évaporé à sec sous pression réduite (2,7 kPa) à une température voisine de 40°C. Le résidu est mis en suspension dans 10 $cm^3$ de cyclohexane et le solvant est évaporé sous pression réduite (2,7 kPa) à une température voisine de 30°C. Cette opération est répétée 2 fois. On obtient 940 mg de chlorure de 2,3-dioxolup-20(29)-èn-28-oyle sous la forme d'une meringue jaune. [Rf=0,62 ; chromatographie sur couche mince ; éluant cyclohexane-acétate d'éthyle 80-20 (en volumes)].

L'acide 2,3-dioxolup-20(29)-èn-28-oïque est préparé de la manière suivante :

On ajoute 3,48 g de terbutylate de potassium à une solution de 5,27 g d'acide 3-oxolup-20(29)-èn-28-oïque dans 248 $cm^3$ de terbutanol puis on fait passer un courant d'oxygène pendant 23 heures à une température voisine de 25°C. On ajoute alors 31 $cm^3$ d'acide chlorhydrique 1N. Après 15 minutes sous agitation, l'insoluble est éliminé par filtration et le filtrat est évaporé à sec sous pression réduite (2,7 kPa) à une température voisine de 40°C. Le résidu est repris par 50 $cm^3$ de cyclohexane et le solvant est évaporé à sec dans les mêmes conditions; cette opération est répétée 3 fois. Les 7,7 g de meringue obtenus sont chromatographiés sur une colonne de 4,5 cm de diamètre contenant 385 g de silice (0,020-0,045 mm), éluée avec un mélange de cyclohexane et d'acétate d'éthyle 80-20 (en volumes), en recueillant des fractions de 50 $cm^3$. Les 23 premières fractions sont éliminées, les 9 suivantes sont rassemblées et concentrées à sec sous pression réduite (2,7 kPa) à une température voisine de 40°C. On obtient ainsi 3,3 g d'acide 2,3-dioxolup-20(29)-èn-28-oïque, sous la forme d'une meringue jaune. [Rf=0,2 ; chromatographie sur couche mince de silice ; éluant : cyclohexane-acétate

d'éthyle 80-20 (en volumes)].

**Exemple 11**

On ajoute 280 mg de chlorhydrate de 11-aminoundécanoate de méthyle et 0,28 $cm^3$ de triéthylamine à 25 $cm^3$ d'une solution dans le dichlorométhane de chlorure de 3β,30-diacétoxylup-20(29)-èn-28-oyle (préparée à partir de 560 mg d'acide 3β,30-diacétoxylup-20(29)-èn-28-oïque). La solution est alors agitée 12 heures à une température voisine de 20°C. On ajoute 10 $cm^3$ d'eau distillée. La phase organique est séparée puis lavée avec 20 $cm^3$ au total d'eau distillée. Les phases organiques réunies sont séchées sur du sulfate de magnésium anhydre et concentrées à sec sous pression réduite (2,7 kPa) à une température voisine de 35°C. L'huile incolore obtenue (750 mg) est chromatographiée sur une colonne de 1,7 cm de diamètre et contenant 15 g de silice (0,02-0,045 mm) éluée avec un mélange de cyclohexane et d'acétate d'éthyle 80-20 (en volumes) en recueillant des fractions de 15 $cm^3$. Les 2 premières fractions obtenues sont éliminées, les 5 suivantes sont concentrées à sec sous pression réduite (22 kPa) à une température voisine de 40°C. On obtient ainsi 650 mg de N-[3β,30-di-acétoxylup-20(29)-èn-28-oyl]-11-aminoundéca-noate de méthyle sous forme d'une laque blanche [Rf=0,28 ; chromatographie sur couche mince de silice ; éluant : cyclohexane acétate d'éthyle 80-20 (en volumes)].

10 $cm^3$ de soude 4N sont ajoutés à une solution de 650 mg de N-[3β,30-diacétoxylup-20(29)-èn-28-oyl]-11-aminoundécanoate de méthyle 10 $cm^3$ de méthanol et 5 $cm^3$ de tétrahydrofurane et le mélange réactionnel est agité pendant 18 heures à une température voisine de 20°C. On acidifie à un pH voisin de 1-2 à l'aide d'une solution d'acide chlorhydrique 5N. Après 1 heure d'agitation, le solide est séparé par filtration, lavé par 30 $cm^3$ au total d'eau distillée. Le solide obtenu est mis en solution dans un mélange de dichlorométhane et d'éthanol 90-10 (en volumes). L'insoluble est filtré et les filtrats sont concentrés sous pression réduite (13,5 Pa) à une température voisine de 40°C. On obtient ainsi une huile qui est agitée ave 20 $cm^3$ d'éther éthylique. Après 12 heures d'agitation à une température voisine de 20°C, le solide formé est filtré puis rincé par 10 $cm^3$ au total d'éther éthylique et séché sous pression réduite (13,5 Pa). On obtient ainsi 400 mg d'acide N-(3β,30-dihydroxylup-20(29)-èn-28-oyl]-11-aminoundécanoïque fondant à une température voisine de 174°C.

Le chlorure de 3β,30-diacétoxylup-20(29)-èn-28-oyle est préparé de la manière suivante :

A une solution de 560 mg d'acide 3β,30-diacétoxylup-20(29)-èn-28-oïque dans 25 $cm^3$ de dichlorométhane, on ajoute 0,22 $cm^3$ de chlorure d'oxalyle. Après 15 heures d'agitation à une température voisine de 20°C, le solvant est évaporé sous pression réduite (22 kPa) et à une température voisine de 40°C. On obtient 600 mg d'une meringue blanche qui est utilisée sans autre purification.

L'acide 3β,30-diacétoxylup-20(29)-èn-28-oïque est obtenu de la manière suivante :

Une suspension de 1,8 g d'acide 3β-acétoxy-30-bromolup-20(29)-èn-28-oïque et 800 mg d'acétate d'argent dans 50 $cm^3$ de toluène est agitée 48 heures à une température voisine de 20°C puis filtrée. La solution obtenue est ensuite concentrée à sec sous pression réduite (2 kPa). Le résidu est mis en solution dans 50 $cm^3$ d'acétate d'éthyle. La phase organique est décantée et lavée par 60 $cm^3$ au total d'eau distillée, séchée sur du sulfate de magnésium anhydre, puis concentrée sous pression réduite (2,7 kPa) à une température vosine de 40°C. Le solide blanc obtenu (1,7 g) est chromatographié sur une colonne de 1,7 cm de diamètre et contenant 35 g de silice (0,02-0,045 mm) éluée avec un mélange de cyclohexane et d'acétate d'éthyle 80-20 (en volumes), en recueillant des fractions de 15 $cm^3$. Les 4 premières fractions obtenues sont éliminées, les 10 suivantes sont concentrées à sec sous pression réduite (13,5 Pa) à une température voisine de 40°C. On obtient ainsi 1,1 g d'acide 3β,30-diacétoxylup-20 (29)-èn-28-oïque sous forme d'une meringue blanche suffisamment pure pour les transformations ultérieures.

L'acide 3β-acétoxy-30-bromolup-20(29)-èn-28-oïque est obtenu de la manière suivante :

Une solution de 2,5 g d'acide 3β-acétoxy-lup-20(29)-èn-28-oïque et 2,41 g de tribromure de tétrabutylammonium dans 50 $cm^3$ de chloroforme est agitée 5 jours à une température voisine de 25°C. Le mélange réactionnel est lavé successivement 2 fois par 25 $cm^3$ d'eau distillée, 2 fois par 25 $cm^3$ d'une solution de thiosulfate de sodium 0,1N et par 2 fois 25 $cm^3$ d'eau distillée puis séché sur du sulfate de magnésium anhydre. La phase organique est concentrée à sec sous pression réduite (2,7 kPa) et à une température voisine de 40°C. On obtient un solide pâteux jaune (4 g) qui est chromatographié sur une colonne de 3,2 cm de diamètre contenant 100 g de silice (0,02-0,045 mm) et éluée avec un mélange de cyclohexane et d'acétate d'éthyle 85-15 (en volumes) en recueillant des fractions de 20 $cm^3$. Les 5 premières fractions obtenues sont éliminées, les 10 suivantes sont évaporées sous pression réduite (13,5 Pa) à une température voisine de 40°C. On obtient ainsi 2,7 g d'acide 3β-acétoxy-30-bromolup-20(29)-èn-28-oïque sous forme d'un solide blanc fondant à une température voisine de 190°C.

L'acide 3β-acétoxylup-20(29)-èn-28-oïque peut être préparé selon Bruckner, Kovacs et Koczka, J. Chem. Soc., 948 (1948).

**Exemple 12**

Après avoir chauffé au reflux pendant 3 heures un mélange de 190 mg d'acide 11-aminoundécanoïque et 308 mg de chlorotriméthylsilane dans 50 $cm^3$ de dichlorométhane, la solution est refroidie vers 20°C et on ajoute 750 mg de chlorure de 30-acétoxy-3-oxolup-20(29)-èn-28-oyle puis 0,72 $cm^3$ de triéthylamine. L'agitation est maintenue pendant 48 heures à une température voisine de 20°C et le solvant est évaporé à sec sous pression réduite (2,7 kPa) à une température voisine de 40°C. Le résidu est chromatographié sur une colonne de 4 cm de diamètre contenant 125 g de silice (0,02-0,045 mm), éluée en présence du mélange chlorure de méthylène méthanol 80-20 (en volumes) en recueillant des fractions de 50 $cm^3$. Les 20 premières fractions sont éliminées, les 20 suivantes sont rassemblées et concentrées à sec sous pression réduite (2,7 kPa) à une température voisine de 40°C. On obtient ainsi 330 mg d'acide N-[30-acétoxy-3-oxolup-20(29)-èn-28-oyl]-11-aminoundécanoïque sous la forme d'une meringue blanche.

A une solution de 330 mg d'acide N-[30-acétoxy-3-oxolup-20(29)-èn-28-oyl ]-11-aminoundécanoïque dans 13 $cm^3$ de méthanol, on ajoute 90 mg de cyanoborohydrure de sodium puis goutte à goutte, en 5 minutes environ, 1,46 $cm^3$ d'une solution aqueuse à 15 % de chlorure de titane III. La solution est maintenue sous agitation pendant 15 heures à une température voisine de 20°C et on ajoute 75 $cm^3$ d'eau distillée, 50 $cm^3$ d'acétate d'éthyle et 10 $cm^3$ d'acide chlorhydrique 1N. La phase organique est séparée, la phase aqueuse est extraite par 30 $cm^3$ au total d'acétate d'éthyle, les extraits organiques sont rassemblés, séchés sur du sulfate de magnésium anhydre et évaporés sous pression réduite (2,7 kPa) à une température voisine de 40°C. Le résidu obtenu (370 mg) est chromatographié sur une colonne de 4 cm de diamètre contenant 125 g de silice (0,02-0,045 mm) éluée avec un mélange de cyclohexane et d'acétate d'éthyle 60-40 (en volumes) en recueillant des fractions de 50 $cm^3$. Les 10 premières fractions sont éliminées ; les 7 suivantes sont réunies et concentrées sous pression réduite (2,7 kPa) à une température voisine de 40°C. Le solide blanc obtenu est dissous dans 0,5 $cm^3$ d'éthanol et on dilue avec 22 $cm^3$ d'eau distillée. Après 10 minutes d'agitation, le solide est séparé par filtration, lavé par 20 $cm^3$ au total d'eau distillée et séché à l'air. On obtient 260 mg de N-[3β,30-dihydroxylup-20(29)-èn-28-oyl]-11-aminoundécanoate de méthyle sous forme d'un solide blanc, fondant vers 80°C.

**Exemple 13**

A une solution de 150 mg d'acide 20,29,30-trinor 3β-acétoxy-19-[(2-acétoxyéthyl) carbamoyl]lupan-28-oïque dans 10 $cm^3$ de chloroforme, on ajoute une solution de 0,03 $cm^3$ de chlorure d'oxalyle dans 5 $cm^3$ de chloroforme. Le mélange réactionnel est agité 12 heures à une température voisine de 20°C puis concentré sous pression réduite (15 kPa). Le résidu est repris par 5 $cm^3$ de chloroforme. A la solution obtenue, on ajoute 80 mg de chlorhydrate de 11-aminoundécanoate de méthyle et 0,09 $cm^3$ de triéthylamine. La solution est alors agitée 12 heures à une température voisine de 20°C. On ajoute 10 $cm^3$ d'eau distillée puis on amène le mélange réactionnel à un pH voisin de 2 au moyen d'une solution d'acide chlorhydrique 4N. La phase organique est séparée et la phase aqueuse est extraite 2 fois 15 $cm^3$ de chloroforme. Les phases organiques réunies sont lavées par 60 $cm^3$ au total d'eau distillée puis séchées 3 heures sur du sulfate de magnésium anhydre et filtrées. La phase organique est concentrée à sec sous pression réduite (10 kPa) à une température voisine de 40°C. Les 200 mg de meringue jaune pâle obtenus sont filtrés sur une colonne de 2,7 cm de diamètre, contenant 50 g de silice, (0,02-0,045 mm) éluée par un mélange de chloroforme, de méthanol et d'ammoniaque à 28 % 24-6-1 (en volumes). On concentre à sec le filtrat sous pression réduite (13,5 Pa). On obtient ainsi 120 mg de N-{20,29,30-trinor 3β-acétoxy-19-[(2-acétoxyéthyl)carbamoyl]lupan-28-oyl}-11-aminoundécanoate de méthyle sous forme d'une laque blanche [Rf=0,90 chromatographie sur couche mince de silice ; éluant : chloroforme, méthanol, ammoniaque à 28 % 24-6-1 (en volumes)].

5 $cm^3$ de soude 4N sont ajoutés à une solution de 200 mg de N-{20,29,30-trinor 3β-acétoxy-19-[(2-acétoxyéthyl)carbamoyl]lupan-28-oyl}-11-aminoundécanoate de méthyle dans 10 $cm^3$ de méthanol et 5 $cm^3$ de tétrahydrofurane et le mélange réactionnel est agité pendant 15 heures à une température voisine de 20°C. Le mélange réactionnel est ensuite acidifié à un pH voisin de 2 à l'aide d'acide chlorhydrique 4N et dilué par 50 $cm^3$ d'eau distillée. Après 2 heures sous agitation à une température voisine de 20°C, le solide est séparé par filtration, lavé par 20 $cm^3$ au total d'eau distillée puis mis en solution dans 10 $cm^3$ d'éthanol. La solution obtenue est filtrée puis concentrée à sec sous pression réduite (13,5 Pa) à une température voisine de 20°C. Le solide est repris trois fois par 15 $cm^3$ d'éthanol et la solution est concentrée à sec sous pression réduite. Le résidu solide est mis en suspension dans 50 $cm^3$ de chloroforme. La suspension est portée à une température voisine de 40°C et filtrée à chaud. Le filtrat est concentré sous pression réduite (20 kPa) à une température voisine de 40°C. Le résidu est trituré avec 5 $cm^3$ d'oxyde de diisopropyle, filtré puis rincé avec 6 $cm^3$ d'oxyde de diisopropyle. Le solide est séché sous pression réduite (1 kPa) à une température voisine de 40°C. On obtient ainsi 90 mg d'acide N-{20,29,30-trinor 3β-hydroxy-19-[(2-hydroxyéthyl)carbamoyl]lupan-28-oyl}-11-aminoundécanoïque sous la forme d'un solide blanc fondant à une température voisine de 170°C.

L'acide 20,29,30-trinor 3β-acétoxy-19-[2-acétoxyéthyl)carbamoyl]lupan-28-oïque peut être obtenu de la manière suivante :

A une solution de 450 mg d'acide 20,29,30-trinor 3β-acétoxy-19-[(2-hydroxyéthyl) carbamoyl]lupan-28-oïque dans 60 $cm^3$ de chloroforme on ajoute 30 mg de 4-diméthylaminopyridine, 0,35 $cm^3$ de triéthylamine et 0,1 $cm^3$ d'anhydride acétique. Le mélange réactionnel est agité 72 heures à une température voisine de 20°C puis amené à un pH voisin de 1-2 au moyen d'une solution d'acide chlorhydrique 4N. La phase organique est décantée puis lavée par 30 $cm^3$ au total d'eau distillée, séchée 1 heure sur du sulfate de magnésium anhydre et concentrée sous pression réduite (40,5 kPa) à une température voisine de 40°C. Le résidu solide est trituré avec 5 $cm^3$ d'oxyde de diisopropyle, filtré, rincé par 4 $cm^3$ au total d'oxyde de diisopropyle puis séché sous pression réduite (100 Pa) à une température voisine de 40°C. On obtient ainsi 350 mg d'acide 20,29,30-trinor 3β-acétoxy-19-[(2-acétoxyéthyl)carbamoyl]lupan-28-oïque sous la forme d'un solide blanc fondant à une température voisine de 190°C.

L'acide 20,29,30-trinor 3β-acétoxy-19-[(2-hydroxyéthyl)carbamoyl]lupan-28-oïque peut être obtenu de la manière suivante :

A une solution de 500 mg d'acide 29,30-dinor 3β-acétoxylupan-20,28-dioïque dans 50 $cm^3$ de chloroforme on ajoute 0,11 $cm^3$ de chlorure d'oxalyle. Le mélange est agité durant 12 heures à une température voisine de 20°C, puis il est concentré sous pression réduite (20 kPa) à une température voisine de 40°C. Le solide résiduel est mis en solution dans 15 $cm^3$ de chloroforme et on ajoute, en 10 minutes, une solution de 0,18 $cm^3$ d'éthanolamine dans 5 $cm^3$ de chloroforme. Après 3 jours d'agitation à une température voisine de 20°C, on ajoute 20 $cm^3$ d'eau distillée puis on amène à un pH voisin de 1-2 au moyen d'une solution d'acide chlorhydrique 4N. La phase organique est décantée, lavée par 80 $cm^3$ au total d'eau distillée, séchée sur du sulfate de magnésium anhydre, filtrée puis évaporée sous pression réduite (20 kPa) à une température voisine de 40°C. Les 600 mg de meringue jaune pâle obtenue sont chromatographiés sur une colonne de 1,8 cm de diamètre, contenant 20 g de silice (0,02-0,045 mm), éluée par un mélange de dichlorométhane et de méthanol 95-5 (en volumes). Les 7 premières fractions sont éliminées, les 12 suivantes sont concentrées à sec sous pression réduite (13,5 Pa). On obtient ainsi 230 mg d'acide 20,29,30-trinor 3β-acétoxy-19-[(2-hydroxyéthyl)carbamoyl]lupan-28-oïque sous forme d'une meringue blanche [Rf=0,30 chromatographie sur couche mince de silice ; éluant : dichlorométhane méthanol 95-5(en volumes)].

L'acide 29,30-dinor 3β-acétoxylupan-20,28-dioïque peut être obtenu selon la méthode décrite par J.M. GUIDER et coll., J. Chem. Soc., 3024 (1953).

## Exemple 14

A une solution de 300 mg d'acide 20,29,30-trinor 3β-acétoxy-19-(2-acétoxy-1-méthoxycarbonyléthylcarbamoyl) lupan-28-oïque dans 20 $cm^3$ de chloroforme, on ajoute en 1 minute une solution de 0,05 $cm^3$ de chlorure d'oxalyle dans 5 $cm^3$ de chloroforme. Le mélange réactionnel est agité 12 heures à une température voisine de 20°C puis concentré sous pression réduite (25 kPa). Le résidu est repris par 15 $cm^3$ de chloroforme. A la solution obtenue, on ajoute 140 mg de chlorhydrate de 11-amino-undécanoate de méthyle et 0,15 $cm^3$ de triéthylamine. La solution est alors agitée 12 heures à une température voisine de 20°C. On ajoute 10 $cm^3$ d'eau distillée puis on amène le mélange réactionnel à un pH voisin de 1 au moyen d'une solution d'acide chlorhydrique 4N. La phase organique est séparée et la phase aqueuse est extraite par 2 fois 10 $cm^3$ de chloroforme. Les phases organiques réunies sont lavées par 30 $cm^3$ au total d'eau distillée puis séchées 3 heures sur du sulfate de magnésium et filtrées. La phase organique est concentrée à sec sous pression réduite (25 kPa) à une température voisine de 40°C. Les 450 mg de laque jaune pâle obtenus sont chromatographiés sur une colonne de 1,7 cm de diamètre, contenant 20 g de silice (0,02-0,045 mm) éluée au moyen d'un mélange de dichlorométhane et de méthanol 97,5-2,5 (en volumes). On récolte des fractions de 10 $cm^3$. Les 10 premières fractions sont éliminées, les 15 suivantes sont concentrées à sec sous pression réduite (13,5 Pa). Les 300 mg de solide obtenus sont chromatographiés sur une colonne de 2,8 cm de diamètre, contenant 50 g de silice (0,02-0,045 mm), éluée au moyen d'un mélange de chloroforme, de méthanol et d'ammoniaque à 28 % 24-12-1 (en volumes). On récolte des fractions de 10 $cm^3$. Les 7 premières fractions sont éliminées, les 10 suivantes sont concentrées à sec sous pression réduite (13,5 Pa). On obtient ainsi 200 mg de N-[20,29,30-trinor 3β-acétoxy-19-(2-acétoxy-1-méthoxycarbonyléthylcarbamoyl)lupan-28-oyl]-11-aminoundécanoate de méthyle sous la forme d'une laque blanche [Rf=0,80 chromatographie sur couche mince de silice ; éluant : chloroforme, méthanol, ammoniaque à 28 % 24-6-1 (en volumes)].

5 $cm^3$ de soude 4N sont ajoutés à une solution de 200 mg de N-[20,29,30-trinor 3β-acétoxy-19-(2-acétoxy-1-méthoxycarbonyléthylcarbamoyl)lupan-28-oyl]-11-aminoundécanoate) de méthyle dans 10 $cm^3$ de méthanol et 5 $cm^3$ de tétrahydrofurane et le mélange réactionnel est agité pendant 15 heures à une température voisine de 20°C. Le mélange réactionnel est ensuite acidifié à un pH voisin de 2 à l'aide d'acide chlorhydrique 4N. On dilue par 50 $cm^3$ d'eau distillée. On agite pendant 2 heures à une température voisine de 20°C. Le solide est séparé par filtration, lavé par 25 $cm^3$ au total d'eau distillée puis séché à l'air. Les 160 mg de solide sont chromatographiés sur une colonne de 1,2 cm de diamètre, contenant 5 g de silice (0,02-0,045 mm) au moyen d'un mélange de chloroforme, de méthanol et d'ammoniaque à 28 % 12-6-1 (en volumes), en recueillant des fractions de 10 $cm^3$. Les 9 premières fractions sont éliminées, les 13 suivantes sont concentrées à sec sous pression réduite (13,5 Pa) à une température voisine de 40°C. Le résidu est trituré avec 5 $cm^3$ d'oxyde de diéthyle, filtré, rincé par 6 $cm^3$ au total d'oxyde de diéthyle puis séché sous pression réduite (10 kPa). Les 80 mg de solide obtenus sont mis en solution dans 2 $cm^3$ de dioxane puis on ajoute 0,5 $cm^3$ d'une solution aqueuse d'acide chlorhydrique 4N et 15 $cm^3$ d'eau distillée. Le solide est filtré, lavé 4 fois par 2 $cm^3$ d'eau distillée puis mis en solution dans 10 $cm^3$ d'éthanol. La solution obtenue est filtrée puis évaporée sous pression réduite (8 kPa) à une température voisine de 35°C. L'addition d'éthanol et l'évaporation est répétée 3 fois. Le résidu est mis en solution dans un mélange de 30 $cm^3$ de chloroforme et de 3 $cm^3$ de tétrahydrofurane. La solution est filtrée puis concentrée sous pression réduite (8 kPa) à une température voisine de 40°C. Le résidu est trituré avec 5 $cm^3$ d'oxyde de diisopropyle, filtré, rincé 3 fois par 2 $cm^3$ d'oxyde de diisopropyle, puis séché sous pression réduite (13,5 Pa). On obtient ainsi 50 mg d'acide N-[20,29,30-trinor 19-(1-carboxy-2-hydroxyéthylcarbamoyl)-3β-hydroxylupan-28-oyl]-11-aminoundécanoïque sous la forme d'un solide blanc fondant à une température voisine de 208°C.

L'acide 20,29,30-trinor 3β-acétoxy-1-(2-acétoxy-19-méthoxycarbonyléthylcarbamoyl)lupan-28-oïque peut être obtenu de la manière suivante :

A une solution de 300 mg d'acide 20,29,30-trinor 3β-acétoxy-19-(2-hydroxy-1-méthoxycarbonyléthylcarbamoyl)lupan-28-oïque dans 25 $cm^3$ de chloroforme on ajoute 30 mg de 4-diméthylaminopyridine et 0,35 $cm^3$ de triéthylamine et 0,21 $cm^3$ d'anhydride acétique. Le mélange réactionnel est agité 12 heures à une température voisine de 20°C. On ajoute 20 $cm^3$ d'eau distillée puis on amène à un pH voisin de 2-3 au moyen d'une solution d'acide chlorhydrique 4N. La phase organique est décantée puis lavée par 60 $cm^3$ au total d'eau distillée, séchée 1 heure sur du sulfate de magnésium anhydre et concentrée sous pression réduite (15 kPa) à une température voisine de 40°C. A l'huile incolore obtenue, on ajoute 2 $cm^3$ de dioxane et 20 $cm^3$ d'eau. On triture et on refroidit à une température voisine de 0°C. Le solide est filtré, lavé au moyen de 3 fois 5 $cm^3$ d'eau distillée puis séché sous pression réduite (100 Pa) à une température voisine de 40°C. On obtient ainsi 300 mg d'acide 20,29,30-trinor 3β-acétoxy-19-(2-acétoxy-1-méthoxycarbonyléthylcarbamoyl) lupan-28-oïque sous la forme d'un solide blanc fondant à une température voisine de 180°C.

L'acide 20,29,30-trinor 3β-acétoxy-19-(2-hydroxy-1-méthoxycarbonyléthylcarbamoyl)lupan-28-oïque peut être obtenu de la manière suivante :

A une solution de 1,8 g d'acide 29,30-dinor 3β-acétoxylupan-20,28-dioïque dans 150 $cm^3$ de chloroforme on ajoute 0,38 $cm^3$ de chlorure d'oxalyle. Le mélange est agité durant 12 heures à une température voisine de 20°C, puis concentré sous pression réduite (20 kPa) à une température voisine de 40°C. Le solide résiduel est mis en solution dans 40 $cm^3$ de chloroforme et on ajoute en 10 minutes une solution de 700 mg de chlorhydrate de L-sérinate de méthyle et 1,5 $cm^3$ de triéthylamine. Après 12 heures d'agitation à une température voisine de 20°C, on ajoute 50 $cm^3$ d'eau distillée puis on amène à un pH voisin de 1-2 au moyen d'une solution d'acide chlorhydrique 4N. La phase organique est décantée, lavée par 30 $cm^3$ d'eau distillée, séchée sur du sulfate de magnésium anhydre et concentrée à sec sous pression réduite (20 kPa) à une température voisine de 40°C. Les 600 mg de meringue jaune pâle obtenus sont chromatographiés sur une colonne de 2,4 cm de diamètre, contenant 50 g de silice (0,02-0,045 mm), éluée au moyen d'un mélange acétate d'éthyle/dichlorométhane 1-1 (en volumes). Les 14 premières fractions sont éliminées, les 9 suivantes sont concentrée à sec sous pression réduite (13,5 Pa). On obtient ainsi 800 mg d'un solide qui est trituré avec 10 $cm^3$ d'acétonitrile, filtré, lavé avec 2 fois 2 $cm^3$ d'acétonitrile et séché sous pression réduite (13,5 Pa). On obtient ainsi 800 mg d'acide 20,29,30-trinor 3β-acétoxy-19-(2-hydroxy-1-méthoxycarbonyl-éthylcarbamoyl)lupan-28-oïque sous forme d'une meringue blanche fondant à une température voisine de 230°C.

## Exemple 15

2,2 $cm^3$ de triéthylamine et 1,94 g de chlorhydrate de 11-amino undécanoate de méthyle sont ajoutés à 195 $cm^3$ d'une solution chlorométhylénique de chlorure de 30-nor 3β-acétoxy-20-oxolupan-28-oyle. La solution est alors agitée 72 heures à une température voisine de 20°C. Le mélange réactionnel est dilué par 100 $cm^3$ de dichlorométhane, la phase organique est lavée par 3 fois 150 $cm^3$ d'eau distillée. Les phases organiques rassemblées sont séchées 3 heures sur du sulfate de magnésium et filtrées. Les phases organiques sont concentrées à sec sous pression réduite (2,7 kPa) à une température voisine de 50°C. L'huile jaune pâle obtenue (5,7 g) est chromatographiée sur une colonne de 4 cm de diamètre, 60 cm de haut et contenant de la

silice (0,02-0,045 mm) éluée successivement avec un mélange de cyclohexane et d'acétate d'éthyle 70-30 (en volumes) pour les fractions 1 à 50 et un mélange de cyclohexane et d'acétate d'éthyle 50-50 (en volumes) pour les fractions suivantes. On recueille des fractions de 100 $cm^3$. Les 30 premières fractions sont éliminées; les 32 suivantes sont réunies et concentrées sous pression réduite (2,7 kPa) à une température voisine de 35°C. On obtient ainsi 5 g d'une meringue blanche qui est triturée avec 2 fois 100 $cm^3$ d'oxyde de diisopropyle puis séchée sous pression réduite (2,7 kPa) à une température voisine de 40°C. On obtient ainsi 4,75 g de N-(30-nor 3β-acétoxy-20-oxolupan-28-oyl)-11-aminoundécanoate de méthyle sous forme d'une meringue blanche (Rf=0,23 ; chromatographie sur couche mince de silice ; éluant : cyclohexane acétate d'éthyle 8-2 (en volumes)].

10 $cm^3$ de soude 5N sont ajoutés à une solution de 700 mg de N-(30-nor 3β-acétoxy-20-oxolupan-28-oyl)-11-aminoundécanoate de méthyle dans 15 $cm^3$ de méthanol et 15 $cm^3$ de tétrahydrofurane et le mélange réactionnel est agité pendant 48 heures à une température voisine de 20°C. On ajoute successivement à trois reprises et à 24 heures d'intervalle un mélange de 10 $cm^3$ de méthanol et de 10 $cm^3$ de tétrahydrofurane en laissant agiter le mélange réactionnel à une température voisine de 20°C. Le mélange réactionnel est ensuite acidifié à un pH voisin de 2 à l'aide d'acide chlorhydrique 4N. On ajoute 150 $cm^3$ d'eau distillée. Après 2 heures sous agitation à une température voisine de 20°C, le solide est séparé par filtration, lavé par 180 $cm^3$ au total d'eau distillée et séché sous pression réduite (13,5 Pa) à une température voisine de 20°C. On obtient ainsi 500 mg d'un solide blanc. La phase aqueuse est extraite par 200 $cm^3$ au total de dichlorométhane. Les phases organiques réunies sont séchées sur sulfate de magnésium anhydre puis évaporées sous pression réduite (13,5 Pa) à une température voisine de 20°C. On obtient ainsi 150 mg d'une huile jaune pâle. Le solide et l'huile sont réunis et chromatographiés sur une colonne de 2 cm de diamètre, 40 cm de haut, contenant de la silice (0,02-0,045 mm) éluée avec un mélange de cyclohexane et d'acétate d'éthyle 50-50 (en volumes), en recueillant des fractions de 10 $cm^3$. Les 15 premières fractions sont éliminées ; les 10 suivantes sont réunies et concentrées sous pression réduite (2,7 kPa) à une température voisine de 35°C. On obtient ainsi 400 mg d'une huile jaune pâle. Cette huile est dissoute dans 1,5 $cm^3$ de tétrahydrofurane. Après 20 minutes sous agitation à une température voisine de 20°C, on ajoute à 20 minutes d'intervalle 1,5 $cm^3$ et 10 $cm^3$ d'oxyde de diisopropyle. Le solide est séparé par filtration et séché sous pression réduite (13,5 Pa) à une température voisine de 40°C. On obtient ainsi 200 mg d'acide N-(30-nor 3β-acétoxy-20-oxolupane-28-oyl)-11-aminoundécanoïque, sous la forme d'un solide blanc fondant vers 150°C.

Le chlorure de 30-nor 3β-acétoxy-20-oxolupan28-oyle est obtenu de la manière suivante :

1,5 $cm^3$ de chlorure d'oxalyle est ajouté en 5 minutes à 150 $cm^3$ d'une solution, dans le dichlorométhane, d'acide 30-nor 3β-acétoxy-20-oxolupanoïque. La solution est alors agitée 20 heures à une température voisine de 20°C. Le mélange réactionnel est concentré à sec sous pression réduite (2,7 kPa) à une température voisine de 40°C. La meringue blanche obtenue est triturée avec 2 fois 200 $cm^3$ de cyclohexane. Le solide est filtré et séché sous pression réduite (2,7 kPa) à une température voisine de 40°C. On obtient ainsi 3,7 g de chlorure de 30-nor 3β-acétoxy-20-oxolupan-28-oyle sous la forme d'une meringue blanche.

L'acide 30-nor 3β-acétoxy-20-oxolupan-28-oïque est préparé d'après A. VYSTRCIL et M. BUDESINSY, Collect. Czech. Chem. Commun., 35, 295 (1970).

## Exemple 16

On ajoute 370 mg de chlorhydrate de 12-aminododécanoate de méthyle puis 0,4 $cm^3$ de triéthylamine à une solution de 660 mg de chlorure de 3β-acétoxylup-20(29)-èn-28-oyle dans 13,2 $cm^3$ de chloroforme et 13 $cm^3$de tétrahydrofurane. La solution est agitée 20 heures à une température voisine de 20°C et le solvant est évaporé sous pression réduite (2,7 kPa) à une température voisine de 35°C. Le résidu est additionné de 60 $cm^3$ d'éther éthylique et 25 $cm^3$ d'eau distillée. La phase organique est décantée et la phase aqueuse est extraite par 40 $cm^3$ au total d'éther éthylique. Les phases organiques rassemblées sont lavées par 60 $cm^3$ au total d'eau distillée, séchées sur sulfate de sodium anhydre et concentrées à sec sous pression réduite (2,7 kPa) à une température voisine de 30°C. Les 860 mg de meringue de couleur crème obtenus sont chromatographiés sur une colonne de 2 cm de diamètre contenant 52 g de silice (0,02-0,045 mm) éluée avec un mélange de cyclohexane et d'acétate d'éthyle 80-20 (en volumes) en recueillant des fractions de 7 $cm^3$. Les 7 premières fractions sont éliminées; les 7 suivantes sont réunies et concentrées sous pression réduite (2,7 kPa) à une température voisine de 35°C. On obtient ainsi 650 mg de N-[3β-acétoxylup-20(29)-èn-28-oyl]-12-aminododécanoate de méthyle, sous la forme d'une meringue blanche.

8,1 $cm^3$ de soude 4N sont ajoutés en 5 minutes environ à une solution de 570 mg de N-[3β-acétoxylup-20(29)-èn-28-oyl]-12-aminododécanoate de méthyle dans 9 $cm^3$ de méthanol et 4,5 $cm^3$ de tétrahydrofurane. Le mélange réactionnel est agité pendant 20 heures à une température voisine de 20°C et le solvant est évaporé sous pression réduite (2,7 kPa) à une température voisine de 35°C. On dilue par 40 $cm^3$ d'eau distillée

et acidifie à un pH voisin de 1 à l'aide de 9,5 $cm^3$ d'acide chlorhydrique 4N. Après 15 heures d'agitation à une température voisine de 20°C, 120 $cm^3$ d'éther éthylique sont ajoutés et la phase organique est décantée. La phase aqueuse est extraite de nouveau par 50 $cm^3$ d'éther éthylique, les phases organiques rassemblées sont lavées par 75 $cm^3$ au total d'eau distillée, séchées sur sulfate de magnésium anhydre et évaporées à sec sous pression réduite (2,7 kPa) à une température voisine de 30°C. Le résidu est mis en suspension dans 6 $cm^3$ d'oxyde de diisopropyle, le solide est séparé par filtration, lavé par 4 $cm^3$ au total d'oxyde de diisopropyle et séché sous pression réduite (13,5 Pa) à une température voisine de 30°C. On obtient ainsi 470 mg d'acide N-[3β-hydroxylup-20(29)-èn-28-oyl]-12-aminododécanoïque fondant vers 182°C.

Le 12-aminododécanoate de méthyle est préparé d'après H. ZAHN, H.D. STOPER, Chem. Ber., 98(10), 3251 (1965).

## Exemple 17

On ajoute 0,24 $cm^3$ de triéthylamine et 500 mg de chlorhydrate de 16-aminohexadécanoate de méthyle à une solution de 820 mg de chlorure de 3β-acétoxylup-20(29)-èn-28-oyle en solution dans un mélange composé de 20 $cm^3$ de chloroforme et de 20 $cm^3$ de tétrahydrofurane. Après 20 heures d'agitation à une température voisine de 20°C, on ajoute 40 $cm^3$ d'eau distillée et décante la phase organique. La phase aqueuse est extraite par 75 $cm^3$ au total de chloroforme, les extraits organiques sont rassemblés, séchés sur du sulfate de magnésium anhydre et évaporés sous pression réduite (2,7 kPa) à une température voisine de 35°C. Le résidu obtenu est chromatographié une première fois sur une colonne de 4 cm de diamètre contenant 150 g de silice (0,02-0,045 mm) éluée avec un mélange de cyclohexane et d'acétate d'éthyle 80-20 (en volumes) et en recueillant des fractions de 10 $cm^3$. Les 80 premières fractions sont éliminées; les 23 suivantes sont réunies et concentrées sous pression réduite (2,7 kPa) à une température voisine de 35°C. On obtient ainsi 568 mg d'une meringue beige qui est chromatographiée une deuxième fois (colonne de 4 cm de diamètre; 150 g de silice (0,02-0,045 mm), fractions de 30 $cm^3$). Le système éluant est composé d'un mélange de chlorure de méthylène et de méthanol 99-1 (en volumes). Les 30 premières fractions sont éliminées, les 6 suivantes sont réunies et évaporées à sec sous pression réduite (2,7 kPa) à une température voisine de 30°C. Les 363 mg obtenus sont chromatographiées une troisième fois sur une colonne de 1,7 cm de diamètre, contenant 20 g de silice (0,02-0,045 mm) en éluant avec un mélange de cyclohexane et d'acétate d'éthyle 80-20 en volumes). On recueille des fractions de 3 $cm^3$. Les 13 premières fractions sont éliminées les 17 suivantes sont rassemblées et concentrées à sec sous pression réduite (2,7 kPa) à une température voisine de 30°C. On obtient ainsi 380 mg de N-[3β-acétoxylup-20(29)-èn-28-oyl]-16-aminohexadécanoate de méthyle sous forme d'une meringue blanche [Rf=0,3 ; chromatographie sur couche mince de silice ; éluant : chlorure de méthylène acétate d'éthyle 97-3 (en volumes)].

Une solution de 380 mg de N-[3β-acétoxylup-20(29)-èn-28-oyl]-16-amino hexadécanoate de méthyle dans un mélange de 5 $cm^3$ de méthanol, 2,5 $cm^3$ de tétrahydrofurane et 0,62 $cm^3$ de soude 4N est agitée pendant 15 heures à une température voisine de 20°C. Le solvant est ensuite évaporé à sec sous pression réduite (2,7 kPa) à une température voisine de 30°C. Le résidu blanc est mis en suspension dans 25 $cm^3$ d'eau distillée. On ajoute 1 $cm^3$ d'acide chlorhydrique 5N et on maintient l'agitation pendant 20 minutes. Le solide est séparé par filtration, lavé par 50 $cm^3$ au total d'eau distillée et séché sous pression réduite (13,5 Pa) à une température voisine de 20°C. Le solide obtenu est mis en suspension dans 20 $cm^3$ d'eau distillée et filtré. On obtient ainsi 292 mg d'acide qui est purifié par chromatographie sur une colonne de 1,7 cm de diamètre contenant 20 g de silice (0,02-0,045 mm) éluée par un mélange de cyclohexane, d'acétate d'éthyle, et de chlorure de méthylène 50-40-10 (en volumes) en recueillant des fractions de 2 $cm^3$. Les 10 premières fractions sont éliminées, les 9 suivantes sont réunies et concentrées à sec sous pression réduite (2,7 kPa) à une température voisine de 30°C. On obtient ainsi 280 mg d'acide N-[3β-hydroxylup-20(29)-èn-28-oyl]-16-aminohexadécanoïque, sous la forme d'un solide blanc fondant vers 85°C.

Le 16-aminohexadécanoate de méthyle peut être préparé de la manière suivante :

Une solution de 1,3 g de 16-phtalimidohexadécanoate de méthyle dans 50 $cm^3$ de méthanol est agitée pendant 3 heures, à une température voisine de 80°C, en présence de 170 mg d'hydrate d'hydrazine. La solution est refroidie vers 20°C et on ajoute 2 $cm^3$ d'éther chlorhydrique 5N. Le solvant est évaporé sous pression réduite (2,7 kPa) à une température voisine de 35°C. Le résidu est repris dans 50 $cm^3$ d'eau distillée. Le solide est séparé par filtration. Les filtrats sont concentrés à sec sous pression réduite à une température voisine de 35°C. On obtient 320 mg d'un solide blanc. Le solide séparé par filtration est lavé par 25 $cm^3$ au total d'eau distillée, mis en suspension dans 20 $cm^3$ d'eau distillée et on ajoute 1 $cm^3$ d'acide chlorhydrique 5N. Après 15 minutes sous agitation, les cristaux sont séparés par filtration, lavés par 20 $cm^3$ d'acide chlorhydrique 1N et 20 $cm^3$ d'eau distillée. Ce lot (1,2 g) additionné des 320 mg obtenus après évaporation des filtrats est chromatographié sur une colonne de 2 cm de diamètre contenant 30 g de silice (0,020-0,045 mm), éluée par un

mélange de chloroforme et de méthanol 90-10 (en volumes) en recueillant des fractions de 20 $cm^3$. Les 3 premières fractions sont éliminées, les 6 suivantes sont rassemblées et évaporées sous pression réduite (2,7 kPa) à une température voisine de 35°C. Le résidu est mis en suspension dans 20 $cm^3$ d'une solution saturée de bicarbonate de sodium, et agité 15 minutes. Le solide est séparé par filtration, lavé par 50 $cm^3$ au total d'eau distillée et séché sous pression réduite (13,5 Pa) à une température voisine de 20°C. On obtient ainsi 500 mg de 16-aminohexadécanoate de méthyle sous la forme d'un solide blanc.

Le 16-phtalimidohexadécanoate de méthyle peut être préparé de la manière suivante :

A un mélange de 1,36 g de 16-hydroxyhexadécanoate de méthyle, 847 mg de phtalimide, 1,49 g de triphénylphosphine, dans 2,6 $cm^3$ de tétrahydrofurane, on ajoute goutte à goutte une solution de 0,89 $cm^3$ d'azidodicarboxylate d'éthyle dans 2,6 $cm^3$ de tétrahydrofurane. L'agitation est maintenue 15 heures à une température voisine de 20°C et le solvant est évaporé sous pression réduite (2,7 kPa) à une température voisine de 30°C. Les 5,4 g de résidu obtenus sont chromatographiés sur une colonne de 3 cm de diamètre contenant 100 g de silice (0,020-0,045 mm) éluée avec du chlorure de méthylène. Les 18 premières fractions sont éliminées, les 36 suivantes sont rassemblées et évaporées à sec sous pression réduite (2,7 kPa) à une température voisine de 30°C. On obtient ainsi 1,5 g de 16-phtalimidohexadécanoate de méthyle, sous la forme d'un solide blanc.

## Exemple 18

On ajoute 0,31 $cm^3$ de triéthylamine et 660 mg de chlorhydrate de 17-aminoheptadécanoate de méthyle à une solution de 1 g de chlorure de 3β-acétoxylup-20(29)-èn-28-oyle dans un mélange de 20 $cm^3$ de chloroforme et de 20 $cm^3$ de tétrahydrofurane. Après 20 heures d'agitation à une température voisine de 20°C, on ajoute 40 $cm^3$ d'eau distillée. La phase organique est décantée et la phase aqueuse est extraite par 100 $cm^3$ au total de chloroforme. Les extraits organiques sont rassemblés, séchés sur du sulfate de magnésium anhydre et le solvant est éliminé sous pression réduite (2,7 kPa) à une température voisine de 30°C. Le résidu obtenu est chromatographié une première fois sur un colonne de 4 cm de diamètre contenant 100 g de silice (0,02-0,045 mm). Le mélange éluant est composé de chlorure de méthylène et d'acétate d'éthyle 95-5 (en volumes), les fractions sont de 30 $cm^3$. Après élimination des 5 premières fractions, les 19 suivantes sont réunies et concentrées sous pression réduite (2,7 kPa) à une température voisine de 35°C. On obtient ainsi 750 mg d'une meringue blanche qui est chromatographiée une deuxième fois : colonne de 2,2 cm de diamètre, 40 g de silice (0,02-0,045 mm), en éluant avec un mélange de chlorure de méthylène et d'acétate d'éthyle 98-2 en volumes et en recueillant des fractions de 10 $cm^3$. Les 16 premières fractions sont éliminées, les 17 suivantes sont rassemblées et concentrées à sec sous pression réduite (2,7 kPa) à une température voisine de 30°C. Les 540 mg obtenus sont chromatographiés une troisième fois sur une colonne de 2 cm de diamètre contenant 40 g de silice (0,02-0,045 mm) éluée avec un mélange de cyclohexane et d'acétate d'éthyle 80-20 (en volumes) en recueillant des fractions de 5 $cm^3$. Après élimination des 4 premières fractions, les 10 suivantes sont rassemblées et concentrées à sec sous pression réduite à une température voisine de 30°C (2,7 kPa). Les 443 mg obtenus sont chromatographiés une dernière fois sur une colonne de 1,4 cm de diamètre contenant 20 g de silice (0,020-0,045 mm) éluée avec un mélange de cyclohexane et d'acétate d'éthyle 80-20 (en volumes), en recueillant des fractions de 5 $cm^3$. Après élimination des 2 premières fractions, les 7 suivantes sont rassemblées et concentrées à sec sous pression réduite (2,7 kPa) à une température voisine de 30°C. On obtient 210 mg de N-[3β-acétoxylup-20(29)-èn-28-oyl]-17-aminoheptadécanoate de méthyle sous forme d'une meringue blanche [Rf=0,4 ; chromatographie sur couche mince de silice ; éluant : cyclohexane, acétate d'éthyle 80-20 (en volumes)].

Une solution de 210 mg de N-[3β-acétoxylup-20(29)-èn-28-oyl]-17-amino heptadécanoate de méthyle dans 2,7 $cm^3$ de méthanol, 1,3 $cm^3$ de tétrahydrofurane et 0,34 $cm^3$ de soude 4N est agitée pendant 15 heures à une température voisine de 20°C. Le solvant est alors éliminé sous pression réduite (2,7 kPa) à une température voisine de 30°C. Le résidu est mis en solution dans 12,5 $cm^3$ d'eau distillée et on ajoute 0,5 $cm^3$ d'acide chlorhydrique 5N. Après 20 minutes d'agitation, le solide est séparé par filtration, lavé par 50 $cm^3$ au total d'eau distillée et séché sous pression réduite (13,5 Pa) à une température voisine de 20°C. Or obtient ainsi 194 mg d'acide qui est chromatographié sur une colonne de 1,7 cm de diamètre contenant 20 g de silice (0,020-0,045 mm), éluée avec un mélange de cyclohexane, d'acétate d'éthyle et de chlorure de méthylène 50-40-10 (en volumes) en recueillant des fractions de 5 $cm^3$. Les 10 premières fractions sont éliminées, les 9 suivantes sont rassemblées et concentrées à sec sous pression réduite (2,7 kPa) à une température voisine de 35°C. Le solide obtenu est mis en suspension dans 20 $cm^3$ d'eau distillée et séparé par filtration. On obtient ainsi 220 mg d'acide N-[3β-hydroxylup-20(29)-èn-28-oyl]-17-aminoheptadécanoïque, sous la forme d'un solide blanc, fondant vers 82°C.

Le 17-aminoheptadécanoate de méthyle est préparé de la manière suivante :

On ajoute 0,37 $cm^3$ de chlorure de thionyle dans 23 $cm^3$ de méthanol refroidi à -20°C et on ajoute 980 mg d'acide 17-aminoheptadécanoïque. La solution est agitée pendant 12 heures à une température voisine de 20°C et le solvant est éliminé sous pression réduite (2,7 kPa) à une température voisine de 40°C. On obtient ainsi 1,08 g de chlorhydrate de 17-aminoheptadécanoate de méthyle, sous la forme d'un solide blanc.

L'acide 17-aminoheptadécanoïque peut être préparé de la manière suivante :

Une solution de 3,4 g de l'acide 17-phtalimidoheptadécanoïque dans 60 $cm^3$ de méthanol est agitée pendant 1 heure, à une température voisine de 80°C, en présence de 1,67 g d'hydrate d'hydrazine. La solution est refroidie vers 20°C et le solide est séparé par filtration et séché à l'air. Le résidu (1,92 g) est repris dans 180 $cm^3$ d'eau distillée. Le solide est séparé par filtration, lavé par 45 $cm^3$ au total d'eau distillée et séché sous pression réduite (13,5 Pa) à une température voisine de 40°C. On obtient 980 mg de l'acide 17-aminoheptadécanoïque sous la forme d'un solide blanc.

L'acide 17-phtalimidoheptadécanoïque peut être préparé de la manière suivante :

A 2,14 g de phtalimide potassé dans 40 $cm^3$ de N,N-diméthylformamide, on ajoute 2 g d'acide 17-bromoheptadécanoïque et on chauffe 6 heures à une température voisine de 80°C. La solution est refroidie vers 20°C, on la verse dans 100 $cm^3$ d'eau distillée et on ajoute goutte à goutte 10 $cm^3$ d'acide chlorhydrique 2N. Le solide formé est séparé par filtration. On obtient 5,13 g d'un résidu qui est chromatographié sur une colonne de 4 cm de diamètre contenant 250 g de silice (0,020-0,045 mm) éluée avec un mélange de chlorure de méthylène et de méthanol 95-5 (en volumes). Les 4 premières fractions sont éliminées, les 3 suivantes sont rassemblées et concentrées à sec sous pression réduite (2,7 kPa) à une température voisine de 40°C. On obtient ainsi 3,39 g d'acide 17-phtalimidoheptadécanoïque, sous la forme d'un solide blanc.

**Exemple 19**

A une solution de 0,54 g de 11-amino-2,2-diméthylundécanoate de méthyle et 0,28 $cm^3$ de triéthylamine dans 50 $cm^3$ de dichlorométhane on ajoute une solution de 1,1 g de chlorure de 3β-acétoxylup-20(29)-èn-28-oyle (préparé à partir de 1 g d'acide 3β-acétoxylup-20(29)-èn-28-oyle) dans 50 $cm^3$ de dichlorométhane. La solution est agitée 15 heures à une température voisine de 20°C. On ajoute 100 $cm^3$ de dichlorométhane. La phase organique est lavée par 300 $cm^3$ au total d'eau distillée, séchée sur du sulfate de magnésium et concentrée à sec sous pression réduite (2,7 kPa) à une température voisine de 40°C. Les 2 g d'huile obtenus sont chromatographiés sur une colonne de 3 cm de diamètre est 50 cm de haut de silice (0,02-0,045 mm) éluée par un mélange de dichlorométhane et d'acétate d'éthyle 95-5 (en volumes), en recueillant des fractions de 50 $cm^3$. Les 12 premières fractions sont éliminées, les 4 suivantes sont réunies et concentrées sous pression réduite (2,7 kPa) à une température voisine de 60°C. L'huile jaune obtenue est mise en solution dans 100 $cm^3$ de dichlorométhane. La phase organique est lavée par 150 $cm^3$ au total d'eau distillée, séchée sur du sulfate de magnésium puis concentrée sous pression réduite (2,7 kPa) à une température voisine de 40°C. Les 1,1 g d'huile jaune obtenus sont chromatographiés sur une colonne de 2 cm de diamètre et 40 cm de haut contenant de la silice (0,02-0,045 mm), éluée avec du dichlorométhane, en recueillant des fractions de 50 $cm^3$ . Les 25 premières fractions sont éliminées. Les 21 suivantes sont réunies et concentrées sous pression réduite (13,5 Pa) à une température voisine de 40°C. On obtient 0,80 g de N-[3β-acétoxylup-20(29)-èn-28-oyl]-11-amino-2,2-diméthylundécanoate de méthyle sous la forme d'une laque blanche (Rf=0,12 ; chromatographie sur couche mince de silice ; éluant dichlorométhane).

19 $cm^3$ de soude 4N sont ajoutés en 5 minutes environ à une solution de 1,4 g de N-[3β-acétoxylup-20(29)-èn-28-oyl]-11-amino-2,2-diméthyl ndécanoate de méthyle dans 40 $cm^3$ de méthanol et 40 $cm^3$ de tétrahydrofurane et le mélange réactionel est agité pendant 48 heures à une température voisine de 20°C. Le mélange réactionnel est acidifié à un pH voisin de 2 à l'aide d'acide chlorhydrique 4N puis on ajoute 200 $cm^3$ d'eau distillée. Après 30 minutes d'agitation, la phase aqueuse est décantée, le solide est séparé par filtration et rincé par 90 $cm^3$ au total d'eau distillée. Le filtrat est repris par 100 $cm^3$ d'acétate d'éthyle. La phase organique est séchée 2 heures sur du sulfate de magnésium et évaporée à sec sous pression réduite (2,7 kPa) à une température voisine de 40°C. Les 1,4 g de meringue blanche obtenus sont chromatographiés sur une colonne de 2,7 cm de diamètre et 36 cm de haut contenant de la silice (0,02-0,045 mm) éluée successivement par du dichlorométhane (fractions 1 à 25), un mélange de dichlorométhane et d'acétate d'éthyle 95-5 (en volumes) (fractions 26 à 80) et un mélange de dichlorométhane et d'acétate d'éthyle 9:1 (en volumes) (fractions 81 à 120) en recueillant des fractions de 50 $cm^3$. Les 95 premières fractions sont éliminées, les 20 suivantes sont réunies et concentrées sous pression réduite (2,7 kPa) à une température voisine de 40°C. On obtient ainsi 1 g d'une meringue blanche qui est trituré avec 15 $cm^3$ de n-heptane, filtré, lavé avec 20 $cm^3$ au total de n-heptane puis séché sous pression réduite (13,5 Pa) à une température voisine de 40°C. On obtient ainsi 750 mg d'acide N-[3(β-hydroxylup-20(29)-èn-28-oyl]-11-amino-2,2-diméthyl undécanoïque fondant à une température voisine de 120°C.

Le 11-amino-2,2-diméthylundécanoate de méthyle est préparé de la manière suivante :

Une solution de 15,4 g de 11-phtalimido-2,2-diméthylundécanoate de méthyle et 2,33 $cm^3$ d'hydrate d'hydrazine à 98%, dans 500 $cm^3$ de méthanol est agitée 12 heures à une température voisine de 20°C. On ajoute 32 $cm^3$ de méthanol chlorhydrique 5N. Le solvant est évaporé sous pression réduite (2,7 kPa) à une température voisine de 40°C. Le résidu est repris par 150 $cm^3$ d'eau distillée. Après 20 minutes d'agitation, le solide est séparé par filtration, lavé par 90 $cm^3$ au total d'eau distillée. La phase aqueuse est amenée à un pH voisin de 9-10 au moyen d'une solution de carbonate de sodium à 10 %, extraite par 500 $cm^3$ au total d'oxyde de diisopropyle, séchée sur du sulfate de magnésium puis concentrée sous pression réduite (2,7 kPa) à une température voisine de 40°C. Le résidu est repris par un mélange de 15 $cm^3$ de méthanol et 50 $cm^3$ d'oxyde de diéthyle. On ajoute 10 $cm^3$ de méthanol chlorhydrique 5N puis on évapore à sec sous pression réduite (2,7 kPa) à une température voisine de 40°C. L'huile jaune obtenue est reprise par 100 $cm^3$ d'eau distillée. On ajoute 2 $cm^3$ d'acide chlorhydrique 4N puis amène à un pH voisin de 9-10 au moyen d'une solution de carbonate de sodium à 10 %. On extrait par 450 $cm^3$ au total d'acétate d'éthyle. La phase organique est séchée sur du sulfate de magnésium puis concentrée à sec sous pression réduite (13,5 Pa) à une température voisine de 40°C. On obtient ainsi 6,8 g de 11-amino-2,2-diméthylundécanoate de méthyle sous la forme d'une huile visqueuse jaune [Rf=0,54 ; chromatographie sur couche mince de silice ; éluant : chloroforme, méthanol, ammoniaque à 28 % 24-6-1 (en volumes)].

Le 11-phtalimido-2,2-diméthylundécanoate de méthyle est obtenu de la manière suivante :

A 8,2 g de phtalimide potassée dans 70 $cm^3$ de N,N-diméthylformamide, on ajoute 12,3 g de 11-bromo-2,2-diméthylundécanoate de méthyle en solution dans 70 $cm^3$ de N,N-diméthylformamide et on chauffe 3 heures à une température voisine de 80°C. La solution est refroidie à une température voisine de 20°C puis versée dans 1400 $cm^3$ d'eau distillée. On acidifie à un pH voisin de 2 au moyen d'acide chlorhydrique 4N puis on extrait par 1000 $cm^3$ au total d'acétate d'éthyle. La phase organique est lavée par 450 $cm^3$ au total d'eau distillée, séchée sur du sulfate de magnésium, puis concentrée sous pression réduite (13,5 Pa) à une température voisine de 60°C. On obtient ainsi 15,4 g de 11-phtalimido-2,2-diméthylundécanoate de méthyle sous la forme d'une huile visqueuse jaune pâle [Rf=0,38 ; chromatographie sur couche mince de silice - éluant : cyclohexane acétate d'éthyle 8-2 (en volumes)].

Le 11-bromo-2,2-dimétlnylundécanoate de méthyle a été obtenu de la manière suivante :

A une solution de 25 $cm^3$ de diisopropylamine dans 90 $cm^3$ de tétrahydrofurane refroidie à une température voisine de -78°C, on ajoute en 25 minutes 110 $cm^3$ d'une solution 1,6 M de n-butylithium dans l'hexane. On laisse 1 heure à une température voisine de -78°C. On ajoute 18,5 $cm^3$ d'isobutyrate de méthyle en 25 minutes. On agite 1 heure à une température voisine de -70°C avant d'ajouter en 15 minutes une solution de 70,4 g de 1,9-dibromononane dans 45 $cm^3$ de tétrahydrofurane. On laisse agiter 2 heures à une température voisine de -70°C puis 12 heures à une température voisine de 20°C. On concentre sous pression réduite (2,7 kPa) à une température voisine de 40°C. On ajoute 200 $cm^3$ d'acide chlorhydrique 1N, puis on extrait au moyen de 250 $cm^3$ de dichlorométhane. La phase organique est lavée successivement par 160 $cm^3$ au total d'acide chlorhydrique 1N, 160 $cm^3$ au total d'eau distillée, 30 $cm^3$ d'une solution aqueuse saturée en bicarbonate de sodium et enfin 80 $cm^3$ au total d'eau distillée. La phase organique est séchée sur du sulfate de magnésium puis concentrée sous pression réduite (2,7 kPa) à une température voisine de 50°C. Les 74 g d'une huile orange obtenue sont distillés sous pression réduite (20 Pa) en recueillant la fraction qui distille entre 120 et 130°C. On obtient ainsi 33,2 g d'une huile qui est redistillée sous pression réduite (10 Pa). On recueille la fraction qui distille entre 114°C et 122°C. On obtient ainsi 25 g de 11-bromo-2,2-diméthylundécanoate de méthyle.

**Exemple 20**

On chauffe au reflux pendant 3 heures, une solution de 1 g de N-[3-oxolup-20(29)-èn-28-oyl]-11-aminoundécanoate de méthyle, 440 mg de chlorhydrate d'aminoxyacétate de méthyle dans 20 $cm^3$ de pyridine, puis la solution est refroidie à une température voisine de 20°C et additionnée de 100 $cm^3$ d'eau distillée et de 50 $cm^3$ d'acétate d'éthyle. La phase organique est décantée, la phase aqueuse extraite par 100 $cm^3$ au total d'acétate d'éthyle, les extraits organiques sont rassemblés, lavés par 50 $cm^3$ au total d'eau distillée, séchés sur du sulfate de magnésium anhydre et évaporés sous pression réduite (2,7 kPa) à une température voisine de 35°C. Le résidu obtenu est chromatographié sur un colonne de 5 cm de diamètre contenant 200 g de silice (0,02-0,045 mm) éluée avec un mélange de cyclohexane et d'acétate d'éthyle 80-20 (en volumes), en recueillant des fractions de 30 $cm^3$. Les 11 premières fractions sont éliminées ; les 16 suivantes sont réunies et concentrées sous pression réduite (2,7 kPa) à une température voisine de 35°C. On obtient ainsi 940 mg d'une huile qui est purifiée par HPLC en 2 fois, sur une colonne de 5 cm de diamètre contenant 300 g de silice greffée en $C_{18}$ (0,015-0,025mm), éluée avec un mélange d'acétonitrile, d'eau, de tétrahydrofurane 50-30-15 puis 65-20-15 (en volumes). Les 18 premières fractions sont éliminées, les 2 suivantes sont rassemblées et concentrées

à sec sous pression réduite (13,5 Pa) à une température voisine de 35°C. On obtient ainsi 450 mg de N-[3-(méthoxycarbonylméthoxyimino]lup-20(29)-èn-28-oyl]-11-aminoundécanoate de méthyle sous la forme d'une d'une laque incolore (Rf=0,4 ; acétate d'éthyle (75-25 en volumes)].

Une solution de 454 mg de N-[-(méthoxycarbonylméthoxyimino)lup-20 (29)-èn-28-oyl]-11-aminoundécanoate de méthyle dans 6 $cm^3$ de méthanol, 3 $cm^3$ de tétrahydrofurane et 0,77 $cm^3$ de soude 4N est agitée pendant 15 heures à une température voisine de 20°C et acidifiée avec 1 $cm^3$ d'acide chlorhydrique 5N. Après 2 heures d'agitation, le solide est séparé par filtration, lavé par 30 $cm^3$ au total d'eau distillée et séché sous pression réduite (13,5 Pa) à une température voisine de 40°C. On obtient ainsi 320 mg d'acide N-[3-(carboxyméthoxyimino)lup-20 (29)-èn-28-oyl]-11-aminoundécanoïque, (mélange des isomères Z et E), sous la forme d'un solide blanc fondant vers 100°C.

## Exemple 21

On chauffe au reflux pendant 1 heure, une solution de 1,35 g de N-[3-oxolup-20(29)-èn-28-oyl]-11-aminoundécanoate de méthyle, 210 mg de chlorhydrate de 2-(4-méthyl-1-pipérazinyl)éthoxyamine dans 25 $cm^3$ de pyridine, puis la solution est refroidie à une température voisine de 20°C. 200 $cm^3$ d'eau distillée sont ajoutés, puis 11 $cm^3$ de soude 1N et 100 $cm^3$ d'acétate d'éthyle. La phase organique est décantée, la phase aqueuse est extraite par 200 $cm^3$ au total d'acétate d'éthyle, les extraits organiques sont rassemblés, lavés par 60 $cm^3$ au total d'eau distillée, séchés sur du sulfate de magnésium anhydre et évaporés sous pression réduite (13,5 kPa) à une température voisine de 35°C. Le résidu obtenu (1,65 g) est chromatographié sur un colonne de 5 cm de diamètre contenant 200 g de silice (0,02-0,045 mm) éluée avec un mélange d'acétate d'éthyle et de méthanol 50-50 (en volumes), en recueillant des fractions de 30 $cm^3$. Les 17 premières fractions sont éliminées; les 18 suivantes sont réunies et concentrées sous pression réduite (2,7 kPa) à une température voisine de 35°C. On obtient ainsi 1,34 g de N-{3-[2-(4-méthyl-1-pipérazi-nyl)éthoxyimino]lup-20(29)-èn-28-oyl}-11-aminoundécanoate de méthyle sous forme d'une meringue blanche [Rf=0,3 ; chromatographie sur couche mince de silice ; éluant : acétate d'éthyle méthanol 50-50 (en volumes)].

Une solution de 1,34 g de N-{3-[2-(4-méthyl-1-pipérazinyl) éthoxyimino]lup-20(29)-èn-28-oyl}-11-aminoundécanoate de méthyle dans 20 $cm^3$ de méthanol, 10 $cm^3$ de tétrahydrofurane et 1,05 $cm^3$ de soude 4N est agitée pendant 15 heures à une température voisine de 20°C et le solvant est éliminé sous pression réduite (2,7 kPa) à une température voisine de 40°C. On ajoute alors 25 $cm^3$ d'acétate d'éthyle, le solide est séparé par filtration et lavé avec 15 $cm^3$ au total d'acétate d'éthyle. On obtient ainsi 760 mg d'acide N-{3-[2-(4-méthyl-1-pipérazinyl)éthoxyimino]lup-20(29)-èn-28-oyl}-11-aminoundécanoïque, (mélange des isomères Z et E), sous la forme d'un solide beige fondant vers 97°C.

## Exemple 22

Une solution de 1 g de N-[3β-acétoxylup-20(29)-èn-28-oyl]-11-amino undécanoate de méthyle dans 50 $cm^3$ d'éthanol est hydrogénée, sous pression atmosphérique et à une température voisine de 20°C, en présence de 0,1 g de palladium sur charbon à 5 % (en poids), pendant 4 heures. Le catalyseur est filtré, lavé par 30 $cm^3$ au total d'éthanol absolu et les filtrats sont évaporés à sec sous pression réduite (2,7 kPa) à une température voisine de 40°C. On obtient 810 mg d'une huile incolore qui est chromatographiée sur une colonne de 4 cm de diamètre contenant 100 g de silice (0,02-0,045 mm) éluée avec un mélange de cyclohexane, de chlorure de méthylène et d'acétate d'éthyle 80-10-10 (en volumes), en recueillant des fractions de 30 $cm^3$. Après élimination des 17 premières fractions, les 6 suivantes sont réunies et concentrées sous pression réduite (2,7 kPa) à une température voisine de 40°C. On obtient 600 mg d'un mélange 50-50 de N-[3β-acétoxylupan-28-oyl]-11-aminoundécanoate de méthyle et d'éthyle sous la forme d'une huile incolore [Rf=0,5 ; chromatographie sur couche mince de silice ; éluant : cyclohexane, acétate d'éthyle 80-20 (en volumes)].

Une solution de 600 mg de N-[3β-acétoxylupan-28-oyl]-11-amino undécanoate de méthyle dans 4,3 $cm^3$ de méthanol et 8,5 $cm^3$ de tétrahydrofurane est additionnée d'1 $cm^3$ de soude 4N, agitée pendant 15 heures à une température voisine de 20°C, acidifiée à un pH voisin de 3-4 à l'aide de 0,9 $cm^3$ d'acide chlorhydrique 4N et diluée avec 25 $cm^3$ d'eau distillée. Après 15 heures sous agitation, le solide est séparé par filtration, lavé par 50 $cm^3$ au total d'eau distillée et séché sous pression atmosphérique à une température voisine de 20°C. On obtient ainsi 410 mg d'acide N-[3β-hydroxylupan-28-oyl]-11-amino undécanoïque, fondant vers 110-115°C.

## Exemple 23

Une solution de 1, 4 g de N-(30-nor 3β-acétoxy-20-oxolupan-28-oyl)-11-aminoundécanoate de méthyle, 100 mg de borohydrure de sodium et 700 mg de chlorure de tétrabutylammonium, dans 120 $cm^3$ de toluène,

est chauffée à une température voisine du reflux. Après 9 heures, on ajoute 100 mg de borohydrure de sodium et 700 mg de chlorure de tétrabutylammonium et on porte 9 heures à une température voisine du reflux. Après avoir ajouté une fois de plus 100 mg de borohydrure de sodium et 700 mg de chlorure de tétrabutylammonium, le mélange réactionnel est chauffé 24 heures à une température voisine du reflux. On ajoute 1 $cm^3$ d'eau distillée et on maintient la température. Après 8 heures, on ajoute successivement 3 fois à 8 heures d'intervalle, 100 mg de borohydrure de sodium et 700 mg de chlorure de tétrabutylammonium. La solution est alors agitée 48 heures à une température voisine de 20°C. On ajoute alors 50 $cm^3$ d'eau distillée en 1 heure. La phase organique est décantée, la phase aqueuse est extraite par 80 $cm^3$ au total d'acétate d'éthyle, les phases organiques rassemblées sont lavées par 90 $cm^3$ au total d'eau distillée, séchées 2 heures sur du sulfate de magnésium anhydre et évaporées à sec sous pression réduite (2,7 kPa) à une température voisine de 45°C. Le résidu obtenu est chromatographié sur une colonne de 3 cm de diamètre contenant 80 g de silice (0,02-0,045 mm) éluée avec un mélange de cyclohexane et d'acétate d'éthyle 6-4 (en volumes) en recueillant des fractions de 50 $cm^3$. Les 12 premières fractions sont réunies et concentrées sous pression réduite (2,7 kPa) à une température voisine de 35°C. On obtient ainsi 800 mg d'un solide qui est mis en solution dans 20 $cm^3$ d'éther diéthylique. La solution éthérée est lavée successivement par 10 $cm^3$ d'une solution d'acide chlorhydrique 2N puis par 4 fois 10 $cm^3$ d'eau distillée. La phase organique est séchée sur du sulfate de magnésium anhydre et concentrée à sec sous pression réduite (2,7 kPa) à une température voisine de 40°C. On obtient ainsi 500 mg de N-(30-nor 3β-acétoxy-20-hydroxylupan-28-oyl)-11-aminoundécanoate de méthyle sous forme d'une meringue blanche.

Une solution de 500 mg de N-(30-nor 3β-acétoxy-20-hydroxylupan-28-oyl)-11-aminoundécanoate de méthyle dans 10 $cm^3$ de méthanol, 5 $cm^3$ de tétrahydrofurane et 5 $cm^3$ de soude 4N est agitée pendant 12 heures à une température voisine de 20°C. La suspension est alors acidifiée à un pH voisin de 1-2 à l'aide d'acide chlorhydrique 4N puis diluée au moyen de 20 $cm^3$ d'eau distillée. Après 30 minutes d'agitation, le solide est séparé par filtration, lavé successivement par 5 fois 10 $cm^3$ d'eau distillée et 3 fois 5 $cm^3$ d'éther diéthylique puis séché sous pression réduite (13,5 Pa) à une température voisine de 40°C. On obtient ainsi 400 mg d'un solide qui est mis en solution dans 5 $cm^3$ de dioxane. A la solution obtenue, or ajoute 15 $cm^3$ d'eau distillée. Le solide blanc obtenu est filtré, lavé par 2 fois 3 $cm^3$ d'un mélange dioxane-eau distillée 1-3 (en volumes) puis séché sous pression réduite (13,5 Pa) à une température voisine de 40°C. On obtient ainsi 250 mg d'acide N-(30-nor 3β,20-dihydroxylupan-28-oyl)-11-aminoundécanoïque, fondant vers 170°C.

## Exemple 24

A une solution de 2,6 g de soude dans 18 $cm^3$ d'eau distillée, refroidie à -5°C, on ajoute 0,7 $cm^3$ de brome. Le mélange réactionnel est agité 15 minutes à -5°C puis on ajoute 12 $cm^3$ de dioxane. La solution obtenue est ajoutée en 10 minutes environ à une solution de 2,3 g de N-(30-nor 3β-acétoxy-20-oxolupan-28-oyl)-11-aminoundécanoate de méthyle dans 50 $cm^3$ de dioxane. La solution est agitée à une température voisine de 10°C pendant 15 heures puis on ajoute une solution de 0,5 g de sulfite de sodium dans 5 $cm^3$ d'eau distillée et on acidifie à un pH voisin de 1 à l'aide d'acide chlorhydrique 4N. Après 30 minutes, on dilue par 50 $cm^3$ d'eau distillée et le solide est séparé par filtration, lavé par 50 $cm^3$ au total d'eau distillée et séché à l'air sous pression atmosphérique à une température voisine de 20°C. On obtient ainsi 2 g d'un solide blanc qui est mis en solution dans un mélange de 10 $cm^3$ de dioxane et 5 $cm^3$ d'eau distillée. Le mélange est abandonné 48 heures à une température voisine de 20°C. Le solide est filtré, rincé successivement par 2 $cm^3$ de dioxane et 2 fois 5 $cm^3$ d'oxyde de diéthyle puis séché sous pression réduite (13,5 Pa) à une température voisine de 40°C. On obtient ainsi 800 mg d'acide N-(30-nor 3β-hydroxy-20-oxolupan-28-oyl)-11-aminoundécanoïque, fondant vers 160°C.

Le N-[30-nor 3β-acétoxy-20-oxolupane-28-oyl]-11-aminoundécanoate de méthyle peut être préparé de la mainère suivante :

2,2 $cm^3$ de triéthylamine et 1,94 g de chlorhydrate de 11-aminoundécanoate de méthyle sont ajoutés à 195 $cm^3$ d'une solution dans le dichlorométhane de chlorure de 30 nor 3β-acétoxy-20-oxolupan-28-oyle. La solution est alors agitée 72 heures à une température voisine de 20°C. Le mélange réactionnel est dilué par 100 $cm^3$ d'eau distillée. La phase organique est extraite par 3 fois 150 $cm^3$ de dichlorométhane. Les phases organiques rassemblées sont séchées 3 heures sur du sulfate de magnésium, le desséchant est filtré, rincé par 90 $cm^3$ au total de dichlorométhane. Les phases organiques, réunies sont concentrées à sec sous pression réduite (2,7 kPa) à une température voisine de 50°C. Les 5,7 g d'huile jaune pâle obtenus sont chromatographiés sur une colonne de 4 cm de diamètre, 60 cm de haut et contenant de la silice (0,02-0,045 mm) éluée successivement avec un mélange de cyclohexane et d'acétate d'éthyle 70-30 (en volumes) pour les fractions 1 à 50 et un mélange de cyclohexane et d'acétate d'éthyle 50-50 (en volumes), pour les fractions suivantes. On recueille des fractions de 100 $cm^3$. Les 30 premières fractions sont éliminées; les 32 suivantes sont réunies et concentrées sous pression réduite (2,7 kPa) à une température voisine de 35°C. On obtient ainsi 5 g d'une

meringue blanche qui est triturée avec 2 fois 100 $cm^3$ d'oxyde de diisopropyle puis séchée sous pression réduite (2,7 kPa) à une température voisine de 40°C. On obtient ainsi 4,75 g de N-(30-nor 3β-acétoxy-20-oxolupan-28-oyl]-11-aminoundécanoate de méthyle sous forme d'une meringue blanche [Rf=0,23 ; chromatographie sur couche mince de silice ; éluant : cyclohexane acétate d'éthyle 8-2 (en volumes)].

Le chlorure de 30-nor 3β-acétoxy-20-oxolupan-28-oyle est obtenu de la manière suivante :

1,5 $cm^3$ de chlorure d'oxalyle sont ajoutés en 5 minutes à 150 $cm^3$ d'une solution dans le dichlorométhane de d'acide 30-nor 3β-acétoxy-20-oxolupan-28-oïque. La solution est alors agitée 20 heures à une température voisine de 20°C. Le mélange réactionnel est concentré à sec sous pression réduite (2,7 kPa) à une température voisine de 40°C. La meringue blanche obtenue est triturée avec 2 fois 200 $cm^3$ de cyclohexane puis le solvant est évaporé à sec sous pression réduite (2,7 kPa) à une température voisine de 40°C. On obtient ainsi 3,7 g de chlorure de 30-nor 3β-acétoxy-20-oxolupan-28-oyle sous la forme d'une meringue blanche.

L'acide 30-nor 3β-acétoxy-20-oxolupan-28-oïque est préparé d'après A. VYSTRCIL et M. BUDESINSY, Collect. Czech. Chem. Commun., 35, 295 (1970) .

## Exemple 25

A une solution de 1 g de N-[3β-acétoxylup-20(29)-èn-28-oyl]-β-alanine dans 100 $cm^3$ de dichlorométhane, on ajoute 420 mg de chlorhydrate de 8-aminooctanoate de méthyle, 240 mg d'hydrate de 1-hydroxybenzotriazole, 760 mg de chlorhydrate de 1-éthyl 3-(3-diméthylaminopropyl)carbodiimide et 1 $cm^3$ de triéthylamine. La solution est agitée pendant 12 heures à une température voisine de 20°C puis on ajoute 100 $cm^3$ d'eau distillée. La phase organique est décantée et la phase aqueuse est extraite par 150 $cm^3$ au total de chlorure de méthylène. Les extraits organiques sont rassemblés, séchés sur du sulfate de magnésium anhydre et concentrés à sec sous pression réduite (2,7 kPa) à une température voisine de 35°C. Le résidu obtenu (1,4 g) est chromatographié sur une colonne de 4 cm de diamètre contenant 125 g de silice (0,02-0,045 mm), éluée avec un mélange de cyclohexane et d'acétate d'éthyle 60-40 (en volumes), en recueillant des fractions de 50 $cm^3$. Les 36 premières fractions sont éliminées; les 15 suivantes sont réunies et concentrées sous pression réduite (2,7 kPa) à une température voisine de 35°C. On obtient ainsi 1,1 g de N-[N-(3β-acétoxylup-20(29)-èn-28-oyl)-3-aminopropionoyl]-8-aminooctanoate de méthyle sous forme d'une meringue blanche [Rf=0,5 ; chromatographie sur couche mince de silice ; éluant : chlorure de méthylène méthanol 90-10 (en volumes)].

Une solution de 1,1 g de N-[N-(3β-acétoxylup-20(29)-èn-28-oyl)-3-aminopropionoyl]-8-aminooctanoate de méthyle dans 15 $cm^3$ de méthanol, 7,5 $cm^3$ de tétrahydrofurane et 1,9 $cm^3$ de soude 4N est agitée pendant 15 heures à une température voisine de 20°C puis on ajoute 20 $cm^3$ de méthanol, 3 $cm^3$ d'acide chlorhydrique 5N et 50 $cm^3$ d'eau distillée. Après 30 minutes d'agitation, le solide formé est séparé par filtration, lavé par 50 $cm^3$ au total d'eau distillée et séché sous pression réduite (13,5 Pa) à une température voisine de 40°C. On obtient ainsi 845 mg d'acide N-[N-(3β-hydroxylup-20(29)-èn-28-oyl)-3-aminopropionoyl]-8-aminooctanoïque, sous la forme d'un solide blanc fondant vers 115°C.

L'acide N-[3β-acétoxylup-20(29)-èn-28-oyl]-3-aminopropionique peut être préparée de la manière suivante :

Après avoir chauffé au reflux pendant 3 heures un mélange de 190 mg d'acide 3-aminopropionique et 410 mg de chlorotriméthylsilane dans 50 $cm^3$ de dichlorométhane, la solution est refroidie vers 20°C et on ajoute 1 g de chlorure de 3-acétoxylup-20(29)-èn-28-oyle en solution dans 30 $cm^3$ de dichlorométhane puis 0,81 $cm^3$ de triéthylamine en solution dans 15 $cm^3$ de dichlorométhane. L'agitation est maintenue pendant 15 heures à une température voisine de 20°C et le solvant est évaporé à sec sous pression réduite (2,7 kPa) à une température voisine de 35°C. Le résidu est chromatographié sur une colonne de 5 cm de diamètre contenant 250 g de silice (0,02-0,045 mm), éluée en présence du mélange chlorure de méthylène méthanol 90-10 (en volumes), en recueillant des fractions de 50 $cm^3$. Les 9 premières fractions sont éliminées, les 15 suivantes sont rassemblées et concentrées à sec sous pression réduite (2,7 kPa) à une température voisine de 35°C. On obtient ainsi 1,04 g d'acide N-(3β-acétoxylup-20(29)-èn-28-oyl)-3-aminopropionique sous la forme d'un meringue blanche [Rf=0,3 ; chromatographie sur couche mince de silice ; éluant : chlorure de méthylèneméthanol 90-10 (en volumes)].

## Exemple 26

A une solution de 1 g de N-(3β-acétoxylup-20(29)-èn-28-oyl)glycine dans 100 $cm^3$ de dichlorométhane, on ajoute 410 mg de chlorhydrate de 8-aminooctanoate de méthyle, 243 mg d'hydrate de 1-hydroxybenzotriazole, 690 mg de chlorhydrate de 1-éthyl 3-(3-diméthylaminopropyl)carbodiimide et 0,5 $cm^3$ de triéthylamine. La solution est agitée pendant 12 heures à une température voisine de 20°C puis on ajoute 100 $cm^3$ d'eau distillée. La phase organique est décantée et la phase aqueuse est extraite par 150 $cm^3$ au total de chlorure

de méthylène. Les extraits organiques sont rassemblés, séchés sur du sulfate de magnésium anhydre et concentrés à sec sous pression réduite (2,7 kPa) à une température voisine de 35°C. Le résidu obtenu (1,26 g) est chromatographié sur un colonne de 5 cm de diamètre contenant 200 g de silice (0,02-0,045 mm), éluée avec un mélange de cyclohexane et d'acétate d'éthyle 50-50 (en volumes) en recueillant des fractions de 50 $cm^3$. Les 17 premières fractions sont éliminées; les 27 suivantes sont réunies et concentrées sous pression réduite (2,7 kPa) à une température voisine de 35°C. On obtient ainsi 1,2 g de N-[N-(3β-acétoxylup-20(29)-èn-28-oyl)glycyl]-8-aminoocta-noate de méthyle sous la forme d'une meringue blanche (Rf=0,2 ; chromatographie sur couche mince de silice ; éluant : cyclohexane acétate d'éthyle 50-50 (en volumes)].

Une solution de 1,2 g de N-[N-(3β-acétoxylup-20(29)-èn-28-oyl) glycyl]-8-aminooctanoate de méthyle dans 17 $cm^3$ de méthanol, 8,5 $cm^3$ de tétrahydrofurane et 2,1 $cm^3$ de soude 4N est agitée pendant 15 heures à une température voisine de 20°C puis on ajoute 20 $cm^3$ de méthanol, 4 $cm^3$ d'acide chlorhydrique 5N et 50 $cm^3$ d'eau distillée et 50 $cm^3$ d'acétate d'éthyle. La phase organique est décantée et la phase aqueuse est extraite par 200 $cm^3$ au total d'acétate d'éthyle. Les extraits organiques sont rassemblés, séchés sur du sulfate de magnésium anhydre et évaporés à sec sous pression réduite (2,7 kPa) à une température voisine de 35°C. Le résidu est dissous dans 4 $cm^3$ d'éthanol et 40 $cm^3$ d'eau distillée sont additionnés. Après 30 minutes d'agitation, le solide est séparé par filtration, lavé par 50 $cm^3$ au total d'eau distillée et séché sous pression réduite (13,5 Pa) à une température voisine de 40°C. On obtient ainsi 883 mg d'acide N-[N-(3β-hydroxylup-20(29)-èn-28-oyl)-glycyl]-8-aminooctanoïque, sous la forme d'un solide blanc, fondant vers 130°C.

La N-(3β-acétoxylup-20(29)-èn-28-oyl)glycine peut être préparée de la manière suivante :

Après avoir chauffé au reflux pendant 6 heures 30 minutes un mélange de 830 mg de glycine et 2,28 g de chlorotriméthylsilane dans 300 $cm^3$ de dichlorométhane, la solution est refroidie vers 20°C et on ajoute 5,17 g de chlorure de 3-acétoxylup-20(29)-èn-28-oyle puis 3,54 $cm^3$ de triéthylamine. L'agitation est maintenue pendant 48 heures à une température voisine de 20°C et le solvant est évaporé à sec sous pression réduite (2,7 kPa) à une température voisine de 35°C. Le résidu est chromatographié sur une colonne de 5 cm de diamètre contenant 250 g de silice (0,02-0,045 mm), éluée avec un mélange chlorure de méthylène méthanol 90-10 (en volumes), en recueillant des fractions de 50 $cm^3$. Les 10 premières fractions sont éliminées, les 15 suivantes sont rassemblées et concentrées à sec sous pression réduite (2,7 kPa) à une température voisine de 35°C. On obtient ainsi 1,04 g de N-[3β-acétoxylup-20(29)-èn-28-oyl]glycine sous la forme d'une meringue blanche [Rf= 0,3 ; chromatographie sur couche mince de silice ; éluant : chlorure de méthylène méthanol (90-10 en volumes)].

## Exemple 27

A une solution de 1,13 g d'acide N-(3β-acétoxylup-20(29)-èn-28-oyl)-8-aminooctanoïque, dans 100 $cm^3$ de dichlorométhane, on ajoute 250 mg de chlorhydrate de glycinate de méthyle, 240 mg d'hydrate de 1-hydroxybenzotriazole, 690 mg de chlorhydrate de 1-(3-diméthylaminopropyl)-3-éthylcarbodiimide et 0,5 $cm^3$ de triéthylamine. La solution est agitée pendant 12 heures à une température voisine de 20°C puis on ajoute 100 $cm^3$ d'eau distillée. La phase organique est décantée et la phase aqueuse est extraite par 150 $cm^3$ au total de chlorure de méthylène. Les extraits organiques sont rassemblés, séchés sur du sulfate de magnésium anhydre et évaporés à sec sous pression réduite (2,7 kPa) à une température voisine de 35°C. Le résidu obtenu (1,3 g) est chromatographié sur une colonne de 4 cm de diamètre contenant 125 g de silice (0,02-0,045 mm), éluée avec un mélange de cyclohexane et d'acétate d'éthyle 30-70 (en volumes), en recueillant des fractions de 50 $cm^3$. Les 15 premières fractions sont éliminées; les 17 suivantes sont réunies et concentrées sous pression réduite (2,7 kPa) à une température voisine de 35°C. On obtient ainsi 1,2 g de N-[3β-acétoxylup-20(29)-èn-28-oyl]-8-aminooctanoylglycinate de méthyle, sous la forme d'une meringue blanche (Rf=0,3 ; chromatographie sur couche mince de silice ; éluant : cyclohexane acétate d'éthyle (50-50 en volumes)].

Une solution de 1,2 g de N-[N-(3β-acétoxylup-20(29)-èn-28-oyl)-8-aminooctanoyl]glycinate de méthyle dans 17 $cm^3$ de méthanol, 8,5 $cm^3$ de tétrahydrofurane et 2,1 $cm^3$ de soude 4N est agitée pendant 15 heures à une température voisine de 20°C puis on ajoute 10 $cm^3$ de méthanol, 2,5 $cm^3$ d'acide chlorhydrique 5N et 40 $cm^3$ d'eau distillée. Après 30 minutes d'agitation, le solide est séparé par filtration, lavé par 50 $cm^3$ au total d'eau distillée et séché à l'air à une température voisine de 20°C. On obtient ainsi 1,03 g d'acide N-[N-(3β-hydroxylup-20(29)-èn-28-oyl)-8-aminooctanoyl]glycine, sous la forme d'un solide blanc, fondant vers 132°C.

L'acide N-[3β-acétoxylup-20(29)-èn-28-oyl]-8-aminooctanoïque peut être préparé de la manière suivante :

Après avoir chauffé au reflux pendant 3 heures un mélange de 330 mg d'acide 8-aminooctanoïque et 410 mg de chlorotriméthylsilane dans 50 $cm^3$ de dichlorométhane, la solution est refroidie vers 20°C et on ajoute 1 g de chlorure de 3β-acétoxylup-20(29)-èn-28-oyle puis 0,81 $cm^3$ de triéthylamine. L'agitation est maintenue pendant 15 heures à une température voisine de 20°C et le solvant est évaporé à sec sous pression réduite (2,7 kPa) à une température voisine de 35°C. Le résidu est chromatographié sur une colonne de 5 cm de diamètre contenant 200 g de silice (0,02-0,045 mm), éluée en présence du mélange cyclohexane-acétate d'éthyle 60-40 (en volumes), en recueillant des fractions de 50 $cm^3$. Les 18 premières fractions sont éliminées, les 31 suivantes sont rassemblées et concentrées à sec sous pression réduite (2,7 kPa) à une température voisine de 35°C. On obtient ainsi 1,1 g d'acide N-[3β-acétoxylup-20(29)-èn-28-oyl]-8-aminooctanoïque sous la forme d'un meringue blanche [Rf=0,4 ; chromatographie sur couche mince de silice ; éluant : cyclohexane acétate d'éthyle 60-40 (en volumes)].

**Exemple 28**

A une solution de 1,05 g d'acide N-[3β-acétoxylup-20(29)-èn-28-oyl] 4-aminobutanoïque dans 60 $cm^3$ de tétrahydrofurane, on ajoute 0,4 g de 4-aminophénylacétate de méthyle puis 0,31 g de 1-hydroxybenzotriazole. Après 15 minutes d'agitation à une température voisine de 20°C, on ajoute 0,84 g de chlorhydrate de 1-(3-diméthylaminopropyl)-3-éthylcarbodiimide puis 1 $cm^3$ de triéthylamine. La solution est agitée 240 heures à une température voisine de 20°C. Le mélange réactionnel est évaporé sous pression réduite (2,7 kPa) à une température voisine de 40°C. Au solide pâteux résiduel,on ajoute 100 $cm^3$ de dichlorométhane. La phase organique est lavée par 150 $cm^3$ au total d'eau distillée, séchée sur du sulfate de magnésium et évaporée à sec sous pression réduite (2,7 kPa) à une température voisine de 40°C. Le solide crème obtenu (1,4 g) est chromatographié sur une colonne de 2 cm de diamètre et 40 cm de haut de silice (0,02-0,045 mm) éluée par un mélange de cyclohexane et d'acétate d'éthyle 1-1 (en volumes), en recueillant des fractions de 70 $cm^3$. Les 8 premières fractions sont éliminées, les 2 suivantes sont réunies et concentrées sous pression réduite (2,7 kPa) à une température voisine de 40°C. On obtient 750 mg de N-[N-(3β-acétoxylup-20(29)-èn-28-oyl)-4 aminobutanoyl]-4-aminophénylacétate de méthyle sous la forme d'une huile jaune pâle visqueuse [Rf=0,69 ; chromatographie sur couche mince de silice : éluant : cyclohexane/ - acétate d'éthyle 1-1 (en volumes)].

10 $cm^3$ de soude 4N sont ajoutés en 5 minutes environ à une solution de 1,1 g de N-[N-(3β-acétoxylup-20(29)-èn-28-oyl)-4 aminobutanoyl)-4-aminophénylacétate de méthyle dans 20 $cm^3$ de méthanol et 20 $cm^3$ de tétrahydrofurane et le mélange réactionnel est agité pendant 72 heures à une température voisine de 20°C. Le mélange réactionnel est acidifié à un pH voisin de 2 à l'aide d'acide chlorhydrique 5N puis on ajoute 100 $cm^3$d'eau distillée. Après 3 heures d'agitation,le solide est séparé par filtration et rincé par 100 $cm^3$ au total d'eau distillée. Le solide est trituré avec 30 $cm^3$ d'oxyde de diisopropyle, filtré, rincé par 30 $cm^3$ au total d'oxyde de diisopropyle, puis séché sous pression réduite (13,5 Pa) à une température voisine de 40°C. On obtient ainsi 500 mg d'acide N-[N-(3β-hydroxylup-20(29)-èn-28-oyl)-4-aminobutanoyl]-4-aminophénylacétique fondant à une température voisine de 190°C (pâteux).

L'acide N-(3β-acétoxylup-20(29)-èn-28-oyl)-4-aminobutanoïque peut être obtenu de la manière suivante :

Après avoir chauffé 4 heures au reflux un mélange de 0,7 g d'acide 4-aminobutyrique et 1,68 $cm^3$ de chlorotriméthysilane dans 180 $cm^3$ de chloroforme, la solution est refroidie vers 20°C et on ajoute une solution de 3,3 g de chlorure de 3-acétoxylup-20(29)-èn-28-oyle dans 90 $cm^3$ de chloroforme, puis 3 $cm^3$ de triéthylamine. L'agitation est maintenue pendant 30 heures à une température voisine de 20°C. Le mélange réactionnel est lavé par 450 $cm^3$ au total d'eau distillée. La phase organique est séchée sur du sulfate de magnésium anhydre puis évaporée à sec sous pression réduite (2,7 kPa) à une température voisine de 35°C. Le résidu obtenu (4,3 g) est chromatographié sur une colonne de 3 cm de diamètre et 40 cm de haut contenant de la silice (0,02-0,045 mm), éluée avec un mélange de dichlorométhane et de méthanol 95-5 (en volumes), en recueillant des fractions de 50 $cm^3$. Les 6 premières fractions sont éliminées; les 44 suivantes sont réunies et concentrées sous pression réduite (2,7 kPa) à une température voisine de 35°C. On obtient ainsi 2,3 g d'acide N-[3β-acétoxylup-20(29)-èn-28-oyl] 4-aminobutanoïque sous forme d'une meringue blanche [Rf=0,51 ; chromatographie sur couche mince de silice ; éluant : dichlorométhane-méthanol 9-1 (en volumes)].

Le 4-aminophénylacétate de méthyle peut être obtenu selon H. Salkowski Chem. Ber., 28, 1917, (1895).

**Exemple 29**

A une solution de 1,05 g d'acide N-(3β-acétoxylup-20(29)-èn-28-oyl) 4-aminobutanoïque dans 100 $cm^3$ de tétrahydrofurane, on ajoute 0,4 g de 4-aminométhylbenzoate de méthyle puis 0,31 g de 1-hydroxybenzotriazole. Après 15 minutes d'agitation à une température voisine de 20°C, on ajoute 0,84 g de chlorhydrate de 1-éthyl-3-(3-diméthylaminopropyl)carbodiimide puis 1 $cm^3$ de triéthylamine. La solution est agitée 72 heures à une température voisine de 20°C. Le mélange réactionnel est concentré à sec sous pression réduite (2,7 kPa) à une température voisine de 40°C. Au solide pâteux résiduel, on ajoute 100 $cm^3$ de dichlorométhane. La phase organique est lavée par 150 $cm^3$ au total d'eau distillée, séchée sur du sulfate de magnésium et concentrée à sec sous pression réduite (2,7 kPa) à une température voisine de 40°C. Le solide crème obtenu (1,4 g) et chromatographié sur une colonne de 2 cm de diamètre et 40 cm de haut de silice (0,02-0,045 mm) éluée par un

mélange de cyclohexane et d'acétate d'éthyle 1-1 (en volumes), en recueillant des fractions de 70 $cm^3$. Les 15 premières fractions sont éliminées, les 24 suivantes sont réunies et concentrées sous pression réduite (2,7 kPa) à une température voisine de 40°C. On obtient 1,15 g de N-[N-(3β-acétoxylup-20(29)-èn-28-oyl)-4-aminobutanoyl]-4-aminométhylbenzoate de méthyle sous la forme d'une meringue blanche [Rf=0,15 ; chromatographie sur couche mince de silice ; éluant cyclo-hexane-acétate d'éthyle 1:1 (en volumes)].

15 $cm^3$ de soude 4N sont ajoutés en 5 minutes environ à une solution de 1,1 g de N-[N-(3β-acétoxylup-20(29)-èn-28-oyl)-4-aminobutanoyl]-4-amino méthylbenzoate de méthyle dans 30 $cm^3$ de méthanol et 30 $cm^3$ de tétra-hydrofurane et le mélange réactionnel est agité pendant 24 heures à une température voisine de 20°C. Le mélange réactionnel est acidifié à un pH voisin de 2 à l'aide d'acide chlorhydrique 5N puis on ajoute 150 $cm^3$ d'eau distillée. Après 3 heures d'agitation,le solide est séparé par filtration et rincé par 150 $cm^3$ au total d'eau distillée. Le solide est trituré avec 30 $cm^3$ d'oxyde de diisopropyle, filtré, rincé par 75 $cm^3$ au total d'oxyde de diisopropyle, puis séché sous pression réduite (13,5 Pa) à une température voisine de 40°C. On obtient ainsi 900 mg d'acide N-[N-(3β-hydroxylup-20(29)-èn-28-oyl]-4-aminobutanoyl]-4-aminométhylbenzoïque fondant à une température voisine de 180-190°C (pâteux).

Le 4-aminométhylbenzoate de méthyle peut être obtenu de la manière suivante:

A 30 $cm^3$ de méthanol refroidis à une température voisine de -30°C, on ajoute en 10 minutes, 3,3 $cm^3$ de chlorure de thionyle. L'agitation est maintenue 30 minutes à une température voisine de -23°C puis on ajoute 4,7 g d'acide 4-aminométhylbenzoïque. On laisse revenir à une température voisine de 20°C en 1 heure. Le mélange est additionné de 15 $cm^3$ de méthanol et l'agitation est maintenue 15 heures à une température voisine de 20°C. Le solide est séparé par filtration, rincé par 90 $cm^3$ au total de méthanol puis 150 $cm^3$ au total d'oxyde de diéthyle. Le solide est séché sous pression réduite (13,5 Pa) à une température voisine de 20°C. On obtient ainsi 3,2 g de chlorhydrate de 4-aminométhylbenzoate de méthyle sous la forme d'un solide blanc fondant à une température voisine de 270°C.

## Exemples 30 à 49

En opérant par analogie avec l'exemple 27 les dérivés suivants ont été préparés :

**30)**

La N'-[N-[3β-hydroxylup-20(29)-èn-28-oyl]-8-aminooctanoyl)-β-alanine, a été préparée à partir d'acide N-[3β-acétoxylup-20(29)-èn-28-oyl]-8-aminooctanoïque et de β-alaninate de méthyle, pF=138°C, (R=51 %).

**31)**

L'acide N'-[N-[3β-hydroxylup-20(29)-èn-28-oyl]-8-aminooctanoyl]-L-aspartique, a été préparé à partir d'acide N-[3β-acétoxylup-20(29)-èn-28-oyl]-8-aminooctanoïque et de L-aspartate de dibenzyle, pF=140°C, (R=47 %).

**32)**

La N'-(N-[3β-hydroxylup-20(29)-èn-28-oyl]-8-aminooctanoyl]-L-phénylglycine, a été préparée à partir d'acide N-[3β-acétoxylup-20(29)-èn-28-oyl]-8-aminooctanoïque et de L-phénylglycinate de méthyle, pF=135°C, (R=45 %).

**33)**

La N'-[N-[3β-hydroxylup-20(29)-èn-28-oyl]-8-aminooctanoyl]-L-alanine, a été préparée à partir d'acide N-[3β-acétoxylup-20(29)-èn-28-oyl]-8-aminooctanoïque et de L-alaninate de benzyle, pF=120°C, (R=34 %).

**34)**

La N'-[N-[3β-hydroxylup-20(29)-èn-28-oyl]-8-aminooctanoyl]-D-sérine, a été préparée à partir d'acide N-[3β-acétoxylup-20(29)-èn-28-oyl]-8-aminooctanoïque et de D-sérinate de méthyle, pF=252°C, (R=8 %).

**35)**

Le N'-[N-[3β-hydroxylup-20(29)-èn-28-oyl]-8-aminooctanoyl]-L-homosérinate de sodium, a été préparé à partir d'acide N-[3β-acétoxylup-20(29)-èn-28-oyl]-8-aminooctanoïque et d'α-amino-γ-butyrolactone, pF=130°C, (R=45 %).

**36)**

La N'-[N-[3β-hydroxylup-20(29)-èn-28-oyl]-8-aminooctanoyl]-D,L-isosérine a été préparée à partir d'acide N-[3β-acétoxylup-20(29)-èn-28-oyl]-8-aminooctanoïque et de D,L-isosérinate de méthyle, pF=140°C, (R=56 %).

**37)**

La N'-[N-[3β-hydroxylup-20(29)-èn-28-oyl]-8-aminooctanoyl]-L-lysine, a été préparée à partir d'acide N-[3β-acétoxylup-20(29)-èn-28-oyl]-8-aminooctanoïque et de N-trifluoroacétyl-L-lysinate de méthyle,(R=16 %).

**38)**

La N'-[N-[3β-hydroxylup-20(29)-èn-28-oyl]-8-aminooctanoyl]-L-asparagine, a été préparée à partir d'acide N-[3β-acétoxylup-20(29)-èn-28-oyl]-8-amiooctanoïque et de L-asparaginate de p-nitrobenzyle, pF=120°C, (R=34 %).

**39)**

La N'-[N-[3β-hydroxylup-20(29)-èn-28-oyl)-8-aminooctanoyl]sarcosine, a été préparée à partir d'acide N-[3β-acétoxylup-20(29)-èn-28-oyl]-8-aminooctanoïque et de sarcosinate d'éthyle, pF=120°C, (R=85 %).

**40)**

La N'-[N-[3β-hydroxylup-20(29)-èn-28-oyl]-8-aminooctanoyl]-L-proline, a été préparée à partir d'acide N-[3β-acétoxylup-20(29)-èn-28-oyl]-8-aminooctanoïque et de L-prolinate de méthyle, pF=142°C, (R=72 %).

**41)**

La N'-[N-[3β-hydroxylup-20(29)-èn-28-oyl]-8-aminooctanoyl]-L-phénylalanine a été préparée à partir d'acide N-[3β-acétoxylup-20(29)-èn-28-oyl]-8-aminooctanoïque et de L-phénylalaninate de méthyle, pF=116°C, (R=58 %).

**42)**

La N'-[N-[3β-hydroxylup-20(29)-èn-28-oyl]-8-aminooctanoyl]-L-valine, a été préparée à partir d'acide N-[3β-acétoxylup-20(29)-èn-28-oyl]-8-aminooctanoïque et de L-valinate de méthyle, pF=145°C,(R=80 %).

**43)**

L'acide N'-[N-[3β-hydroxylup-20(29)-èn-28-oyl]-8-aminooctanoyl]-3-amino-2-hydroxy-3-phénylpropionique (2S,3S), a été préparé à partir d'acide N-[3β-acétoxylup-20(29)-èn-28-oyl)-8-aminooctanoïque et de 3-amino-2-hydroxy-3-phénylpropionate de méthyle (2S,3S), pF=120°C, (R=58 %).

**44)**

L'acide N'-[N-[3β-hydroxylup-20(29)-èn-28-oyl]-4-aminobutyryl]-6-aminohexanoïque a été préparé à partir d'acide N-[3β-acétoxylup-20(29)-èn-28-oyl]-4-aminobutyrique et de 6-aminohexanoate de méthyle, pF=140°C, (R=100 %).

L'acide N-[3β-acétoxylup-20(29)-èn-28-oyl]-4-aminobutyrique a été préparé à partir de chlorure de 3β-acétoxylup-20(29)-èn-28-oyle et d'acide 4-aminobutyrique, (Rf=0,43 ; chromatographie sur couche mince de silice ; éluant : chlorure de méthylène, méthanol 90-10 (en volumes)].

**45)**

L'acide N'-[N-[3β-hydroxylup-20(29)-èn-28-oyl]-5-aminopentanoyl]-5-aminopentanoïque a été préparé à partir d'acide N-[3β-acétoxylup20(29)-èn-28-oyl]-5-aminopentanoïque et de 5-aminopentanoate de méthyle, pF=132°C, (R=85 %).

L'acide N-[3β-acétoxylup-20(29)-èn-28-oyl]-5-aminopentanoïque a été préparé à partir de chlorure de 3β-acétoxylup-20(29)-èn-28-oyle et d'acide 5-aminopentanoïque, [Rf=0,51 ; chromatographie sur couche mince de silice ; éluant : chlorure de méthylène, méthanol 90-10 (en volumes)].

**46)**

L'acide N'-[N-[3β-hydroxylup-20(29)-èn-28-oyl]-7-aminoheptanoyl]-3-aminopropionique a été préparé à partir d'acide N-[3β-acétoxylup-20(29)-èn-28-oyl]-7-aminoheptanoïque et de 3-aminopropionate de méthyle, pF=194°C, (R=36 %).

L'acide N-[3β-acétoxylup-20(29)-èn-28-oyl]-7-aminoheptanoïque a été préparé à partir de chlorure de 3β-acétoxylup-20(29)-èn-28-oyle et d'acide 7-aminoheptanoïque, [Rf=0,26 ; chromatographie sur couche mince de silice ; éluant : cyclohexane, acétate d'éthyle 60-40 (en volumes)].

**47)**

L'acide N'-[N-[3β-hydroxylup-20(29)-èn-28-oyl]-6-aminohexanoyl]-4-aminobutyrique a été préparé à partir d'acide N-(3β-acétoxylup-20(29)-èn-28-oyl]-6-aminohexanoïque et de 4-aminobutyrate de méthyle, pF=206°C, (R=10 %).

L'acide N-[3β-acétoxylup-20(29)-èn-28-oyl]-6-aminohexanoïque a été préparé à partir de chlorure de 3β-acétoxylup-20(29)-èn-28-oyle et d'acide 6-aminohexanoïque, [Rf=0,2 ; chromatographie sur couche mince de silice ; éluant : cyclohexane, acétate d'éthyle 60-40 (en volumes)].

**48)**

L'acide N'-[N-[3β-hydroxylup-20(29)-èn-28-oyl]-8-aminooctanoyl]-3-aminopropionique a été préparé à partir d'acide N-[3β-acétoxylup-20(29)-èn-28-oyl]-8-aminooctanoïque et de 3-aminopropionate de méthyle, pF=138°C, (R=51 %).

**49)**

L'acide N'-[N-[3β-hydroxylup-20(29)-èn-28-oyl]-8-aminooctanoyl]-4-aminobutyrique a été préparé à partir d'acide N-[3β-acétoxylup-20(29)-èn-28-oyl]-8-aminooctanoïque et de 4-aminobutyrate de méthyle,

pF=168°C, (R=23 %).

**Exemple 50**

La N'-[N-[3β-hydroxylup-20(29)-èn-28-oyl]-8-aminooctanoyl]-L-thréonine peut être obtenue de la façon suivante:
à une solution de 1 g d'acide N-[3β-acétoxylup-20(29)-èn-28-oyl)-8-aminooctanoïque, dans 50 $cm^3$ de dichlorométhane, on ajoute, sous un courant d'argon, 470 mg d'hémioxalate de L-thréoninate de benzyle, 240 mg d'hydrate de 1-hydroxybenzotriazole, 600 mg de chlorhydrate de 1-(3-diméthylaminopropyl)-3-éthylcarbodiimide et 0,88 $cm^3$ de triéthylamine. La solution est agitée pendant 12 heures à une température voisine de 20°C puis on ajoute 60 $cm^3$ d'eau distillée. La phase organique est décantée, séchée sur du sulfate de magnésium anhydre et concentrée à sec sous pression réduite (2,7 kPa) à une température voisine de 45°C. Le résidu obtenu (1,36 g) est chromatographié sur une colonne de 4 cm de diamètre contenant 100 g de silice (0,02-0,045 mm), éluée avec un mélange de cyclohexane et d'acétate d'éthyle 30-70 (en volumes) en recueillant des fractions de 40 $cm^3$. Les 10 premières fractions sont éliminées ; les 10 suivantes sont réunies et concentrées sous pression réduite (2,7 kPa) à une température voisine de 45°C. On obtient ainsi 1,12 g d'une meringue blanche qui est mise en solution dans un mélange de 13,5 $cm^3$ de méthanol, de 6,7 $cm^3$ de tétrahydrofurane et de 1,4 $cm^3$ de soude 5N. Après 15 heures sous agitation à une température voisine de 20°C, on ajoute 1,4 $cm^3$ d'acide chlorhydrique 5N et 15 $cm^3$ d'eau distillée. Le méthanol est éliminé sous pression réduite (2,7 kPa) à une température voisine de 45°C. Après 30 minutes d'agitation, le solide obtenu est séparé par filtration, lavé par 35 $cm^3$ au total d'eau distillée et séché à l'air à une température voisine de 20°C. On obtient ainsi 880 mg de N'-[N-[3β-hydroxylup-20(29)-èn-28-oyl]-8-aminooctanoyl]-L-thréonine, sous la forme d'un solide blanc, fondant vers 130°C.

**Exemple 51**

La N'-[N-[3β-hydroxylup-20(29)-èn-28-oyl]-8-aminooctanoyl]-L-statine peut être obtenue de la façon suivante :

A une solution de 500 mg d'acide N-[3β-acétoxylup-20(29)-èn-28-oyl]-8-aminooctanoïque, dans 30 $cm^3$ de dichlorométhane, on ajoute 211 mg de chlorhydrate de L-statinate de méthyle, 105 mg d'hydrate de 1-hydroxybenzotriazole, 298 mg de chlorhydrate de 1-(3-diméthylaminopropyl)-3-éthylcarbodiimide et 0,38 $cm^3$ de triéthylamine. La solution est agitée pendant 18 heures à une température voisine de 20°C puis on ajoute 50 $cm^3$ d'eau distillée. La phase organique est décantée, séchée sur du sulfate de magnésium anhydre et évaporée à sec sous pression réduite (2,7 kPa) à une température voisine de 40°C. Le résidu obtenu (570 mg) est mis en solution dans un mélange de 15 $cm^3$ de méthanol, de 10 $cm^3$ de tétrahydrofurane et de 10 $cm^3$ de soude 5N. Après 15 heures sous agitation à une température voisine de 20°C, on ajoute 5 $cm^3$ de méthanol, puis 10 $cm^3$ d'acide chlorhydrique 5N et 5 $cm^3$ d'eau distillée. Le mélange est extrait par 60 $cm^3$ d'éther diéthylique. La phase organique est décantée et lavée par 30 $cm^3$ au total d'eau distillée, séchée sur du sulfate de magnésium anhydre, et concentrée à sec sous pression réduite (4,0 kPa) à une température voisine de 40°C. Le résidu (520 mg) est mis en suspension dans 10 $cm^3$ d'éther diéthylique. Après 1 heure d'agitation, le solide obtenu est séparé par filtration, lavé par 5 $cm^3$ d'éther diéthylique et séché sous pression réduite (13,5 Pa) à une température voisine de 20°C. On obtient ainsi 340 mg de N'-[N-[3β-hydroxylup-20(29)-èn-28-oyl]-8-aminooctanoyl]-L-statine sous la forme d'un solide blanc, fondant vers 158°C.

Le L-statinate de méthyle peut être préparé selon la méthode décrite par Y. TAKEMOTO et coll., Tetrahedron Lett., 31 (2), 217, 1990.

**Exemple 52**

En opérant par analogie avec l'exemple 11.

L'acide N-(3β,30-dihydroxylup-20(29)-èn-28-oyl)-10-aminodécanoïque a été préparé à partir de chlorure de 3β,30-diacétoxylup-20(29)-èn-28-oyle et de 10-aminodécanoate de méthyle, pF=130-140°C, (R=14 %).

**Exemple 53**

En opérant par analogie avec l'exemple 11 et l'exemple 27 on prépare la N'-[N-[3β,30-dihydroxylup-20(29)-èn-28-oyl]-8-aminooctanoyl]glycine, pF=154°C, (R=83 %).

**Exemple 54**

Le N-[3β,30-dihydroxylup-20(29)-èn-28-oyl]-10-aminodécanoate de méthyle peut être obtenu de la manière suivante :

Une solution de 760 mg d'acide N-[3β,30-dihydroxylup-20(29)-èn-28-oyl]-10-aminodécanoïque, 0,48 $cm^3$ d'iodure de méthyle, 0,45 $cm^3$ de 1,8-diazabi-cyclo[5.4.0]undéc-7-ène dans 6 $cm^3$ de N,N-diméthylformamide est agitée 4 heures à une température voisine de 20°C. Après addition de 60 $cm^3$ d'eau distillée et de 25 $cm^3$ de chloroforme, la phase organique est décantée. La phase aqueuse est extraite par 25 $cm^3$ au total de chloroforme. Les phases organiques rassemblées sont séchées sur du sulfate de sodium anhydre et concentrées à sec sous pression réduite (2,7 kPa) à une température voisine de 40°C. L'huile jaune obtenue (1,34 g) est chromatographiée sur une colonne de 3,2 cm de diamètre contenant 90 g de silice (0,02-0,04 mm) éluée avec un mélange de dichlorométhane et d'acétate d'éthyle 50-50 (en volumes) en recueillant des fractions de 10 $cm^3$. Après élimination des 52 premières fractions les 14 suivantes sont réunies et concentrées à sec sous pression réduite (2,7 kPa) à une température voisine de 35°C. Les 800 mg de meringue obtenus sont mis en solution dans 8 $cm^3$ de méthanol et on ajoute, goutte-à-goutte, 8 $cm^3$ d'eau distillée. Après 30 minutes d'agitation les cristaux obtenus sont séparés par filtration, lavés par 10 $cm^3$ au total d'eau distillée et séchés sous pression réduite (13,5 Pa) à une température voisine de 30°C. On obtient ainsi 800 mg de N-[3β,30-dihydroxylup-20(29)-èn- 28-oyl]-10-aminodécanoate de méthyle, sous la forme d'un solide blanc fondant vers 100°C.

**Exemple 55 et 56**

En opérant par analogie avec l'exemple 54, les produits suivants sont préparés :

**55)**

Le N'-[N-[3β,30-dihydroxylup-20(29)-èn-28-oyl]-8-aminooctanoyl]glycinate de méthyle a été préparé à partir d'acide N'-[N-[3β,30-dihydroxylup-20(29)-èn-28-oyl]-8-aminooctanoyl)glycine, pF=115°C, (R=77 %).

**56)**

Le N'-[N-(3β-hydroxylup-20(29)-èn-28-oyl]-8-aminooctanoyl]glycinate de méthyle a été préparé à partir d'acide N'-(N-[3β-hydroxylup-20(29)-èn-28-oyl]- 8-aminooctanoyl]glycine, pF=100°C, (R=90 %).

**Exemple 57**

En opérant par analogie avec l'exemple 19, on prépare le produit suivant :

L'acide N-[3β,30-dilnydroxylup-20(29)-èn-28-oyl]-11-amino 2,2-diméthylundécanoïque est préparé à partir de 11-amino-2,2-diméthylundécanoate de méthyle et d'acide 3β,30-diacétoxylup-20(29)-èn-28-oïque. pF=190°C.

**Exemple 58**

L'acide N-[3β-hydroxylup-20(29)-èn-28-oyl]-7-aminoheptylcarbamoylacétique peut être obtenu de la manière suivante :

A une solution de 3,5 g de chlorure de 3β-acétoxylup-20(29)-èn-28-oyle dans 60 $cm^3$ de chloroforme, on ajoute sous agitation, 1,3 g de 7-aminoheptylcarbamoylacétate d'éthyle, puis 1,1 $cm^3$ de triéthylamine. Après 12 heures sous agitation à une température voisine de 25°C, 50 $cm^3$ d'eau distillée sont ajoutés, la phase organique est décantée et la phase aqueuse est extraite par 25 $cm^3$ au total de chloroforme. Les extraits organiques sont rassemblés, lavés par 40 $cm^3$ au total d'eau distillée, séchés sur du sulfate de magnésium anhydre et le solvant est évaporé sous pression réduite (2,7 kPa) à une température voisine de 35°C. La meringue beige obtenue (1,8 g) est chromatographiée sur une colonne de 3 cm de diamètre contenant 90 g de silice (0,02-0,045 mm), éluée avec un mélange d'acétate d'éthyle et de cyclohexane 75-25 (en volumes), en recueillant des fractions de 10 $cm^3$. Les 42 premières fractions sont éliminées, les 15 suivantes sont réunies et concentrées sous pression réduite (2,7 kPa) à une température voisine de 35°C. Le résidu obtenu (830 mg) est mis en solution dans un mélange de 8 $cm^3$ de méthanol, 4 $cm^3$ de tétrahydrofurane et 2,75 $cm^3$ de soude 4N. Après 12 heures sous agitation à une température voisine de 25°C, le solvant est évaporé à sec sous pression réduite (2,7 kPa) à une température voisine de 35°C. Le résidu est repris par 40 $cm^3$ d'eau distillée et acidifié à l'aide de 3 $cm^3$ d'acide chlorhydrique 4N. Les cristaux obtenus sont séparés par filtration, lavés par 90 $cm^3$ au total d'eau distillée et séchés sous pression réduite (13,5 Pa) à une température voisine de 35°C. On obtient ainsi 670 mg d'acide N-[3β-hydroxylup-20(29)-èn-28-oyl]-7-aminoheptylcarbamoylacétique sous la forme d'un solide blanc, fondant vers 140°C.

Le 7-aminoheptylcarbamoylacétate d'éthyle peut être préparé de la manière suivante :

On ajoute, goutte-à-goutte, 1,3 $cm^3$ de chlorure de malonate d'éthyle à un mélange de 3,4 g de mono N-benzyloxycarbonyl-1,7-heptyldiamine, 1,68 $cm^3$ de triéthylamine dans 120 $cm^3$ de tétrahydrofurane et la solution est agitée 48 heures à une température voisine de 25°C. Après addition de 400 $cm^3$ d'eau distillée et de 200 $cm^3$ de chloroforme, la phase organique est décantée et la phase aqueuse est extraite par 400 $cm^3$ au total de chloroforme. Les extraits organiques sont rassemblés, lavés par 400 $cm^3$ au total d'eau distillée, séchés sur du sulfate de sodium anhydre et évaporés sous pression réduite (12,7 kPa) à une température voisine de 35°C. L'huile orangée obtenue (4,0 g) est chromatographiée sur une colonne de 3,5 cm de diamètre contenant 200 g de silice (0,02-0,045 mm), éluée avec de l'acétate d'éthyle, en recueillant des fractions de 15 $cm^3$. Les 47 premières fractions sont éliminées, les 17 suivantes sont réunies et concentrées sous pression réduite (2,7 kPa) à une température voisine de 35°C. L'huile jaune obtenue (1,62 g) est mise en solution dans un mélange de 5 $cm^3$ d'acide acétique et de 5 $cm^3$ d'acide bromhydrique à 30 % (en solution dans l'acide acétique) et est agitée pendant 15 minutes à une température voisine de 25°C. Le solvant est éliminé sous pression réduite (2,7 kPa) à une température voisine de 45°C. On obtient ainsi 1,23 g de 7-aminoheptylcarbamoylacétate d'éthyle sous forme d'un miel épais utilisé tel quel.

La mono N-benzyloxycarbonyl-1,7-heptyldiamine peut être préparée de la manière suivante :

A une solution de 10 g d'acide N-benzyloxycarbonyl-8-aminocaprylique dans 500 $cm^3$ de chlorure de méthylène, on ajoute 2 g de chlorure d'ammonium, 5,2 g d'hydrate de 1-hydroxybenzotriazole, 13 g de chlorhydrate de 1-(3-diméthylaminopropyl)-3-éthylcarbodiimide et 9,6 $cm^3$ de triéthylamine. La solution est agitée pendant 18 heures à une température voisine de 20°C puis on ajoute 1 litre d'eau distillée et 12 $cm^3$ d'acide chlorhydrique 4N. La phase organique est décantée et la phase aqueuse est extraite par 200 $cm^3$ au total de chloroforme. Les extraits organiques sont rassemblés, lavés par 200 $cm^3$ au total d'eau distillée, séchés sur du sulfate de magnésium anhydre et évaporés à sec sous pression réduite (2,7 kPa) à une température voisine de 35°C. La poudre blanche obtenue (7,1 g) est mise en solution dans un mélange de 92 $cm^3$ d'acétonitrile et de 30 $cm^3$ d'eau distillée et on ajoute 3,6 g de bis(trifluoroacétoxy)iodobenzène. Après 24 heures sous agitation à une température voisine de 20°C, on ajoute 400 $cm^3$ d'eau distillée, 120 $cm^3$ d'ammoniaque 4N et 100 $cm^3$ de chloroforme. La phase organique est décantée, la phase aqueuse est extraite par 200 $cm^3$ au total de chloroforme. Les extraits organiques sont rassemblés, lavés par 100 $cm^3$ d'eau distillée, séchés sur du sulfate de magnésium anhydre, et concentrés à sec sous pression réduite (2,7 kPa) à une température voisine de 35°C. On obtient ainsi 3,47 g de mono N-benzyloxycarbonyl-1,7-heptyldiamine, sous la forme d'une huile orangée [Rf= 0,5 ; chromatographie sur couche mince de silice ; éluant : chloroforme, méthanol, ammoniaque à 20 % 12-3-0,5 (en volumes)].

L'acide N-benzyloxycarbonyl-8-aminocaprylique peut être préparé selon : A. KAPOOR, L.W. GERENCSER, J. Pharmac. Sci., 1969, 58(8), 976.

## Exemple 59

L'acide N-[3β-hydroxy-30-(2'-hydroxyéthyl)thiolup-20(29)-èn-28-oyl]-11-aminoundécanoïque est préparé de la manière suivante :

En opérant par analogie avec l'exemple 7, on prépare le N-[3β-acétoxy-30-(2'-acétoxyéthyl)thiolup-20(29)-èn-28-oyl]-11-aminoundécanoate de méthyle à partir du chlorure de 3β-acétoxy-30-(2'-acétoxyéthyl)thiolup-20(29)-èn-28-oyle. 3,1 $cm^3$ d'hydroxyde de sodium 4N sont ajoutés à une solution de 250 mg de N-[3β-acétoxy-30-(2'-acétoxyéthyl)thiolup-20(29)-èn-28-oyl]-11-aminoundécanoate de méthyle dans 20 $cm^3$ de tétrahyrofurane et 10 $cm^3$ de méthanol. Le mélange réactionnel est agité 12 heures à une température voisine de 20°C puis acidifié à un pH voisin de 3 au moyen d'acide chlorhydrique 4N puis dilué à l'aide de 200 $cm^3$ d'eau distillée. Après 2 heures d'agitation, le solide est séparé par filtration, lavé par 90 $cm^3$ au total d'eau distillée puis séché sous pression réduite (10 kPa) à une température voisine de 40°C. On obtient ainsi 150 mg d'acide N-[3β-hydroxy-30-(2'-hydroxyéthyl)thiolup-20(29)-èn-28-oyl]-11-aminoundécanoïque sous la forme d'un solide blanc fondant à une température voisine de 170°C.

Le chlorure de l'acide 3β-acétoxy-30-(2'-acétoxyéthyl)thiolup-20(29)-èn-28-oïque est préparé de manière analogue à celle décrite à l'exemple 11 : à partir de 300 mg d'acide 3β-acétoxy-30-(2'-acétoxyéthyl)thiolup-20(29)-èn-28-oïque on obtient ainsi 350 mg d'une laque incolore qui est utilisée sans autre purification.

L'acide 3β-acétoxy-30-(2'-acétoxyéthyl)thiolup-20(29)-èn-28-oïque peut être obtenu de la manière suivante :

Un mélange de 400 mg d'acide 3β-hydroxy-30-(2'-hydroxyéthyl)thiolup-20(29)-èn-28-oïque, 930 mg d'acétate de sodium et 4,3 $cm^3$ d'anhydride acétique est chauffé 15 minutes à reflux. On ajoute 4,3 $cm^3$ d'anhydride acétique, et on chauffe à reflux durant 15 minutes. On refroidit à une température voisine de 5°C et on ajoute goutte-à-goutte, en 3 heures environ, 50 $cm^3$ d'eau distillée. Après 12 heures d'agitation à une température voisine de 25°C, le solide formé est séparé par filtration, lavé avec au total 50 $cm^3$ d'eau distillée et séché sous vide (20 kPa) à une température voisine de 25°C. Les 500 mg de solide blanc crème obtenus sont mis en suspension dans 30 $cm^3$ d'éthanol. On ajoute 3 $cm^3$ d'ammoniaque (densité 0,8). On agite 12 heures à une température voisine de 25°C. Le solvant est évaporé sous pression réduite (2,7 kPa). Le résidu est chromatographié sur une colonne de 2 cm de diamètre, contenant 200 g de silice (0,02-0,045 mm), éluée par un mélange de cyclohexane et d'acétate d'éthyle 7-3 (en volumes) en recueillant des fractions de 10 $cm^3$. Les 18 premières fractions sont éliminées, les 7 suivantes sont rassemblées et évaporées à sec sous pression réduite (2,7 kPa) à une température voisine de 35°C. On obtient ainsi 300 mg d'acide 3β-acétoxy-30-(2'-acétoxyéthyl)thiolup-20(29)-èn-28-oïque sous la forme d'un solide blanc [Rf=0,49 ; chromatographie sur couche mince de silice ; éluant : cyclohexane, acétate d'éthyle 7-3 (en volumes)].

L'acide 3β-hydroxy-30-(2'-hydroxyéthyl)thiolup-20(29)-èn-28-oïque peut être préparé de la manière suivante :

Après avoir agité durant 3 heures à une température voisine de 25°C, une solution de 215 mg de 2-hydroxyéthanethiol et de 200 mg d'éthylate de sodium dans 15 $cm^3$ d'éthanol, le mélange est concentré à sec sous pression réduite (2,7 kPa) à un température voisine de 40°C. Au solide résiduel sont ajoutés successivement 20 $cm^3$ de tétrahydrofurane et 480 mg d'acide 3β-hydroxy-30-bromolup-20(29)-èn-28-oïque. La suspension obtenue est agitée 48 heures à une température voisine de 25°C puis on ajoute 10 $cm^3$ d'eau distillée. Le mélange est acidifié à un pH voisin de 2 à l'aide d'une solution aqueuse d'acide chlorhydrique 2N puis dilué au moyen de 300 $cm^3$ d'eau distillée. Le solide blanc formé est séparé par filtration, lavé successivement par 300 $cm^3$ au total d'eau distillée et 25 $cm^3$ d'éther diéthylique. Le solide blanc obtenu est séché sous pression réduite (20 kPa) à une température voisine de 35°C. On obtient ainsi 400 mg d'acide 3β-hydroxy-30-(2'-hydroxyéthyl)thiolup-20(29)-èn-28-oïque sous la forme d'un solide blanc fondant à une température voisine de 220°C.

## Exemples 60 et 61

En opérant de manière analogue à l'exemple 59 les dérivés suivants ont été préparés :

**60)**

L'acide N-[3β-hydroxy-30-méthylthiolup-20(29)-èn-28-oyl]-11-aminoundécanoïque à partir de l'acide 3β-hydroxy-30-bromolup-20(29)-èn-28-oïque et de méthanethiol, pF=148°C.

**61)**

L'acide N-[3β-hydroxy-30-(2'acétylaminoéthyl)thiolup-20(29)-èn-28-oyl]-11-aminoundécanoïque à partir de l'acide 3β-hydroxy-30-bromolup-20(29)-èn-28-oïque et de N-acétylaminoéthanethiol, pF=150°C.

## Exemple 62

L'acide N-[3β-hydroxy-30-diéthylaminolup-20(29)-èn-28-oyl]-11-aminoundécanoïque est préparé de la manière suivante :

Le N-[3β-acétyl-30-diéthylaminolup-20(29)-èn-28-oyl]-11-aminoundécanoate de méthyle est préparé de manière analogue à celle décrite à l'exemple 11 à partir de chlorhydrate de 11-aminoundécanoate de méthyle et d'acide 3β-acétyl-30-diéthylaminolup-20(29)-èn-28-oïque.

2,4 $cm^3$ d'hydroxyde de sodium 1 N sont ajoutés à une solution de 450 mg de N-[3β-acétyl-30-diéthylaminolup-20(29) -èn-28-oyl] -11 -amino undécanoate de méthyle dans 10 $cm^3$ de méthanol et 5 $cm^3$ de tétrahydrofurane. Le mélange réactionnel est agité 12 heures à une température voisine de 20°C puis acidifié à pH voisin de 4-5 au moyen d'acide citrique solide puis dilué par 100 $cm^3$ d'eau distillée. On extrait la phase aqueuse par 100 $cm^3$ au total de chloroforme. Les phases organiques réunies sont lavées avec 75 $cm^3$ au total d'eau distillée, puis séchées sur sulfate de magnésium. On évapore le solvant sous pression réduite (2,7 kPa) à une température voisine de 50°C. Le résidu (400 mg) est chromatographié sur une colonne de 2,8 cm de diamètre, contenant 40 g de silice (0,02-0,045 mm), éluée par un mélange de chloroforme, de méthanol et d'ammoniaque 24-6-1 (en volumes), en recueillant des fractions de 20 $cm^3$. Les 4 premières fractions sont éliminées, les 6 suivantes sont rassemblées et évaporées à sec sous pression réduite (2,7 kPa) à une température voisine de 35°C. Le résidu obtenu est repris par 8 $cm^3$ d'acétonitrile. Le solide obtenu est séparé par filtration, lavé avec au total 4 $cm^3$ d'acétonitrile et séché sous pression réduite (2,7 kPa) à une température voisine de 40°C. On obtient ainsi 140 mg d'acide N-[3β-hydroxy-30-diéthylaminolup-20(29)-èn-28-oyl]-11-aminoundécanoïque sous la forme d'un solide blanc, pF =130°C.

L'acide 3β-acétyl-30-diéthylaminolup-20(29)-èn-28-oïque peut être préparé de la manière suivante :

Une solution de 200 mg de 3β-acétyl-30-diéthylaminolup-20(29)-èn-28-oate de méthyle et 800 mg d'iodure de lithium dans 60 $cm^3$ de collidine est chauffée 8 heures à une température voisine du reflux. On évapore

le solvant sous pression réduite (15 Pa) à une température voisine de 70°C. On reprend le résidu par 50 $cm^3$ de dichlorométhane. On lave la phase organique par 100 $cm^3$ au total d'eau distillée. La phase organique est séchée sur du sulfate de magnésium et concentrée à sec sous pression réduite (2,7 kPa) à une température voisine de 35°C. On obtient ainsi 152 mg d'acide 3β-acétyl-30-diéthylaminolup-20(29)-èn-28-oïque qui est utilisé sans autre purification.

Le 3β-acétyl-30-diéthylaminolup-20(29)-èn-28-oate de méthyle peut être obtenu de la manière suivante :

A 30 $cm^3$ de tétrahydrofurane, on ajoute, 30 mg d'acétate de palladium, 140 mg de triphénylphosphine et 0,02 $cm^3$ de triéthylamine. On laisse agiter le mélange 30 minutes à une température voisine de 25°C. Le solide jaune obtenu est ajouté à une solution de 770 mg de 3β-acétyl-30-bromolup-20(29)-èn-28-oate de méthyle, 70 $cm^3$ de tétrahydrofurane et 0,4 $cm^3$ de diéthylamine. On chauffe le milieu réactionnel durant 30 heures à une température voisine du reflux.

Après évaporation du solvant sous pression réduite (2,7 kPa) à une température voisine de 40°C, le solide résiduel est repris par 50 $cm^3$ de dichlorométhane. La phase organique est lavée par 45 $cm^3$ au total d'eau distillée, séchée sur sulfate de magnésium anhydre puis évaporée sous pression réduite (2,7 kPa) à une température voisine de 40°C. Le résidu est chromatographié sur une colonne de 2,3 cm de diamétre contenant 60 g de silice (0,02-0,045 mm) éluée par un mélange de chloroforme et de méthanol 9-1 (en volumes) en recueillant des fractions de 15 $cm^3$. Les 8 premières fractions sont éliminées, les 50 suivantes sont rassemblées et concentrées à sec sous pression réduite (2,7 kPa) à une température voisine de 35°C. On obtient ainsi 400 mg de 3β-acétyl-30-diéthylaminolup-20(29)-èn-28-oate de méthyle.

Le 3β-acétyl-30-bromolup-20(29)-èn-28-oate de méthyle a été synthétisé selon B.Pradham, Indian J. Chem. 22B, 1983, 12-16.

**Exemples 63 à 65**

Les dérivés suivants ont été préparés de manière analogue à la méthode décrite dans l'exemple 62.

**63)**

L'acide N-[3β-hydroxy-30-pyrrolidinolup-20(29)-èn-28-oyl] 11-aminoundécanoïque est préparé à partir de 3β-acétyl-30-bromolup-20(29)-èn-28-oate de méthyle et de pyrrolidine, pF=146°C.

**64)**

L'acide N-[3β-hydroxy-30-(2'-hydroxyéthyl)aminolup-20(29)-èn-28-oyl]-11-aminoundécanoïque est préparé à partir de 3β-acétyl-30-bromolup-20(29)-èn-28-oate de méthyle et de 2-acétyloxyéthanamine, pF=214°C.

**65)**

L'acide N-(3β-hydroxy-30-(4'-méthylpipérazino)lup-20(29)-èn-28-oyl] 11-aminoundécanoïque est préparé à partir de 3β-acétyl-30-bromolup-20(29)-èn-28-oate de méthyle et de N-méthylpipérazine, pF=160°C.

**Exemple 66**

Acide N-[30-acétoxy-3β-hydroxylup-20(29)-èn-28-oyl]-11-aminoundécanoïque ;
Acide N'-[N-[3β-hydroxylup-20(29)-èn-28-oyl]-8-aminooctanoyl]-2-aminoisobutyrique ;
Acide N'-[N-(3β-hydroxylup-20(29)-èn-28-oyl]-8-aminooctanoyl]-4-amino-3(R,S)-hydroxybutyrique ;
Acide N'-[N-[3β-hydroxylup-20(29)-èn-28-oyl]-8-aminooctanoyl]-4-amino-4-benzyl-3-hydroxybutyrique (3S,4S) ;
Acide N'-[N-[3β-hydroxylup-20(29)-èn-28-oyl]-8-aminooctanoyl]-4-amino-4-cyclohexylméthyl-3-hydroxybutyrique (3R,4S) ;
Acide N'-[N-[3β-hydroxylup-20(29)-èn-28-oyl]-8-aminooctanoyl]-5-hydroxy-2-pipéridinecarboxylique (2S,5R) ;
Acide 3-[[N-[3β-hydroxylup-20(29)-èn-28-oyl]-7-aminoheptylcarbamoyl]-propionique ;
Acide 3-[[N-[3β-hydroxylup-20(29)-èn-28-oyl]-7-aminoheptylcarbamoyl]-2(R)-hydroxypropionique ;
Acide 3-[[N-[3β-hydroxylup-20(29)-èn-28-oyl]-7-aminoheptylcarbamoyl]-2(S)-hydroxypropionique ;
Acide 3-[[N-[3β-hydroxylup-20(29)-èn-28-oyl]-7-aminoheptylcarbamoyl]-2(R,S )-hydroxypropionique ;
Acide 3-[[N-(3β-hydroxylup-20(29)-èn-28-oyl]-7-aminoheptylcarbamoyl]-3(R)-hydroxypropionique ;
Acide 3-[(N-(3β-hydroxylup-20(29)-èn-28-oyl]-7-aminoheptylcarbamoyl]-3(S)-hydroxypropionique ;
Acide 3-[N-[3β-hydroxylup-20(29)-èn-28-oyl]-7-aminoheptylcarbamoyl-3(R,S)-hydroxypropionique ;
Acide 3-[[N-[3β-hydroxylup-20(29)-èn-28-oyl]-7-aminoheptylcarbamoyl]-2(R),3(R)-dihydroxypropionique ;
Acide 3-[[N-[3β-hydroxylup-20(29)-èn-28-oyl]-7-aminoheptylcarbamoyl]-2(S),3(S)-dihydroxypropionique ;
Acide 3-[[N-[3β-hydroxylup-20(29)-èn-28-oyl]-7-aminoheptylcarbamoyl]-2(R),3(S)-dihydroxypropionique ;
Acide 3-[[N-[3β-hydroxylup-20(29)-èn-28-oyl]-7-aminoheptylcarbamoyl]-2(S),3(R)-dihydroxypropionique ;
Acide 4-[N-(3β-hydroxylup-20(29)-èn-28-oyl]-7-aminoheptylcarbamoyl]-butyrique ;

Acide 4-[N-[3β-hydroxylup-20(29)-èn-28-oyl]-7-aminoheptylcarbamoyl]-4(R)-hydroxybutyrique ;
Acide 4-[N-[3β-hydroxylup-20(29)-èn-28-oyl]-7-aminoheptylcarbamoyl]-4(S)-hydroxybutyrique ;
Acide 4-[N-[3β-hydroxylup-20(29)-èn-28-oyl]-7-aminoheptylcarbamoyl]-4(R,S) hydroxybutyrique ;
Acide 4-[N-[3β-hydroxylup-20(29)-èn-28-oyl]-7-aminoheptylcarbamoyl]-2(R)-hydroxybutyrique ;
Acide 4-[N-[3β-hydroxylup-20(29)-èn-28-oyl]-7-aminoheptylcarbamoyl]-2(S)-hydroxybutyrique ;
Acide 4-[N-[3β-hydroxylup-20(29)-èn-28-oyl]-7-aminoheptylcarbamoyl]-2(R,S)-hydroxybutyrique ;
Acide 4-[N-(3β-hydroxylup-20(29)-èn-28-oyl]-7-aminoheptylcarbamoyl]-2(R,S)-phénylbutyrique ;
Acide 4-[N-[3β-hydroxylup-20(29)-èn-28-oyl]-7-aminoheptylcarbamoyl]-3(R,S)-phénylbutyrique ;
Acide 4-[N-[3β-hydroxylup-20(29)-èn-28-oyl]-7-aminoheptylcarbamoyl]-4(R,S) phénylbutyrique ;
Acide 4-[N-[3β-hydroxylup-20(29)-èn-28-oyl]-7-aminoheptylcarbamoyl]-3(R,S)-méthylbutyrique ;
Acide 4-[N-[3β-hydroxylup-20(29)-èn-28-oyl]-7-aminoheptylcarbamoyl]-3(R,S)-hydroxybutyrique ;
Acide 4-(N-[3β-hydroxylup-20(29)-èn-28-oyl]-7-aminoheptylcarbamoyl]-3,3-diméthylbutyrique ;
Acide 4-[N-[3β-hydroxylup-20(29)-èn-28-oyl]-7-aminoheptylcarbamoyl]-2,2-diméthylbutyrique ;
Acide 4-[N-[3β-hydroxylup-20(29)-èn-28-oyl]-7-aminoheptylcarbamoyl]-4,4-diméthylbutyrique ;
Acide 4-[N-[3β-hydroxylup-20(29)-èn-28-oyl]-7-aminoheptylcarbamoyl]-3-hydroxy-3-méthylbutyrique 3(R,S) ;
Acide 4-[N-[3β-hydroxylup-20(29)-èn-28-oyl]-7-aminoheptylcarbamoyl]-3,4-dihydroxybutyrique, 3(R,S),4(R,S) ;
Acide 4-[N-[3β-hydroxylup-20(29)-èn-28-oyl]-7-aminoheptylcarbamoyl]-2,3-dihydroxybutyrique, 2(R,S),3(R,S) ;
Acide 4-[N-[3β-hydroxylup-20(29)-èn-28-oyl]-7-aminoheptylcarbamoyl]-4-isobutyl-3-hydroxybutyrique, 3(R,S),4(R,S) ;
Acide [N-[3β,30-dihydroxylup-20(29)-èn-28-oyl]-7-aminoheptylcarbamoyl]acétique ;
Acide N-[3β-hydroxylup-20(29)-èn-28-oyl]-11-amino-2(R,S)-méthylundécanoïque ;
N'-[N-[3β,30-dihydroxylup-20(29)-èn-28-oyl]-8-aminooctanoyl]-L statine ;
N'-[N-[3β,30-dihydroxylup-20(29)-èn-28-oyl]-8-aminooctanoyl]-L sérine ;
N'-[N-[3β,30-dihydroxylup-20(29)-èn-28-oyl]-8-aminooctanoyl]-L thréonine ;
N'-[N-[3β-hydroxylup-20(29)-èn-28-oyl]-7-aminoheptanoyl]-L statine ;
N'-[N-[3β-hydroxylup-20(29)-èn-28-oyl]-7-aminoheptanoyl]-L thréonine ;
N'-[N-[3β-hydroxylup-20(29)-èn-28-oyl]-7-aminoheptanoyl]-L sérine ;
N'-[N-[3β-hydroxylup-20(29)-èn-28-oyl]-11-aminoundécanoyl]-glycine ;
N'-[N-[3β-hydroxylup-20(29)-èn-28-oyl]-11-aminoundécanoyl]-L statine ;
N'-[N-[3β-hydroxylup-20(29)-èn-28-oyl]-11-aminoundécanoyl]-L thréonine ;
N'-[N-[3β-hydroxylup-20(29)-èn-28-oyl]-11-aminoundécanoyl]-L sérine ;
N'-[N-[3β-hydroxylup-20(29)-èn-28-oyl]-11-aminoundécanoyl]-D statine ;
N'-[N-[3β-hydroxylup-20(29)-èn-28-oyl]-8-aminooctanoyl]-D statine ;
N'-[N-[3β-hydroxy-30-(2'-hydroxyéthyl)thiolup-20(29)-èn-28-oyl]-8-aminooctanoyl]-glycine ;
N'-[N-[3β-hydroxy-30-(2'-hydroxyéthyl)thiolup-20(29)-èn-28-oyl]-8-aminooctanoyl]-L statine ;
N'-[N-[3β-hydroxy-30-(2'-hydroxyéthyl)thiolup-20(29)-èn-28-oyl]-8-aminooctanoyl]-L thréonine ;
N'-[N-[3β-hydroxy-30-(2'-hydroxyéthyl)thiolup-20(29)-èn-28-oyl)-8-aminooctanoyl]-L sérine ;
Acide N-[3β-hydroxy-30-méthoxylup-20(29)-èn-28-oyl]-11-aminoundécanoïque ;
Acide N-[30-t.butyloxy-3β-hydroxylup-20(29)-èn-28-oyl]-11-aminoundécanoïque ;
Acide N-[3β-hydroxy-30-(2',3'-dihydroxypropyl)oxylup-20(29)-èn-28-oyl]-11-aminoundécanoïque ;
Acide N-[3β-hydroxy-30-(2'-hydroxyéthyl)oxylup-20(29)-èn-28-oyl]-11-aminoundécanoïque ;
Acide N-[3β-hydroxy-30-(2',3'-dihydroxypropyl)thiolup-20(29)-èn-28-oyl]-11-aminoundécanoïque ;
Acide N-[3β-hydroxylup-20(29)-èn-28-oyl]-3-(8-aminooctamido)phénylacétique ;
Acide N-[3β-hydroxylup-20(29)-èn-28-oyl]-4-(8-aminooctamido)phénylacétique ;
Acide N-[3β-hydroxylup-20(29)-èn-28-oyl]-2-(8-aminooctamido)phénylacétique ;
Acide N-[3β-hydroxylup-20(29)-èn-28-oyl]-3-(11-aminoundécanamido)phénylacétique ;
Acide N-(3β-hydroxylup-20(29)-èn-28-oyl]-4-(11-aminoundécanamido)phénylacétique ;
Acide N-[3β-hydroxylup-20(29)-èn-28-oyl]-2-(11-aminoundécanamido)phénylacétique ;
Acide N-[3β-hydroxylup-20(29)-èn-28-oyl]-3-(8-aminooctamido)méthylbenzoïque ;
Acide N-[3β-hydroxylup-20(29)-èn-28-oyl]-2-(8-aminooctamido)méthylbenzoïque ;
Acide N-[3β-hydroxylup-20(29)-èn-28-oyl]-4-(8-aminooctamido)méthylbenzoïque ;
Acide N-[3β-hydroxylup-20(29)-èn-28-oyl]-3-(11-aminoundécanamido)méthylbenzoïque ;
Acide N-[3β-hydroxylup-20(29)-èn-28-oyl]-2-(11-aminoundécanamido)métlnylbenzoïque ;
Acide N-[3β-hydroxylup-20(29)-èn-28-oyl]-4-(11-aminoundécanamido)méthylbenzoïque.
Acide N'-[N-(3β-hydroxylup-20(29)-èn-28-oyl)-8-aminooctanoyl]-2-aminoisobutyrique ;
Acide N'-[N-(3β-hydroxylup-20(29)-èn-28-oyl)-8-aminooctanoyl]-5-hydroxy-2-pipéridinecarboxylique (2-R,S) ;
Acide N'-[N-(3β-hydroxylup-20(29)-èn-28-oyl)-8-aminooctanoyl]-3-aminobenzoïque ;
Acide N'-[N-[3β-hydroxylup-20(29)-èn-28-oyl]-8-aminooctanoyl]-2-aminobenzoïque ;

Acide N'-[N-[3β-hydroxylup-20(29)-èn-28-oyl]-8-aminooctanoyl]-4-aminobenzoïque ;
Acide N'-[N-(3β-hydroxylup-20(29)-èn-28-oyl)-8-aminooctanoyl]-3-(R,S)-amino-3-phénylpropionique ;
Acide N'-[N-(3β-hydroxylup-20(29)-èn-28-oyl)-8-aminooctanoyl]-3-aminoisobutyrique (2-R,S) ;
Acide N'-[N-(3β-hydroxylup-20(29)-èn-28-oyl)-8-aminooctanoyl]-3-aminobutyrique (3-R,S) ;
Acide N'-[N-(3β-hydroxylup-20(29)-èn-28-oyl)-8-aminooctanoyl]-N'-hydroxycarbonyléthylpropionique ;
N'-[N-[3β-hydroxylup-20(29)-èn-28-oyl]-8-aminooctanoyl]-trans-4-hydroxy-L proline ;
N'-[N-[3β-hydroxylup-20(29)-èn-28-oyl]-9-aminononanoyl] glycine ;
Acide N'-[N-[3β-hydroxylup-20(29)-èn-28-oyl]-8-aminooctanoyl]-nipécotique ;
Acide N'-[N-[3β-hydroxylup-20(29)-èn-28-oyl]-8-aminooctanoyl]-4-amino-6-méthylheptanoïque-(4S) ;
N'-[N-(3β-hydroxylup-20(29)-èn-28-oyl]-8-aminooctanoyl]-4-amino-5-phenyl-3-hydroxypentanoïque(3S,4S) ;
Acide 2-[N-[3β-hydroxylup-20(29)-èn-28-oyl]-7-aminoheptylcarbamoyl]-2(R)-propionique ;
Acide 2-[N-[3β-hydroxylup-20(29)-èn-28-oyl]-7-aminoheptylcarbamoyl]-2(S)-propionique ;
Acide 2-[N-[3β-hydroxylup-20(29)-èn-28-oyl]-7-aminoheptylcarbamoyl]-2(R,S)-propionique ;
Acide 2-[N-[3β-hydroxylup-20(29)-èn-28-oyl]-7-aminoheptylcarbamoyl]-2,2-diméthylacétique ;
Acide 2-[N-[3β-hydroxylup-20(29)-èn-28-oyl]-7-aminoheptylcarbamoyl]-2(R)-benzylacétique ;
Acide 2-[N-[3β-hydroxylup-20(29)-èn-28-oyl]-7-aminoheptylcarbamoyl]-2(S)-benzylacétique ;
Acide 2-[N-[3β-hydroxylup-20(29)-èn-28-oyl]-7-aminoheptylcarbamoyl]-2(R,S)-benzylacétique ;
Acide [N-[3β-hydroxy-30-(2'-hydroxyéthyl)thio-lup-20(29)-èn-28-oyl]-7-aminoheptylcarbamoyl]acétique ;
Acide 2-[N-[3β-hydroxylup-20(29)-èn-28-oyl]-7-aminoheptylcarbamoyl]-benzoïque ;
Acide 3-[N-[3β-hydroxylup-20(29)-èn-28-oyl]-7-aminoheptylcarbamoyl]-benzoïque ;
Acide N'-[N-[3β-hydroxylup-20(29)-èn-28-oyl]-7-aminoheptylcarbamoyl]-benzoïque ;
Acide 2-[N-[3β-hydroxylup-20(29)-èn-28-oyl]-8-aminooctylcarbamoyl]-2(R)-propionique ;
Acide 2-[N-[3β-hydroxylup-20(29)-èn-28-oyl]-8-aminooctylcarbamoyl]-2(S)-propionique ;
Acide 2-(N-[3β-hydroxylup-20(29)-èn-28-oyl]-8-aminooctylcarbamoyl]-2,2-diméthylacétique ;
Acide 2-[N-[3β-hydroxylup-20(29)-èn-28-oyl]-8-aminooctylcarbamoyl]-2(R)-benzylacétique ;
Acide 2-[N-[3β-hydroxylup-20(29)-èn-28-oyl]-8-aminooctylcarbamoyl]-2(S)-benzylacétique ;
Acide 2-[N-[3β-hydroxylup-20(29)-èn-28-oyl]-8-aminooctylcarbamoyl]-2(R,S)-benzylacétique ;
Acide [N-[3β-hydroxy-30-(2'-hydroxyéthyl)thio-lup-20(29)-èn-28-oyl]-8-aminooctylcarbamoyl]acétique ;
Acide 2-[N-[3β-hydroxylup-20(29)-èn-28-oyl]-8-aminooctylcarbamoyl]-benzoïque ;
Acide 3-[N-[3β-hydroxylup-20(29)-èn-28-oyl]-8-aminooctylcarbamoyl]-benzoïque ;
Acide 4-[N-[3β-hydroxylup-20(29)-èn-28-oyl]-8-aminooctylcarbamoyl]-benzoïque.

La présente invention concerne également les compositions pharmaceutiques contenant au moins un produit de formule générale (I) éventuellement sous forme de sel, à l'état pur ou sous forme d'une association avec un ou plusieurs diluants ou adjuvants compatibles et pharmaceutiquement acceptables, ou avec un autre agent destiné au traitement du SIDA, un agent antiviral, immunomodulateur ou antimicrobien.

La composition selon l'invention est capable de maintenir en vie les cellules infectées par un virus HIV et donc de réduire la progression vers le SIDA ou de diminuer sa gravité chez les sujets déjà infectés en réduisant la mortalité des cellules infectées. Les compositions peuvent être utilisées par voie orale, parentérale ou rectale.

Les compositions peuvent être utilisées à titre curatif ou à titre préventif chez des sujets présentant une immunodéficience et/ou infectés par un virus HIV. Bien entendu, la constitution de ces compositions sera adaptée au cas particulier du tractus digestif des immunodéprimés.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon.

Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle.

Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles

qui peuvent être dissoutes au moment de l'emploi dans de l'eau stérile ou tout autre milieu stérile injectable.

Les compositions par administration rectale sont les suppositoires ou les capsules rectales, qui contiennent outre le principe actif, des excipients tels que le beurre de cacao, des glycérides semi-synthétiques ou des polyéthylèneglycols.

En thérapeutique humaine, le médecin déterminera la posologie qu'il estime la plus appropriée en fonction d'un traitement préventif ou curatif, en fonction de l'âge, du poids, du degré de l'infection et des autre facteurs propres au sujet à traiter. Généralement, les doses sont comprises entre 10 et 100 mg/kg par voie intra veineuse pour un adulte.

La présente invention concerne également les associations constituées d'un ou plusieurs dérivés du lupane de formule générale (I), et/ou le cas échéant leurs sels, et d'un autre principe actif connu pour son activité anti-rétrovirus, éventuellement en présence d'excipients pharmaceutiquement acceptables.

Les agents anti-rétrovirus pouvant être associés sont choisis parmi des agents compatibles et inertes vis-à-vis du dérivé du lupane de formule générale (I). A titre non limitatif ces agents sont choisis parmi des inhibiteurs de la reverse transcriptase [zidovudine (AZT), didanosine (DDI), didéoxycytidine (DDC), TIBO, névirapine, PMEA, HEPT... ], parmi des inhibiteurs de la protéase [comme par exemple le A 77003], ou parmi des inhibiteurs de la protéine tat [comme par exemple le RO 24-7429].

L'exemple suivant illustre une composition selon l'invention.

**Exemple**

On prépare des ampoules contenant une solution dosée à 10 mg d'acide N-[3β-hydroxylup-20(29)-èn-28-oyl]-11-aminoundécanoïque en ajoutant 2 % de glycine et 0,1 % de cystéine à 500 $cm^3$ d'une solution à 0,2 % d'acide N-[3β-hydroxylup-20(29)èn-28-oyl)-11-aminoundécanoïque dans du tampon phosphate isotonique. La solution est divisée et répartie aseptiquement dans des ampoules. Chaque ampoule contient 10 mg de produit actif.

## Revendications

1. Nouveau dérivé du lupane de formule générale:

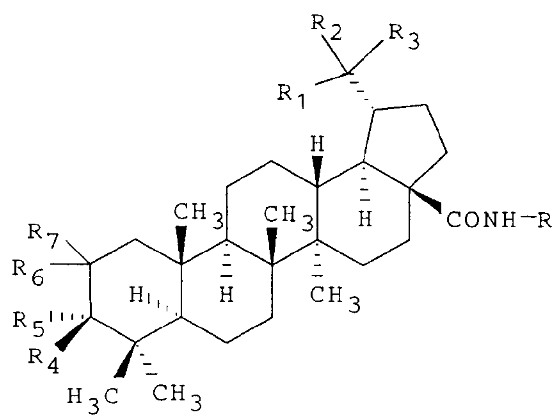

dans laquelle :

R représente un radical de formule générale:

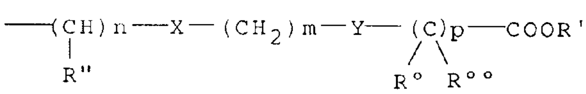

dans laquelle R' et R" identiques ou différents sont des atomes d'hydrogène ou des radicaux alcoyle,

X est une liaison ou représente un radical carbamoyle, N-méthyl carbamoyle, aminocarbonyle ou N-méthylaminocarbonyle,

Y est une liaison ou représente un radical phénylène,

R° et R°° identiques ou différents sont des atomes d'hydrogène ou des radicaux alcoyle (étant entendu

que R° et R°° ne sont pas nécessairement identiques sur chaque motif -CR°R°°-), ou R° est hydroxy, hydroxyalcoyle, phényle, benzyle, carbamoylméthyle ou bien, lorsque Y est une liaison et X est carbamoyle, R° peut former un cycle à 5 ou 6 chaînons avec l'atome d'azote contenu dans X, ce cycle pouvant en outre comprendre un autre hétéroatome choisi parmi l'oxygène ou le soufre et,
n, m et p sont des nombres entiers de 0 à 16 tels que m + n + p soit compris entre 4 et 16,
$R_1$ est un radical méthyle, ou forme avec $R_3$ un radical méthylène ou un radical oxo,
$R_2$ est un radical hydroxy, méthyle, hydroxyméthyle ou un radical $-CH_2OR'_2$, $-CH_2SR'_2$ ou $-CH_2NHR'_2$ pour lequel $R'_2$ est alcoyle, hydroxyalcoyle, dihydroxyalcoyle, acétamidoalcoyle, ou acétyle, ou $R_2$ est un radical amino substitué par un radical hydroxyalcoyle ou carboxyhydroxyalcoyle, ou un radical dialcoylamino dont les parties alcoyle peuvent former avec l'atome d'azote auquel elle sont rattachées, un hétérocycle à 5 ou 6 chaînons contenant éventuellement un autre hétéroatome choisi parmi l'oxygène, le soufre ou l'azote et éventuellement N-alcoylé,
$R_3$ est un atome d'hydrogène ou forme avec $R_1$ ou $R_2$ un radical méthylène ou un radical oxo,
$R_4$ et $R_5$ sont différents et représentent un atome d'hydrogène ou un radical hydroxy, ou forment ensemble un radical oxo, hydroxyimino ou alcoyloxyimino éventuellement substitué (par un radical carboxy ou dialcoylamino dont les parties alcoyle peuvent former avec l'atome d'azote auquel elles sont rattachées, un hétérocycle à 5 ou 6 chaînons contenant éventuellement un autre hétéroatome choisi parmi l'oxygène, l'azote ou le soufre et éventuellement substitué par un radical alcoyle) et
$R_6$ et $R_7$ sont des atomes d'hydrogène, ou bien
$R_4$ et $R_5$, et $R_5$ et $R_7$ forment ensemble des radicaux oxo,
étant entendu que les radicaux alcoyle sont droits ou ramifiés et contiennent 1 à 4 atomes de carbone, ainsi que leurs sels lorsqu'ils existent.

2. Nouveau dérivé du lupane selon la revendication 1 pour lequel :
R représente un radical de formule générale :

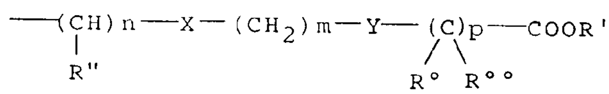

dans laquelle R' et R" identiques ou différents sont des atomes d'hydrogène ou des radicaux alcoyle,
X est une liaison ou représente un radical carbamoyle, N-méthyl carbamoyle, aminocarbonyle,
Y est une liaison ou représente un radical phénylène,
R° et R°° identiques ou différents sont des atomes d'hydrogène ou des radicaux alcoyle (étant entendu que R° et R°° ne sont pas nécessairennent identiques sur chaque motif -CR°R°°-), ou R° est hydroxy, hydroxyalcoyle, phényle, benzyle, carbamoylméthyle ou bien, lorsque Y est une liaison et X est carbamoyle, R° peut former un cycle à 5 ou 6 chaînons avec l'atome d'azote contenu dans X et,
n, m et p sont des nombres entiers de 0 à 16 tels que m + n + p soit compris entre 4 et 16,
$R_1$ est un radical méthyle, ou forme avec $R_3$ un radical méthylène ou un radical oxo,
$R_2$ est un radical hydroxy, méthyle, hydroxyméthyle ou un radical $-CH_2SR'_2$, ou $-CH_2NHR'_2$ pour lequel $R'_2$ est alcoyle, hydroxyalcoyle, acétamidoalcoyle, ou acétyle, ou $R_2$ est un radical amino substitué par un radical hydroxyalcoyle ou carboxyhydroxyalcoyle, ou un radical dialcoylamino dont les parties alcoyle peuvent former avec l'atome d'azote auquel elle sont rattachées, un hétérocycle à 5 ou 6 chaînons contenant éventuellement un autre atome d'azote et éventuellement N-méthylé,
$R_3$ est un atome d'hydrogène ou forme avec $R_1$ ou $R_2$ un radical méthylène ou un radical oxo,
$R_4$ et $R_5$ sont différents et représentent un atome d'hydrogène ou un radical hydroxy, ou forment ensemble un radical oxo, ou alcoyloxyimino éventuellement. substitué (par un radical carboxy ou dialcoylamino dont les parties alcoyle peuvent former avec l'atome d'azote auquel elles sont rattachées, un hétérocycle à 5 ou 6 chaînons contenant éventuellement un autre atome d'azote éventuellement substitué par un radical méthyle) et
$R_6$ et $R_7$ sont des atomes d'hydrogène, ou bien
$R_4$ et $R_5$, et $R_5$ et $R_7$ forment ensemble des radicaux oxo,
étant entendu que les radicaux alcoyle sont droits ou ramifiés et contiennent 1 à 4 atomes de carbone, ainsi que leurs sels lorsqu'ils existent.

3. Un dérivé du lupane selon la revendication 1, caractérisé en ce qu'il s'agit de la N'-[N-[3β-hdroxylup-20(29)-èn-28-oyl]-8-aminooctanoyl]-β-alanine.

4. Un dérivé du lupane selon la revendication 1, caractérisé en ce qu'il s'agit de la N'-[N-[3β-hydroxylup-20(29)-èn-28-oyl]-8-aminooctanoyl]-L-alanine.

5. Un dérivé du lupane selon la revendication 1, caractérisé en ce qu'il s'agit de la N'-[N-[3β-hydroxylup-20(29)-èn-28-oyl]-8-aminooctanoyl]-L-thréonine.

6. Un dérivé du lupane selon la revendication 1, caractérisé en ce qu'il s'agit de la N'-[N-[3β-hydroxylup-20(29)-èn-28-oyl]-8-aminooctanoyl]-L-statine.

7. Un dérivé du lupane selon la revendication 1, caractérisé en ce qu'il s'agit de l'acide N-[3β-hydroxylup-20(29)-èn-28-oyl]-7-amino-heptylcarbamoylacétique.

8. Procédé de préparation d'un nouveau dérivé du lupane selon la revendicationn 1, caractérisé en ce que l'on fait agir un amino acide de formule générale :

$$H_2N - R$$

dans laquelle R est défini comme dans la revendication 1, et dont le cas échéant, la fonction acide a été préalablement protégée, sur le chlorure de l'acide de formule générale :

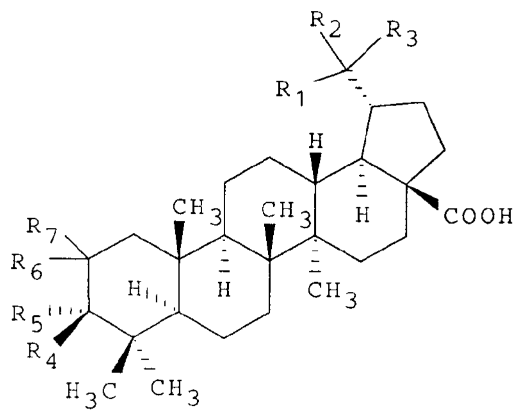

dans laquelle $R_1$, $R_2$, $R_3$, $R_4$, $R_5$, $R_6$ et $R_7$ sont définis comme dans la revendication 1, étant entendu que les fonctions hydroxy ou acides représentées ou contenues dans ces radicaux sont préalablement protégées, puis transforme le cas échéant en un ester, élimine les radicaux protecteurs et transforme éventuellement le produit obtenu en un sel.

9. Procédé de préparation d'un dérivé du lupane selon la revendication 1, pour lequel $R_4$ et $R_5$ forment ensemble un radical hydroxyimino ou alcoyloxyimino éventuellement substitué comme défini dans la revendication 1, et les autres radicaux sont définis comme dans la revendication 1, caractérisé en ce que l'on fait agir l'hydroxylamine ou un de ses dérivés de formule générale :

$$R_5\text{-O-}NH_2$$

dans laquelle $R_8$ est un atome d'hydrogène ou un radical alcoyle éventuellement substitué (par un radical carboxy ou dialcoylamino dont les parties alcoyle peuvent former avec l'atome d'azote auquel elles sont rattachées, un hétérocycle à 5 ou 6 chaînons contenant éventuellement un autre hétéroatome choisi parmi l'oxygène, l'azote ou le soufre et éventuellement substitué par un radical alcoyle), sur un dérivé du lupane selon la revendication 1 pour lequel $R_4$ et $R_5$ forment ensemble un radical oxo, puis le cas échéant, élimine les radicaux protecteurs et transforme éventuellement le produit obtenu en un sel.

10. Procédé de préparation d'un dérivé du lupane selon la revendication 1, pour lequel $R_1$ et $R_2$ sont des radicaux méthyle, $R_3$ est un atome d'hydrogène et les autres radicaux sont définis comme dans la revendication 1, caractérisé en ce que l'on réduit un dérivé du lupane selon la revendication 1 pour lequel $R_1$ et $R_3$ forment ensemble un radical méthylène et $R_2$ est un radical méthyle, puis le cas échéant, élimine les radicaux protecteurs et transforme éventuellement le produit obtenu en un sel.

11. Procédé de préparation d'un dérivé du lupane selon la revendication 1, pour lequel $R_2$ est un radical hydroxy, $R_1$ est un radical méthyle, $R_3$ est un atome d'hydrogène et les autres radicaux sont définis comme dans la revendication 1, caractérisé en ce que l'on effectue la réduction d'un dérivé du lupane selon la

revendication 1, pour lequel $R_2$ et $R_3$ forment ensemble un radical oxo et $R_1$ est un radical méthyle, puis le cas échéant, élimine les radicaux protecteurs et transforme éventuellement le produit obtenu en un sel.

**12.** Procédé de préparation d'un dérivé du lupane selon la revendication 1, pour lequel $R_1$ et $R_3$ forment ensemble un radical oxo, $R_2$ est un radical hydroxy et les autres radicaux sont définis comme dans la revendication 1, caractérisé en ce que l'on oxyde un dérivé du lupane selon la revendication 1, pour lequel $R_1$ et $R_3$ forment ensemble un radical oxo et $R_2$ est un radical méthyle, puis le cas échéant, élimine les radicaux protecteurs et transforme éventuellement le produit obtenu en un sel.

**13.** Procédé de préparation d'un dérivé du lupane selon la revendication 1, pour lequel X (dans le radical R) représente un radical carbamoyle et les autres radicaux sont définis comme dans la revendication 1, caractérisé en ce que l'on fait agir une amine de formule générale :

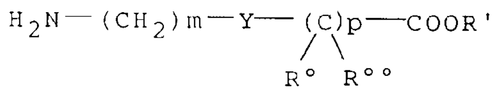

dans laquelle R', R°, R°°, m et p sont définis comme dans la revendication 1, sur un dérivé du lupane de formule générale :

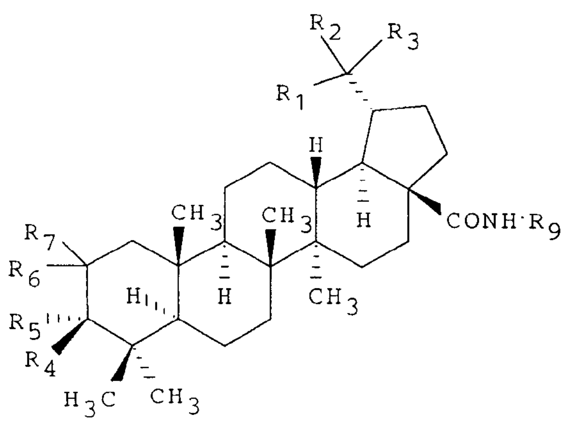

dans laquelle $R_1$, $R_2$, $R_3$, $R_4$, $R_5$, $R_5$ et $R_7$ sont définis comme dans la revendication 1 et $R_9$ représente un radical de formule générale :

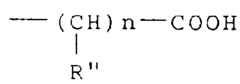

dans laquelle n et R" sont définis comme dans la revendication 1, puis élimine le cas échéant des radicaux protecteurs et transforme éventuellement le produit obtenu en un sel.

**14.** Composition pharmaceutique caractérisée en ce qu'elle contient au moins un produit selon la revendication 1, à l'état pur ou en association avec tout diluant ou adjuvant compatible et pharmaceutiquement acceptable et/ou en association avec un autre principe actif antiviral, immuomodulateur ou antimicrobien.

Office européen des brevets

# RAPPORT DE RECHERCHE EUROPEENNE

Numero de la demande

EP 92 40 3046

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.5 ) |
| A | COLLECTION OF CZECHOSLOVAK CHEMICAL COMMUNICATIONS. vol. 41, no. 4, 1976, PRAGUE CS pages 1200 - 1207 J. PROTIVA ET AL 'Triterpines. IL. Reaction of Amides of 28-Lupanoic Acid with Lead Tetraacetate and Iodine. Mass Spectra of 12-Lupene Derivatives.' * page 1201, composés I,IV,VII,VIII,X ; page 1203, composé XVIII, et lignes 3-5 * | 1,8,14 | C07J63/00<br>A61K31/56 |
| A | JOURNAL OF NATURAL PRODUCTS vol. 50, no. 6, 1987, COLUMBUS OHIO US. pages 1167 - 1170 TAKAO KONOSHIMA ET AL 'Studies on Inhibitors of Skin-Tumor Promotion, I. Inhibitory Effects of Triterpenes from Euptelea polyandra on Epstein-Barr Virus Activation' * page 1167; exemple 14 * * page 1169, colonne 1, ligne 17 - ligne 40 * | 1,14 | DOMAINES TECHNIQUES RECHERCHES (Int. Cl.5 ) |
| A | BULLETIN DE LA SOCIETE CHIMIQUE DE FRANCE. 1962, PARIS FR pages 1133 - 1136 J. M. LEHN ET AL 'Synthèses dans la Série du Lupane.' * page 1133, composé 4 ; page 1134 colonne 2 * | 1,8 | C07J<br>A61K |

-----

Le présent rapport a été établi pour toutes les revendications

| Lieu de la recherche | Date d'achèvement de la recherche | Examinateur |
|---|---|---|
| LA HAYE | 23 FEVRIER 1993 | WATCHORN P.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)